# EUROPEAN PATENT SPECIFICATION

(11) **EP 3 132 037 B1**
(45) Date of publication and mention of the grant of the patent: **02.06.2021**
(21) Application number: 15780364.4
(22) Date of filing: 17.04.2015
(51) Int. Cl.: C12N 15/10, C12Q 1/68, C12N 15/64, C12N 15/66, C12Q 1/6844

(54) **METHODS AND SYSTEMS FOR DROPLET TAGGING AND AMPLIFICATION**
VERFAHREN UND SYSTEME ZUR TRÖPFCHENMARKIERUNG UND -VERGRÖSSERUNG
PROCÉDÉS ET SYSTÈMES POUR L'ÉTIQUETAGE ET L'AMPLIFICATION DES GOUTTELETTES

(30) Priority: 17.04.2014 US 201461981108 P
(43) Date of publication of application: 22.02.2017
(73) Proprietor: President and Fellows of Harvard College, Cambridge, MA 02138 (US); The General Hospital Corporation d/b/a Massachusetts General Hospital, Boston, MA 02199-8001 (US)
(72) Inventor: WEITZ, David, A., Bolton, MA 01740 (US); IAFRATE, Anthony J., Newton, MA 02465 (US); ZHENG, Zongli, Boston, MA 02114 (US); ZHUANG, Huidan, Cambridge, MA 02138 (US)
(74) Representative: Hoffmann Eitle
(86) International application number: PCT/US2015/026422
(87) International publication number: WO 2015/161223

(56) References cited:
- WO-A1-2009/085215
- WO-A1-2013/134261
- WO-A1-2014/047561
- WO-A2-2010/025310
- US-A1- 2007 077 572
- US-A1- 2010 136 544
- US-A1- 2011 033 854
- US-A1- 2012 309 002
- US-A1- 2013 165 346
- US-A1- 2013 225 418
- BROUZES ET AL.: 'Droplet microfluidic technology for single- cell high-throughput screening' PROCEEDINGS OF THE NATIONAL ACADEMY OF SCIENCES OF THE UNITED STATES OF AMERICA vol. 106, no. 34, 15 July 2009, ISSN 0027-8424 pages 14195 - 14200, XP055074334

## Description

### FIELD

The present invention generally relates to microfluidic devices, including methods and systems for tagging droplets within such devices.

### BACKGROUND

A variety of techniques exist for producing fluidic droplets within a microfluidic system, such as those disclosed in Int. Pat. Pub. Nos. WO 2004/091763, WO 2004/002627, WO 2006/096571, WO 2005/021151, WO 2010/033200, and WO 2011/056546, In some cases, relatively large numbers of droplets may be produced, and often at relatively high speeds, e.g., the droplets may be produced at rates of least about 10 droplets per second. The droplets may also contain a variety of species therein. However, it can be difficult to accurately track such droplets, especially when large numbers of droplets are produced and/or the droplets are produced at very high rates. In addition, such tracking may be complicated if the droplets are exposed to a variety of different conditions, contain different species, etc., such that the droplets are not all identical.

### SUMMARY

The present invention is defined by the claims. The present description generally relates to microfluidic devices, including methods and systems for tagging droplets within such devices. The subject matter of the present description involves, in some cases, interrelated products, alternative solutions to a particular problem, and/or a plurality of different uses of one or more systems and/or articles.

In one aspect, the present invention is generally directed to a method. According to one set of embodiments, the method comprises acts of exposing a microfluidic droplet to a first condition and adding a first nucleic acid to the droplet, where the first nucleic acid encodes the first condition, exposing the microfluidic droplet to a second condition and adding a second nucleic acid to the droplet, where the second nucleic acid encodes the second condition, and amplifying the first nucleic acid and the second nucleic acid within the microfluidic droplet.

The method, in another set of embodiments, is generally directed to acts of exposing a plurality of microfluidic droplets to a plurality of conditions such that substantially each microfluidic droplet is sequentially exposed to at least two different conditions, where when a microfluidic droplet is exposed to a condition, a nucleic acid encoding the condition is added to the microfluidic droplet, and amplifying the nucleic acids within at least some of the microfluidic droplets.

In another aspect, the present description is generally directed to an article. In accordance with one set of embodiments, the article comprises a plurality of microfluidic droplets, at least some of the droplets containing one or more primers and one or more nucleic acids encoding a plurality of conditions that the at least some droplets were exposed to.

In another aspect, the present description encompasses methods of making one or more of the embodiments described herein. In still another aspect, the present description encompasses methods of using one or more of the embodiments described herein.

Other advantages and novel features of the present invention will become apparent from the following detailed description of various non-limiting embodiments of the invention when considered in conjunction with the accompanying figures.

### BRIEF DESCRIPTION OF THE DRAWINGS

Non-limiting embodiments of the present invention will be described by way of example with reference to the accompanying figures, which are schematic and are not intended to be drawn to scale. In the figures, each identical or nearly identical component illustrated is typically represented by a single numeral. For purposes of clarity, not every component is labeled in every figure, nor is every component of each embodiment of the invention shown where illustration is not necessary to allow those of ordinary skill in the art to understand the invention. In the figures:
Figs. 1A-1C schematically illustrate various methods for tagging droplets, in accordance with various embodiments of the invention;
Figs. 2A-2B schematically illustrates joining together various nucleic acid tags, in certain embodiments of the invention;
Fig. 3 illustrates a schematic for PCR amplification within droplets, according to some embodiments of the invention;
Fig. 4 illustrates evenly amplified nucleic acids, in another embodiment of the invention;
Fig. 5 illustrates a schematic for PCR amplification within droplets, according to some embodiments of the invention;
Figs. 6A-6B illustrate amplified nucleic acids, in yet another embodiment of the invention;
Figs. 7A-7F illustrate apoptosis detection of cells contained within droplets, in accordance with one set of embodiments of the invention;
Fig. 8 illustrates amplification of nucleic acids in the presence of an apoptosis assay reagent, in another set of embodiments of the invention;
Fig. 9 illustrates a schematic for PCR amplification within droplets, according to another embodiment of the invention;
Figs. 10A-10D illustrates the design of a microfluidic device used in one embodiment of the invention;
Fig. 11 illustrates the sequences used in one embodiment of the invention;
Figs. 12A-12D illustrates cells in agarose particles, in accordance with one embodiment of the invention;
Figs. 13A-13B illustrate cell detection using flow cytometry, in another embodiment of the invention;
Figs. 14A-14D illustrate fluorescence microscope images of cells, in yet another embodiment of the invention;
Figs. 15A-15D illustrate fluorescence microscope images of cells, in still another embodiment of the invention;
Figs. 16A-16B illustrate streptavidin magnetic beads in agarose gel particles, in accordance with yet another embodiment of the invention; and
Fig. 17 illustrates barcoded beads, in another embodiment of the invention.

### DETAILED DESCRIPTION

The present description generally relates to microfluidic devices, including methods and systems for tagging droplets within such devices. In some aspects, microfluidic droplets are manipulated by exposing the droplets (or other discrete entities) to a variety of different conditions. By incorporating into the droplets a plurality of nucleic acid "tags," and optionally amplifying the nucleic acids, e.g., within the droplets, the conditions that a droplet was exposed to may be encoded by the nucleic acids. Thus, even if droplets exposed to different conditions are mixed together, the conditions that each droplet encountered may still be determined, for example, by sequencing the nucleic acids.

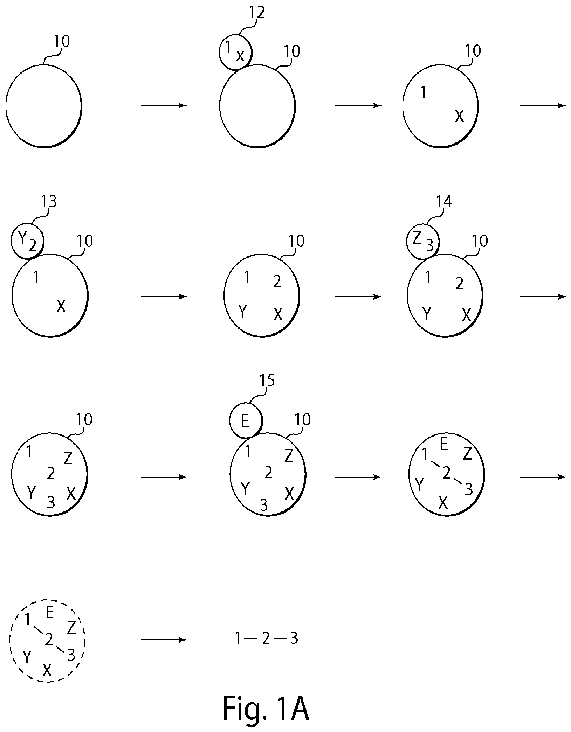

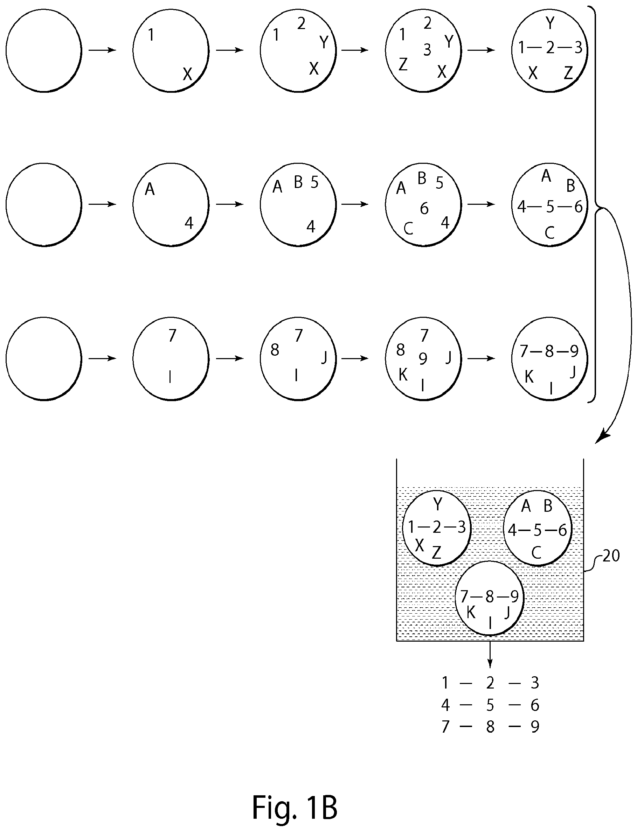

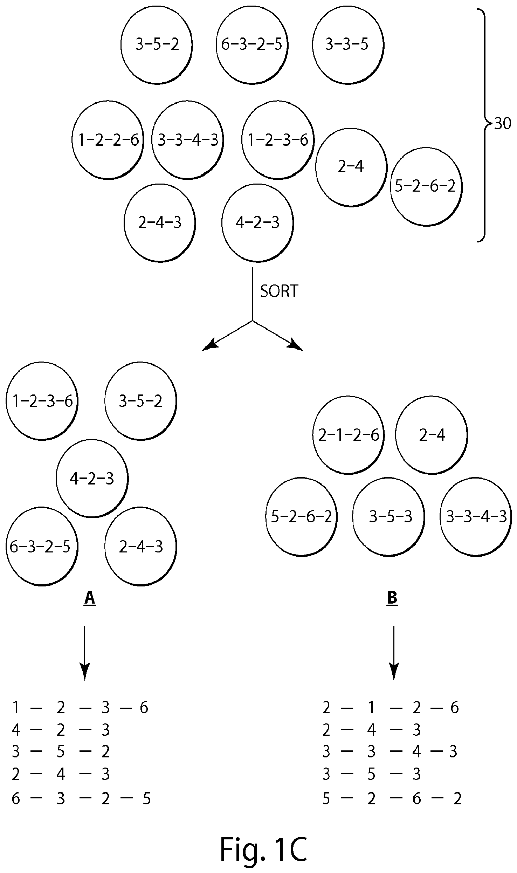

Initially, certain non-limiting examples are discussed with reference to Fig. 1. However, in other embodiments, other configurations may be used as well. Turning first to Fig. 1A, a droplet is shown that is exposed to a variety of conditions (for example, a species such as a drug), and each time the droplet is exposed to a condition, a nucleic acid encoding the condition is added to the droplet. It should be noted that the condition need not be the addition of a chemical species, but could also be a physical condition, such as exposure to a particular temperature. It should further be noted that although droplets are discussed with reference to Fig. 1, this is for ease of presentation only; in other embodiments, other discrete entities may be used instead of these droplets, for example, microwells in a microwell plate.

As is shown in this example, droplet 10 first encounters, and is fused with, droplet 12 containing species X and nucleic acid 1. Subsequently, droplet 10 encounters and is fused with droplet 13 (containing species Y and nucleic acid 2) and droplet 14 (containing species Z and nucleic acid 3). An enzyme E may then be introduced in some fashion into droplet 10, for example, as part of another droplet fusion event (as is shown here with droplet 15), or the enzyme may initially be found in any one of droplets 10, 12, 13, or 14. The enzyme may be used to join nucleic acids 1, 2, and 3 together, as is shown with nucleic acids 1-2-3. For instance, in one set of embodiments, the enzyme may be a polymerase (such as Taq polymerase), and a PCR reaction used to join nucleic acids 1, 2, and 3 together to from a DNA "barcode." The droplet can then be burst to access the joined nucleic acid, and the nucleic acid can be sequenced or otherwise determined to determine the history of droplet 10.

In some embodiments, two or more droplets may be treated in such a fashion, as is shown in Fig. 1B. For instance, two or more droplets may be exposed to a variety of different conditions, where each time a droplet is exposed to a condition, a nucleic acid encoding the condition is added to the droplet. However, even if such droplets with different histories are later combined (e.g., in a mixture), as is shown in container 20, the conditions each of the droplets was exposed to is still determinable through the different nucleic acids contained in each droplet. This is true even if the droplets themselves are burst and/or the contents are mixed together. For example, as is shown in Fig. 1B, by determining nucleic acids 1-2-3, 4-5-6, and 7-8-9, one knows that at least one droplet in container 20 was exposed to conditions A, B, and C, at least one droplet was exposed to conditions I, J, and K, and at least one droplet was exposed to conditions X, Y, and Z. In addition, one may also be able to determine that no droplets in container 20 were exposed to other sets of conditions. For example, one may be able to determine that no droplet was exposed to conditions X, Y, and K, as there is no sequence 1-2-9 present in container 20.

As is shown in Fig. 1C, in certain embodiments of the invention, testing and/or sorting of the droplets may also occur. As shown in this figure, a plurality of droplets exposed to different conditions (as encoded by the nucleic acids contained therein, represented by various numbers) may be sorted based on some criteria. For example, if the droplet further contain cells, then a property of the cell (e.g., whether the cell is alive or dead) may be used to sort the droplets into 2 (or more) populations. Each population can then be separately analyzed, e.g., by determining the nucleic acids contained therein, to determine which conditions may have led to the different populations. It should be noted that although cells are used here, this is by way of example only, and that other species may be determined, instead of or in addition to cells. In the example of Fig. 1C, a population of droplets 30 was sorted into a first group (A) and a second group (B), whether using cells or other suitable species. The nucleic acids in each group can then be sequenced. Thus, for example, it may be determined that the members of Group A each have conditions 2 and 3 in common, while the members of Group B do not have conditions 2 and 3 in common (although conditions 2 or 3 may be separately present in some of the Group B droplets). Thus, these sorting experiments would demonstrate that the combination of conditions 2 and 3 is necessary for some effect to occur (Group A), and if both are not present, the effect does not occur (Group B).

Also noteworthy is that in Fig. 1C, the number of conditions each droplet is exposed to is not necessarily the same; instead, the conditions may vary, i.e., intentionally, or due to experimental errors or uncertainty. For example, droplet 2-4 was exposed to conditions 2 and 4, while droplet 1-2-3-6 was exposed to conditions 1, 2, 3, and 6. Furthermore, it should also be noted that, due to the joining of the nucleic acids prior to removing the nucleic acids from the droplets, the conditions each droplet was exposed to may be separately maintained and analyzed, even if the droplets and/or the nucleic acids are subsequently mixed together. Thus, with reference to Fig. 1C, if the nucleic acids were not joined together, then each of Groups A and B would appear to be identical, since each group contains the same numbers (representing conditions) in exactly the same proportions in this illustrative example.

Thus, one surprising feature of certain embodiments of the invention is that the joined nucleic acids allow more complex conditions to be readily determined, for example a single condition or multiple conditions. This can be accomplished without loss of information even if the droplets are burst or otherwise combined together, e.g., for ease of processing or analysis. In contrast, in techniques in which various tags are separately introduced into droplets, without combining the tags together, the conditions cannot be so readily analyzed; instead, care must be taken in keeping each of the droplets separate, as any tags that are accidently combined (for example by bursting or fusing different droplets together) would result in loss of information.

The above discussion is not intended to be limiting; other embodiments of the invention are also possible for tagging droplets, as will now be discussed. For instance, various aspects of the present description are generally directed to systems and methods for tagging or identifying droplets within microfluidic devices, e.g., using nucleic acids and other "tags," that can be bound together. By binding the tags together, information about the droplet containing the tag may be retained, e.g., even after the tag is separated from the droplet and/or combined with other, different tags. Thus, for example, a plurality of tags from different droplets (e.g., exposed to different conditions) may be combined and analyzed together.

Certain aspects of the present description involve the use of a plurality of droplets or other discrete entities or compartments, e.g., where the contents of the entities are not readily mixed with the contents of other entities. For example, the discrete entities may be droplets contained within a carrying fluid, microwells of a microwell plate, individual spots on a slide or other surface, or the like. As discussed herein, each of the entities may a specific location that can contain one or more tags or other species, without accidental mixing with other entities. The entities may be relatively small in some cases, for example, each entity may have a volume of less than about 1 ml, less than about 300 microliters, less than about 100 microliters, less than about 30 microliters, less than about 10 microliters, less than about 3 microliters, less than about 1 microliter, less than about 500 nl, less than about 300 nl, less than about 100 nl, less than about 50 nl, less than about 30 nl, or less than about 10 nl.

In some embodiments, the droplet or other entity may contain various species, e.g., cells, chemicals, or the like. Other examples of species are discussed herein. For example, if the droplet or other entity contains one or more cells, the cells may be substantially identical or different. For example, a droplet or other entity may contain more than one cell or other species), where the cells (or other species) are the same or different; the cells (or other species) in different droplets or entities may also be the same or different. If cells are used, the cells may also be, in some embodiments, from a specific population of cells, such as from a certain organ or tissue (e.g., cardiac cells, immune cells, muscle cells, cancer cells, etc.), cells from a specific individual or species (e.g., human cells, mouse cells, bacteria, etc.), cells from different organisms, cells from a naturally-occurring sample (e.g., pond water, soil, etc.), or the like.

Thus, as non-limiting examples, the effects of one or more conditions on a specific type of cell (or specific types of cells) may be studied. As another example, the effects of a certain specific condition (or conditions) on a suitable population of cells may be studied. It addition, it should be noted that the present invention is not limited to only study of cells. In other embodiments, for example, the droplets or other entities may contain species in which a variety of conditions is to be applied. For example, the species may be a chemical reagent, e.g., one that is biological or nonbiological, organic or inorganic, etc. For instance, the species may be a polymer, a nucleic acid, a protein, a drug, a small molecular compound (e.g., having a molecular weight of less than about 1000 Da or less than about 2000 Da), an antibody, an enzyme, a peptide, or the like. Other examples of species are discussed in more detail below.

One or more tags may be present within a droplet (or other entity), which can be analyzed to determine the identity and/or history of the droplet. In some cases, the tags may be chosen to be relatively inert relative to other components of the droplet or other entity. The tags may be present initially in the droplet or other entity, and/or subsequently added, e.g., using processes such as those described below. For instance, tags may be added when the droplet or other entity is exposed to one or more conditions (or proximate in time to such exposure). In some cases, more than one tag may be present in a droplet or other entity.

In certain embodiments of the invention, the tags within a droplet or other entity can be joined together, for example, chemically, to produce a joined tag. Any suitable technique may be used to join tags together, e.g., prior to removal from the droplet or entity. The tags may be joined using any suitable technique. For example, the tags may be joined together using an enzyme, a catalyst, or a reactant, which may be added to the droplet or other entity using any suitable technique. For instance, a droplet containing the tags may be fused to another droplet containing the chemical agent, or a chemical reactant may be added or inserted into a droplet or other entity, for example, using pipetting or other techniques, and in some cases, using automated techniques.

By joining the tags in a droplet (or other entity) together to produce a joined tag, the identity and/or history of the droplet may be maintained by maintaining the joined tags, even if the tags are separated from the droplet or tags from different droplets are mixed together. For example, joined tags from a variety of droplets or entities can be collected together and analyzed. In some embodiments, a series of droplets or other entities may be separated into various groups depending on various properties, and the tags within each group may be manipulated together and/or used to identify such droplets or entities having such properties.

The tags may include, for example, nucleic acids, which may be joined together. In one set of embodiments, the nucleic acids may be joined together using enzymes. For instance, in certain embodiments, the nucleic acids together are joined together using ligases. Non-limiting examples of ligases include DNA ligases such as DNA Ligase I, DNA Ligase II, DNA Ligase III, DNA Ligase IV, T4 DNA ligase, T7 DNA ligase, T3 DNA Ligase, *E. coli* DNA Ligase, Taq DNA Ligase, or the like. Many such ligases may be purchased commercially. As additional examples, in some embodiments, two or more nucleic acids may be ligated together using annealing or a primer extension method.

In yet another set of embodiments, the nucleic acids may be joined together and or amplified using PCR (polymerase chain reaction) or other amplification techniques. Typically, in PCR reactions, the nucleic acids are heated to cause dissociation of the nucleic acids into single strands, and a heat-stable DNA polymerase (such as Taq polymerase) is used to amplify the nucleic acid. This process is often repeated multiple times to amplify the nucleic acids.

In one set of embodiments, the PCR may be performed within the droplets. For example, the droplets may contain a polymerase (such as Taq polymerase), and DNA nucleotides, and the droplets may be processed (e.g., via repeated heated and cooling) to amplify the nucleic acid within the droplets. The polymerase and nucleotides may be added at any suitable point, e.g., before, during, or after various nucleic acids encoding various conditions are added to the droplets. For instance, as a non-limiting example, droplet 15 in Fig. 1A may contain polymerase and DNA nucleotides, which is fused to droplet 10 to allow amplification to occur. Those of ordinary skill in the art will be aware of suitable PCR techniques and variations, such as assembly PCR or polymerase cycling assembly, which may be used in some embodiments to produce an amplified nucleic acid. Non-limiting examples of such procedures are also discussed below. In addition, in some cases, suitable primers may be used to initiate polymerization, e.g., P5 primer, P7 primer, PE1 primer, PE2 primer, A19 primer, or other primers known to those of ordinary skill in the art. In some embodiments, primers may be added to the droplets, or the primers may be present on one or more of the nucleic acids within the droplets. Those of ordinary skill in the art will be aware of suitable primers, many of which can be readily obtained commercially.

Typically, a primer is a single-stranded or partially double-stranded nucleic acid (e.g., DNA) that serves as a starting point for nucleic acid synthesis, allowing polymerase enzymes such as nucleic acid polymerase to extend the primer and replicate the complementary strand. A primer may be complementary to and to hybridize to a target nucleic acid. In some embodiments, a primer is a synthetic primer. In some embodiments, a primer is a non-naturally-occurring primer. A primer typically has a length of 10 to 50 nucleotides. For example, a primer may have a length of 10 to 40, 10 to 30, 10 to 20, 25 to 50, 15 to 40, 15 to 30, 20 to 50, 20 to 40, or 20 to 30 nucleotides. In some embodiments, a primer has a length of 18 to 24 nucleotides.

Other non-limiting examples of amplification methods known to those of ordinary skill in the art that may be used include, but are not limited to, reverse transcriptase (RT) PCR amplification, in vitro transcription amplification (IVT), multiple displacement amplification (MDA), or quantitative real-time PCR (qPCR).

By amplifying the nucleic acids within the droplets, e.g., prior to releasing the nucleic acids from the droplets, "even" amplification of the various nucleic acids may be achieved in some embodiments of the invention. Generally, in "even" amplification, approximately the same amount of nucleic acids may be produced within each droplet. In contrast, if a variety of nucleic acids are mixed together then amplified, differences in reaction rate between the various nucleic acids during PCR may result in some nucleic acids being amplified over other nucleic acids. However, according to certain embodiments, the nucleic acids within a plurality of droplets may be amplified such that the number of nucleic acid molecules for each type of nucleic acid may have a distribution such that no more than about 5%, no more than about 2%, or no more than about 1% of the nucleic acids have a number less than about 90% (or less than about 95%, or less than about 99%) and/or greater than about 110% (or greater than about 105%, or greater than about 101%) of the overall average number of nucleic acid molecules per droplet.

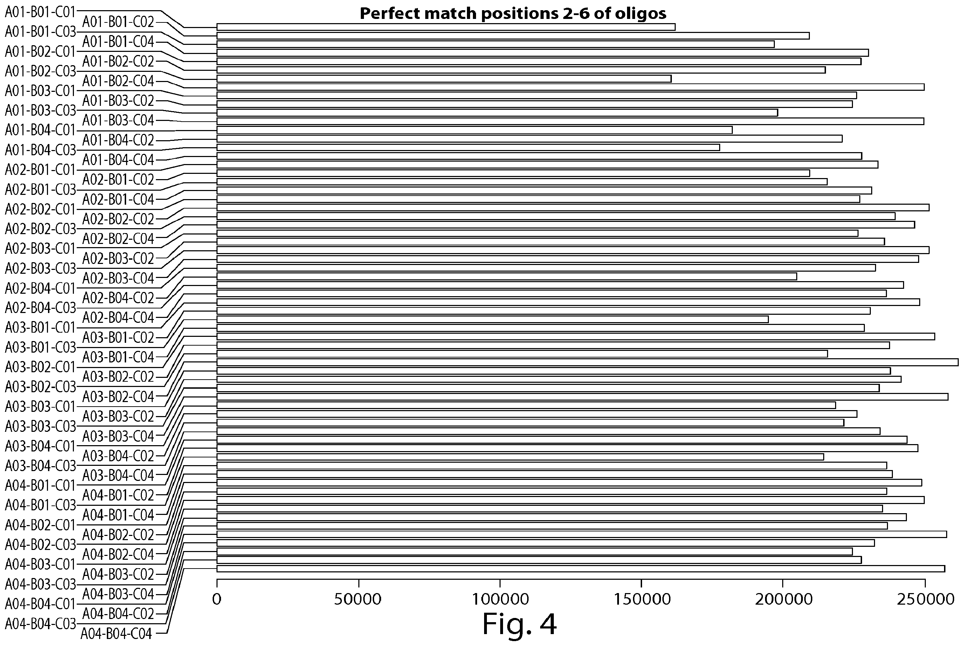

In some embodiments, a population of droplets may have at least about 50,000, at least about 100,000, at least about 150,000, at least about 200,000, at least about 250,000, at least about 300,000, at least about 400,000, at least about 500,000, at least about 750,000, at least about 1,000,000 or more molecules of the amplified nucleic acid per droplet. See, e.g., Fig. 4 for an example of a population of nucleic acid molecules that have been evenly amplified within droplets.

As discussed herein, various sequences of nucleic acids can be used to encode specific conditions that a droplet or other entity may be exposed to, and such nucleic acids can be added thereto to indicate such exposure to a condition, in accordance with certain embodiments. In some cases, the nucleic acids within a droplet or other entity may be joined together prior to removal (for example, upon bursting of a droplet, washing of a slide, etc.). Different nucleic acids from different droplets or entities may be mixed together; however, even after such mixing, each nucleic acid can be individually sequenced to determine the specific conditions that the corresponding droplet or entity had been exposed to.

Any suitable system may be used for encoding. For example, in one set of embodiments, a nucleic acid tag may include an encoding region of nucleotides, and optionally a connecting region. The nucleotides in the encoding region may correspond to a specific condition (or set of conditions). Any suitable number of conditions may be arbitrarily encoded in such a fashion, where the number of conditions that are encodable by such an encoding region may be determined by the number of nucleotides in the encoding region. Thus, for instance, an encoding region having length *n* can encode up to 4*ⁿ* regions (based on the four types of nucleotides). For example, a first condition may be encoded with A, a second condition may be encoded with T (or U if the nucleic acid is an RNA), a third condition may be encoded with G, a fourth condition may be encoded with C, etc. As a more complex example, an encoding region containing 3 nucleotides is sufficient to encode over 50 different conditions (since 4³ = 64). One or more than one encoding region may be used. In addition, the encoding region may also include other nucleotides used for error detection and/or correction, redundancy, or the like, in certain embodiments.

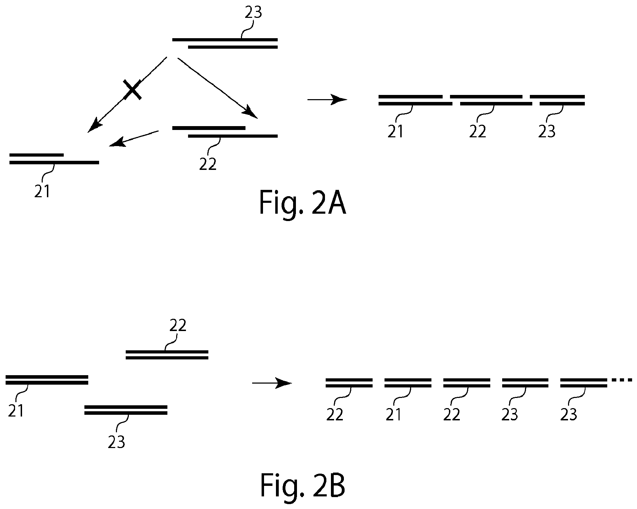

A nucleic acid tag may also include, in some cases, one or more connecting regions, which are joined together. For example, the connecting regions may include "sticky ends," or overhangs of nucleic acids, such that only specific nucleic acids can be properly joined together. For instance, as is shown in Fig. 2A, a first nucleic acid tag 21 (encoding a first condition) may include a first sticky end that is substantially complementary to a sticky end on second nucleic acid tag 22 but not to a sticky end on third nucleic acid tag 23; similarly, second nucleic acid 22 (encoding a second condition) may include a sticky end that is substantially complementary to a sticky end on third nucleic acid tag 23 (encoding a third condition) but not to the sticky end on first nucleic acid 21. Thus, upon exposure to suitable ligases, the first, second, and third nucleic acids may be joined together in an order suitable for subsequent study, without the nucleic acids being incorrectly joined together in an incorrect order (e.g., a first nucleic acid being joined to another first nucleic acid). Accordingly, by sequencing the final joined nucleic acid, it can be determined that this nucleic acid was in a droplet or other entity exposed to the first, second, and third conditions.

However, it should be understood that in other embodiments, there may be no need to ensure that the nucleic acid tags are joined together in a certain configuration or order. For example, as is shown in Fig. 2B, nucleic acids 21, 22, and 23 may be joined together in any suitable order; and the resulting nucleic acid may be analyzed to determine that that the nucleic acid encoded the conditions encoded by nucleic acids 21, 22, and 23.

The nucleic acid tag may also have any suitable length or number of nucleotides, depending on the application. For example, a nucleic acid tag may have a length shorter or longer than 10 nt, 30 nt, 50 nt, 100 nt, 300 nt, 500 nt, 1000 nt, 3000 nt, 5000 nt, or 10,000 nt, etc. In some cases, other portions of the nucleic acid tag may also be used for other purposes, e.g., in addition to encoding conditions. For example, portions of the nucleic acid tag may be used to increase the bulk of the nucleic acid tag (e.g., using specific sequences or nonsense sequences), to facilitate handling (for example, a tag may include a poly-A tail), to increase selectivity of binding (e.g., as discussed below), to facilitate recognition by an enzyme (e.g., a suitable ligase), to facilitate identification, or the like. The nucleic acid tag may comprise DNA, RNA, and/or other nucleic acids such as PNA, and/or combinations of these and/or other nucleic acids. In some cases, the nucleic acid tag is single stranded, although it may be double stranded in other cases.

In some cases, the nucleic acid tag may have a length of at least about 10 nt, at least about 30 nt, at least about 50 nt, at least about 100 nt, at least about 300 nt, at least about 500 nt, at least about 1000 nt, at least about 3000 nt, at least about 5000 nt, at least about 10,000 nt, etc. In some cases, the nucleic acid tag may have a length of no more than about 10,000 nt, no more than about 5000 nt, no more than about 3000 nt, no more than about 1000 nt, no more than about 500 nt, no more than about 300 nt, no more than about 100 nt, no more than about 50 nt, etc. Combinations of any of these are also possible, e.g., the nucleic acid tag may be between about 10 nt and about 100 nt. The length of the nucleic acid tag is not critical, and a variety of lengths may be used in various embodiments.

As mentioned, in certain aspects of the description,one or more conditions may be encoded using such tags, e.g., added to droplets or other entities exposed to such conditions. Thus, for example, a droplet may be exposed to a first condition and a first tag added to the droplet, then the droplet may be exposed to a second condition and a second tag added to the droplet, then the droplet may be exposed to a third condition and a third tag added to the droplet, then the droplet may be exposed to a fourth condition and a fourth tag added to the droplet, etc. Accordingly, any number of conditions may be present, e.g., the droplet or other entity may be exposed to 2 3, 4, 5, 6, 7, 8, 9, 10, or more conditions. The tags may also be combined together (e.g., joined together) to produce a joined tag that encodes the history and/or identity of the droplet or entity, as previously discussed.

Any suitable conditions may be encoded by the tags. For example, in one set of embodiments, the identity of the droplet or other entity may be encoded using one or more tags. For example, each droplet or entity may be assigned a unique tag, or a unique combination of tags. As another example, the condition may be a species that a droplet or other entity is exposed to, internally and/or externally. Any of a wide variety of species may be encoded by a suitable tag. For example, the species may be a drug (or a suspected drug), a cell, a polymer, a peptide, a protein, an enzyme, a hormone, an antibiotic, a vitamin, a carbohydrate, a sugar, an antibody, a reagent, a gas, a dye, an ion, a virus, a bacterium, pH level (e.g., acidic or basic conditions), or the like. Thus, for example, a plurality of cells, or a plurality of cell types may be encoded using a unique tag, or a unique combination of tags. Any number of tags may be used to encode such conditions, depending on the application. For example, there may be at least 10, at least 30, at least 50, at least 100, at least 300, at least 500, at least 1,000, at least 3,000, at least 5,000, at least 10,000, at least 30,000, at least 50,000, at least 100,000, or more unique tags, e.g., substantially encoding a like number of suitable conditions such as any of those described herein. For instance, as a first condition, a library of droplets, containing a plurality of cells or cell types, may be encoded such that substantially each droplet contains one cell or one cell type, and an associated tag, such as a nucleic acid.

In addition, the condition may also be an external physical condition in some instances. For example, the droplet or other entity may be exposed to an external physical condition such as a certain or predetermined temperature, pressure, electrical condition (e.g., current, voltage, etc.), etc. and a suitable tag may be introduced into the droplet or entity before, during, or after exposure to the external physical condition. As yet another example, the condition may be a processing condition, for example, filtration, sedimentation, centrifugation, etc.

In one set of embodiments, the condition may be a condition used to lyse cells that may be contained within the droplets. For example, the condition could be exposure to a lysing chemical (e.g., pure water, a surfactant such as Triton-X or SDS, an enzyme such as lysozyme, lysostaphin, zymolase, cellulase, mutanolysin, glycanases, proteases, mannase, proteinase K, etc.), or a physical condition (e.g., ultrasound, ultraviolet light, mechanical agitation, etc.). In some cases, lysing a cell will cause the cell to release its contents, e.g., cellular nucleic acids, proteins, enzymes, sugars, etc. In some embodiments some of the cellular nucleic acids may also be joined to one or more nucleic acids contained within the droplet. For example, in one set of embodiments, RNA transcripts typically produced within the cells may be released and then joined to the nucleic acid tags.

Combinations of any of these and/or other conditions are also contemplated. For example, as noted above, a droplet may be exposed to a first condition encoded by a first tag, a second condition encoded by a second tag, a third condition encoded by a third tag, etc. The conditions may be the same or different. In addition, a droplet or other entity may be exposed to any number of conditions (e.g. 1, 2, 3, 4, 5, 6, etc.), and different droplets or entities need not be exposed to the same conditions, or the same number of conditions. In addition, in some embodiments, a plurality of droplets (or other entities) may be randomly exposed to a plurality of conditions, e.g., such that not all droplets are exposed to all conditions. As noted above, when a droplet or other entity is exposed to a condition, a nucleic acid encoding the condition may be added to the droplet or entity, such that the specific history of the droplet or entity need not be predetermined, and can be randomly determined. For instance, a plurality of initial, substantially identical droplets (or other entities) may be exposed to a plurality of second droplets containing different species (and/or one or more species at different concentrations), such that each initial droplet is randomly fused to one or more second droplets.

In addition, in some embodiments, droplets or other entities may be separated or sorted into various groups depending on various properties, and the tags within each group may be manipulated together and/or used to identify such droplet or entities having such properties. Thus, as non-limiting examples, a droplet (or other entity) may be sorted into a first group or a second group depending on whether a reaction occurred within the droplet or not, the droplet may be sorted based on a property of a cell or other species contained within the droplet, etc. The sorting may also occur into two or more groups.

In some cases, some or all of the groups of droplets (or other entities) may be analyzed, e.g., using the tags contained therein, to determine characteristics of the droplets or entities within those groups, and/or to determine characteristics of the droplets or entities within a group that separates that group from other groups. For instance, members of a group may have, in common, one or more tags, and/or one or more specific combinations of tags, that separates those group members from other group members, e.g., as was explained above with respect to Fig. 1C. Such tags may be used, for example, to identify or distinguish one or more conditions that result in a particular outcome from conditions that do not result in that outcome.

Thus, in one set of embodiments, a property of a droplet or other entity is determined, and the droplet or other entity is sorted based on that property. The property may also be a property of a species contained within the droplet or entity, such as a cell. The property may be any physical or chemical property that can be determined. For instance, properties such as fluorescence, transparency, density, size, volume, etc., of a droplet or other entity (or species contained therein) may be determined, and used for sorting purposes. In some cases, one or more reagents may also be added to the droplets or other entities, e.g., to start or facilitate a reaction that can be determined, e.g., for sorting purposes.

In some cases, the droplets or other entities may be burst or disrupted in order to access the tags. This may occur at any suitable time, e.g., before or after joining of the tags together. For example, droplets contained in a carrying fluid may be disrupted using techniques such as mechanical disruption or ultrasound. Similarly, entities on a surface or droplets may be disrupted using exposure to chemical agents or surfactants (for example, 1H,1H,2H,2H-perfluorooctanol), or washed or rinsed to collect the tags.

The tags may then be determined to determine the identity and/or history of the droplet (or other entity), e.g., to determine conditions that the droplet or other entity was exposed to. Any suitable method can be used to determine the tags, depending on the type of tags used. For example, fluorescent particles may be determined using fluorescence measurements, or nucleic acids may be sequenced using a variety of techniques and instruments, many of which are readily available commercially. Examples of such techniques include, but are not limited to, chain-termination sequencing, sequencing-by-hybridization, Maxam-Gilbert sequencing, dye-terminator sequencing, chain-termination methods, Massively Parallel Signature Sequencing (Lynx Therapeutics), polony sequencing, pyrosequencing, sequencing by ligation, ion semiconductor sequencing, DNA nanoball sequencing, single-molecule real-time sequencing, nanopore sequencing, microfluidic Sanger sequencing, digital RNA sequencing ("digital RNA-seq"), etc.

In addition, in some cases, one or more other species may be associated with the tags, e.g., covalently bound or otherwise joined thereto. For example, as previously discussed, in some cases, nucleic acids released by a lysed cell may be joined to one or more tags. These may include, for example, chromosomal DNA, RNA transcripts, tRNA, mRNA, mitochondrial DNA, or the like. Such nucleic acids may be sequenced, in addition to sequencing the tags themselves, which may yield information about the nucleic acid profile of the cells, which can be associated with the tags, or the conditions that the corresponding droplet or cell was exposed to. Thus, as a non-limiting example, RNA transcripts from the cell may be joined to one or more tags, which may be sequenced and correlated with conditions that the corresponding droplet or cell to determine information such as apoptosis gene signatures, growth arrest signatures, immune signatures, metabolic gene sets, or expression of genes that confer susceptibility or resistance to other known agents, etc.

As another example, in some cases, the tags may be bound to particles, e.g., to facilitate identification, collection, or the like. For example, the tags may include a sequence or a moiety that can be used to bind to a particle. For instance, the tag may contain an acrylic phosphoramidite at its 5' end can be incorporated into a polyacrylamide mesh of certain particles during the polymerization process. As another example, acrydite-modified oligonucleotide tags can react covalently with thiol groups and thus, particles having thiol groups would bind acrydite-modified oligonucleotides. In another example, oligonucleotide tags having amino groups can be covalently bound to the carboxy group of certain particles. In yet another example, oligonucleotides with tag a biotin group can be attached to streptavidin-coated tags. In yet another example, the particle may include antibodies or antibody fragments able to recognize certain oligonucleotide sequences present on the tags. Therefore, different types of incorporation of nucleic acids into/onto the particles are possible.

The particle may be, in one set of embodiments, a hydrogel particle or a colloidal particle (polystyrene, magnetic or polymer particle, etc.). See, e.g., Int. Pat. Apl. Pub. No. WO 2008/109176, entitled "Assay and other reactions involving droplets" for examples of hydrogel particles, including hydrogel particles containing DNA. The microspheres may be porous in some embodiments. Examples of hydrogels include, but are not limited to agarose or acrylamide-based gels, such as polyacrylamide, poly-N-isopropylacrylamide, or poly N-isopropylpolyacrylamide. For example, an aqueous solution of a monomer may be dispersed in a droplet, and then polymerized, e.g., to form a gel. Another example is a hydrogel, such as alginic acid that can be gelled by the addition of calcium ions. In some cases, gelation initiators (ammonium persulfate and TEMED for acrylamide, or Ca²⁺ for alginate) can be added to a droplet, for example, by co-flow with the aqueous phase, by co-flow through the oil phase, or by coalescence of two different drops, e.g., as discussed in U.S. Patent Application Serial No. 11/360,845, filed February 23, 2006, entitled "Electronic Control of Fluidic Species," by Link, *et al.,* published as U.S. Patent Application Publication No. 2007/000342 on January 4, 2007; or in U.S. Patent Application Serial No. 11/698,298, filed January 24, 2007, entitled "Fluidic Droplet Coalescence," by Ahn, *et al.;*

In another set of embodiments, the particles may comprise one or more polymers. Exemplary polymers include, but are not limited to, polystyrene (PS), polycaprolactone (PCL), polyisoprene (PIP), poly(lactic acid), polyethylene, polypropylene, polyacrylonitrile, polyimide, polyamide, and/or mixtures and/or co-polymers of these and/or other polymers. In addition, in some cases, the particles may be magnetic, which could allow for the magnetic manipulation of the particles. For example, the particles may comprise iron or other magnetic materials. The particles could also be functionalized so that they could have other molecules attached, such as proteins, nucleic acids or small molecules. Thus, some embodiments of the present invention are directed to a set of particles defining a library of, for example, nucleic acids, proteins, small molecules, or other species such as those described herein. In some embodiments, the particle may be fluorescent.

The particle is a microparticle in certain aspects of the description. The particle may be of any of a wide variety of types; as discussed, the particle may be used to bind a particular oligonucleotide tag in a droplet, and any suitable particle to which oligonucleotide tags can associate with (e.g., physically or chemically) may be used. The exact form of the particle is not critical. The particle may be spherical or non-spherical, and may be formed of any suitable material. In some cases, a plurality of particles is used, which have substantially the same composition and/or substantially the same average diameter. The "average diameter" of a plurality or series of particles is the arithmetic average of the average diameters of each of the particles. Those of ordinary skill in the art will be able to determine the average diameter (or other characteristic dimension) of a plurality or series of particles, for example, using laser light scattering, microscopic examination, or other known techniques. The average diameter of a single particle, in a non-spherical particle, is the diameter of a perfect sphere having the same volume as the non-spherical particle. The average diameter of a particle (and/or of a plurality or series of particles) may be, for example, less than about 1 mm, less than about 500 micrometers, less than about 200 micrometers, less than about 100 micrometers, less than about 75 micrometers, less than about 50 micrometers, less than about 25 micrometers, less than about 10 micrometers, or less than about 5 micrometers in some cases. The average diameter may also be at least about 1 micrometer, at least about 2 micrometers, at least about 3 micrometers, at least about 5 micrometers, at least about 10 micrometers, at least about 15 micrometers, or at least about 20 micrometers in certain cases.

Tags such as those described herein may be used in a variety of situations, for example, to screen or bioprospect for new drugs or other treatments, as a high-throughput screening technique, to study the effect various exposure conditions have on cells, or the like. In addition, it should be understood that the invention is not limited to only cell studies. For example, a chemical contained within a droplet may be exposed to a variety of different conditions (e.g., potential reactants or catalysts, reaction conditions, initiators, etc.) and the different conditions tagged for determining or optimizing the reactions that the chemical is able to participate in. In some cases, the chemical may be a biologically active chemical (e.g., a drug, a protein, a polymer, a carbohydrate, etc.), although in other cases, the chemical need not be biologically relevant. For example, the chemical may be a monomer or a polymer, a catalyst, a semiconductor material, etc.

As a non-limiting example, in one set of embodiments, a plurality of substantially identical cells can be exposed to a variety of substances (e.g., other cells, chemical compounds, soil samples, naturally-occurring samples, etc.), optionally under various physical conditions (e.g., various temperatures), to determine whether any of the substances have a beneficial or a detrimental effect on the cells. For example, the substantially identical cells may be cancer cells that are screened against a panel of substances to identify those substances that, alone or in combination, have anti-cancer or anti-tumor properties, or the substantially identical cells may be immune or other cells to which a certain activity is desired. The panel may include, for example, naturally-occurring compounds, synthetic compounds, compounds from a library, compounds sharing certain properties, etc. In some cases, the compounds may also be present at more than one concentration. Each member of the panel may be tagged as discussed herein, such that, upon exposure of a cell in a droplet (or other entity) to a panel member, an appropriate corresponding tag is added. The tags may also be joined together. Thus, for example, the substantially identical cells may be exposed to a variety of substances, in various combinations, with subsequent sorting of live cells from dead cells; the tags from the droplets containing the live cells (and/or the dead cells) may be separated and sequenced as discussed herein to determine those conditions or panel members which were able to kill the cells. In addition, the invention is not limited to only substantially identical cells. For example, in another set of embodiments, droplets containing different cells (or other species, e.g., chemicals) may be used.

In some aspects of the description, cells may be contained within gels to protect the cells during manipulation of the cells. For example, one or more cells may be contained within gel particles, which can then be exposed to various conditions and/or tags, as discussed herein. Thus, for example, a cell contained within a gel may be exposed to a first condition, a second condition, etc., and one or more nucleic acids encoding the conditions may be associated with the gel. As a non-limiting example, a gel containing a cell may be contained within a microfluidic droplet such as discussed herein, and the microfluidic droplet may then be exposed to a variety of conditions, along with nucleic acids encoding such conditions. In some embodiments, additional agents may also be added to the gels. For example, magnetic particles may be added to some or all of the gels, e.g., to facilitate later sorting. As another example, fluorescent agents may be added to some or all of the gels, e.g., to facilitate identification of certain cells (or other species to be determined) that are contained within the gels.

As a non-limiting example, in one set of embodiments, one or more cells may be contained within a gel particle, which may be contained within a droplet. The droplet (containing the gel particle) may be exposed to a variety of conditions (for example, a species such as a drug), and each time the droplet is exposed to a condition, a nucleic acid encoding the condition is added to the droplet. In some cases, after exposure, a condition of the cells may be determined (for example sorting of live cells from dead cells), and in some cases, the sorting may be facilitated using microfluidic techniques, FACS, magnetic sorting (e.g., using magnetic beads), centrifugation, or other sorting or separation techniques known to those of ordinary skill in the art. In one set of embodiments, the gel is an agarose gel. Other examples of gels include acrylamide-based gels, such as polyacrylamide or poly N-isopropylpolyacrylamide, or hydrogels such as alginic acid that can be gelled by the addition of gelation initiators (for instance, ammonium persulfate and TEMED for acrylamide, or Ca²⁺ for alginate, etc.). Other examples of gels encapsulating cells can be seen in Int. Pat. Apl. Pub. No. WO 2008/109176.

In some embodiments, the gel may be one that is low-melt, e.g., the agarose may melt at temperatures below about 100 °C, below about 80 °C, below about 70 °C, or below about 60 °C in some cases. In some cases, the gel may be chosen to be able to solidify upon exposure to relatively low temperatures, e.g., below about 60 °C, below about 50 °C, below about 40 °C, below about 35 °C, below 30 °C, below about 25 °C, or below about 20 °C.

In some embodiments, as non-limiting examples, the average diameter of the gel may be less than about 1 mm, less than about 500 micrometers, less than about 300 micrometers, less than about 200 micrometers, less than about 100 micrometers, less than about 75 micrometers, less than about 50 micrometers, less than about 30 micrometers, less than about 25 micrometers, less than about 20 micrometers, less than about 15 micrometers, less than about 10 micrometers, less than about 5 micrometers, less than about 3 micrometers, less than about 2 micrometers, less than about 1 micrometer, less than about 500 nm, less than about 300 nm, less than about 100 nm, or less than about 50 nm. The average diameter of the gel may also be at least about 30 nm, at least about 50 nm, at least about 100 nm, at least about 300 nm, at least about 500 nm, at least about 1 micrometer, at least about 2 micrometers, at least about 3 micrometers, at least about 5 micrometers, at least about 10 micrometers, at least about 15 micrometers, or at least about 20 micrometers in certain cases. Combinations of these are also possible, e.g., the gel may have an average diameter of between about 25 micrometers and about 100 micrometers. In some embodiments, a gel may be prepared by containing a cell (or other suitable species) within a microfluidic droplet with a gel precursor. For example, the gel may contain agarose which can form a gel upon exposure to a suitable temperature, or some gels may be prepared by containing a precursor such as disclosed herein and exposing the precursor to a suitable gelation initiator.

Additional details regarding systems and methods for manipulating droplets in a microfluidic system follow, e.g., for determining droplets (or species within droplets), sorting droplets, etc. For example, various systems and methods for screening and/or sorting droplets are described in U.S. Patent Application Serial No. 11/360,845, filed February 23, 2006, entitled "Electronic Control of Fluidic Species," by Link, et al., published as U.S. Patent Application Publication No. 2007/000342 on January 4, 2007_{;} As a non-limiting example, by applying (or removing) a first electric field (or a portion thereof), a droplet may be directed to a first region or channel; by applying (or removing) a second electric field to the device (or a portion thereof), the droplet may be directed to a second region or channel; by applying a third electric field to the device (or a portion thereof), the droplet may be directed to a third region or channel; etc., where the electric fields may differ in some way, for example, in intensity, direction, frequency, duration, etc.

In certain embodiments of the invention, sensors are provided that can sense and/or determine one or more characteristics of the fluidic droplets, and/or a characteristic of a portion of the fluidic system containing the fluidic droplet (e.g., the liquid surrounding the fluidic droplet) in such a manner as to allow the determination of one or more characteristics of the fluidic droplets. Characteristics determinable with respect to the droplet and usable in the invention can be identified by those of ordinary skill in the art. Non-limiting examples of such characteristics include fluorescence, spectroscopy (e.g., optical, infrared, ultraviolet, etc.), radioactivity, mass, volume, density, temperature, viscosity, pH, concentration of a substance, such as a biological substance (e.g., a protein, a nucleic acid, etc.), or the like.

In some cases, the sensor may be connected to a processor, which in turn, cause an operation to be performed on the fluidic droplet, for example, by sorting the droplet, adding or removing electric charge from the droplet, fusing the droplet with another droplet, splitting the droplet, causing mixing to occur within the droplet, etc., for example, as previously described. For instance, in response to a sensor measurement of a fluidic droplet, a processor may cause the fluidic droplet to be split, merged with a second fluidic droplet, etc.

One or more sensors and/or processors may be positioned to be in sensing communication with the fluidic droplet. "Sensing communication," as used herein, means that the sensor may be positioned anywhere such that the fluidic droplet within the fluidic system (e.g., within a channel), and/or a portion of the fluidic system containing the fluidic droplet may be sensed and/or determined in some fashion. For example, the sensor may be in sensing communication with the fluidic droplet and/or the portion of the fluidic system containing the fluidic droplet fluidly, optically or visually, thermally, pneumatically, electronically, or the like. The sensor can be positioned proximate the fluidic system, for example, embedded within or integrally connected to a wall of a channel, or positioned separately from the fluidic system but with physical, electrical, and/or optical communication with the fluidic system so as to be able to sense and/or determine the fluidic droplet and/or a portion of the fluidic system containing the fluidic droplet (e.g., a channel or a microchannel, a liquid containing the fluidic droplet, etc.). For example, a sensor may be free of any physical connection with a channel containing a droplet, but may be positioned so as to detect electromagnetic radiation arising from the droplet or the fluidic system, such as infrared, ultraviolet, or visible light. The electromagnetic radiation may be produced by the droplet, and/or may arise from other portions of the fluidic system (or externally of the fluidic system) and interact with the fluidic droplet and/or the portion of the fluidic system containing the fluidic droplet in such as a manner as to indicate one or more characteristics of the fluidic droplet, for example, through absorption, reflection, diffraction, refraction, fluorescence, phosphorescence, changes in polarity, phase changes, changes with respect to time, etc. As an example, a laser may be directed towards the fluidic droplet and/or the liquid surrounding the fluidic droplet, and the fluorescence of the fluidic droplet and/or the surrounding liquid may be determined. "Sensing communication," as used herein may also be direct or indirect. As an example, light from the fluidic droplet may be directed to a sensor, or directed first through a fiber optic system, a waveguide, etc., before being directed to a sensor.

Non-limiting examples of sensors useful in the invention include optical or electromagnetically-based systems. For example, the sensor may be a fluorescence sensor (e.g., stimulated by a laser), a microscopy system (which may include a camera or other recording device), or the like. As another example, the sensor may be an electronic sensor, e.g., a sensor able to determine an electric field or other electrical characteristic. For example, the sensor may detect capacitance, inductance, etc., of a fluidic droplet and/or the portion of the fluidic system containing the fluidic droplet.

As used herein, a "processor" or a "microprocessor" is any component or device able to receive a signal from one or more sensors, store the signal, and/or direct one or more responses (e.g., as described above), for example, by using a mathematical formula or an electronic or computational circuit. The signal may be any suitable signal indicative of the environmental factor determined by the sensor, for example a pneumatic signal, an electronic signal, an optical signal, a mechanical signal, etc.

In one set of embodiments, a fluidic droplet may be directed by creating an electric charge and/or an electric dipole on the droplet, and steering the droplet using an applied electric field, which may be an AC field, a DC field, etc. As an example, an electric field may be selectively applied and removed (or a different electric field may be applied, e.g., a reversed electric field) as needed to direct the fluidic droplet to a particular region. The electric field may be selectively applied and removed as needed, in some embodiments, without substantially altering the flow of the liquid containing the fluidic droplet. For example, a liquid may flow on a substantially steady-state basis (i.e., the average flowrate of the liquid containing the fluidic droplet deviates by less than 20% or less than 15% of the steady-state flow or the expected value of the flow of liquid with respect to time, and in some cases, the average flowrate may deviate less than 10% or less than 5%) or other predetermined basis through a fluidic system of the description (e.g., through a channel or a microchannel), and fluidic droplets contained within the liquid may be directed to various regions, e.g., using an electric field, without substantially altering the flow of the liquid through the fluidic system.

In some embodiments, the fluidic droplets may be screened or sorted within a fluidic system of the description by altering the flow of the liquid containing the droplets. For instance, in one set of embodiments, a fluidic droplet may be steered or sorted by directing the liquid surrounding the fluidic droplet into a first channel, a second channel, etc.

In another set of embodiments, pressure within a fluidic system, for example, within different channels or within different portions of a channel, can be controlled to direct the flow of fluidic droplets. For example, a droplet can be directed toward a channel junction including multiple options for further direction of flow (e.g., directed toward a branch, or fork, in a channel defining optional downstream flow channels). Pressure within one or more of the optional downstream flow channels can be controlled to direct the droplet selectively into one of the channels, and changes in pressure can be effected on the order of the time required for successive droplets to reach the junction, such that the downstream flow path of each successive droplet can be independently controlled. In one arrangement, the expansion and/or contraction of liquid reservoirs may be used to steer or sort a fluidic droplet into a channel, e.g., by causing directed movement of the liquid containing the fluidic droplet. The liquid reservoirs may be positioned such that, when activated, the movement of liquid caused by the activated reservoirs causes the liquid to flow in a preferred direction, carrying the fluidic droplet in that preferred direction. For instance, the expansion of a liquid reservoir may cause a flow of liquid towards the reservoir, while the contraction of a liquid reservoir may cause a flow of liquid away from the reservoir. In some cases, the expansion and/or contraction of the liquid reservoir may be combined with other flow-controlling devices and methods, e.g., as described herein. Non-limiting examples of devices able to cause the expansion and/or contraction of a liquid reservoir include pistons and piezoelectric components. In some cases, piezoelectric components may be particularly useful due to their relatively rapid response times, e.g., in response to an electrical signal. In some embodiments, the fluidic droplets may be sorted into more than two channels.

As mentioned, certain embodiments are generally directed to systems and methods for sorting fluidic droplets in a liquid, and in some cases, at relatively high rates. For example, a property of a droplet may be sensed and/or determined in some fashion (e.g., as further described herein), then the droplet may be directed towards a particular region of the device, such as a microfluidic channel, for example, for sorting purposes. In some cases, high sorting speeds may be achievable using certain systems and methods of the description. For instance, at least about 10 droplets per second may be determined and/or sorted in some cases, and in other cases, at least about 20 droplets per second, at least about 30 droplets per second, at least about 100 droplets per second, at least about 200 droplets per second, at least about 300 droplets per second, at least about 500 droplets per second, at least about 750 droplets per second, at least about 1,000 droplets per second, at least about 1,500 droplets per second, at least about 2,000 droplets per second, at least about 3,000 droplets per second, at least about 5,000 droplets per second, at least about 7,500 droplets per second, at least about 10,000 droplets per second, at least about 15,000 droplets per second, at least about 20,000 droplets per second, at least about 30,000 droplets per second, at least about 50,000 droplets per second, at least about 75,000 droplets per second, at least about 100,000 droplets per second, at least about 150,000 droplets per second, at least about 200,000 droplets per second, at least about 300,000 droplets per second, at least about 500,000 droplets per second, at least about 750,000 droplets per second, at least about 1,000,000 droplets per second, at least about 1,500,000 droplets per second, at least about 2,000,000 or more droplets per second, or at least about 3,000,000 or more droplets per second may be determined and/or sorted.

In some aspects, a population of relatively small droplets may be used. In certain embodiments, as non-limiting examples, the average diameter of the droplets may be less than about 1 mm, less than about 500 micrometers, less than about 300 micrometers, less than about 200 micrometers, less than about 100 micrometers, less than about 75 micrometers, less than about 50 micrometers, less than about 30 micrometers, less than about 25 micrometers, less than about 20 micrometers, less than about 15 micrometers, less than about 10 micrometers, less than about 5 micrometers, less than about 3 micrometers, less than about 2 micrometers, less than about 1 micrometer, less than about 500 nm, less than about 300 nm, less than about 100 nm, or less than about 50 nm. The average diameter of the droplets may also be at least about 30 nm, at least about 50 nm, at least about 100 nm, at least about 300 nm, at least about 500 nm, at least about 1 micrometer, at least about 2 micrometers, at least about 3 micrometers, at least about 5 micrometers, at least about 10 micrometers, at least about 15 micrometers, or at least about 20 micrometers in certain cases. Combinations of these are also possible, e.g., the droplet may have an average diameter of between about 25 micrometers and about 100 micrometers. The "average diameter" of a population of droplets is the arithmetic average of the diameters of the droplets.

In some embodiments, the droplets may be of substantially the same shape and/or size (i.e., "monodisperse"), or of different shapes and/or sizes, depending on the particular application. In some cases, the droplets may have a homogenous distribution of cross-sectional diameters, i.e., the droplets may have a distribution of diameters such that no more than about 5%, no more than about 2%, or no more than about 1% of the droplets have a diameter less than about 90% (or less than about 95%, or less than about 99%) and/or greater than about 110% (or greater than about 105%, or greater than about 101%) of the overall average diameter of the plurality of droplets. Some techniques for producing homogenous distributions of cross-sectional diameters of droplets are disclosed in International Patent Application No. PCT/US2004/010903, filed April 9, 2004, entitled "Formation and Control of Fluidic Species," by Link et al., published as WO 2004/091763 on October 28, 2004.

Those of ordinary skill in the art will be able to determine the average diameter of a population of droplets, for example, using laser light scattering or other known techniques. The droplets so formed can be spherical, or non-spherical in certain cases. The diameter of a droplet, in a non-spherical droplet, may be taken as the diameter of a perfect mathematical sphere having the same volume as the non-spherical droplet.

In some embodiments, one or more droplets may be created within a channel by creating an electric charge on a fluid surrounded by a liquid, which may cause the fluid to separate into individual droplets within the liquid. In some embodiments, an electric field may be applied to the fluid to cause droplet formation to occur. The fluid can be present as a series of individual charged and/or electrically inducible droplets within the liquid. Electric charge may be created in the fluid within the liquid using any suitable technique, for example, by placing the fluid within an electric field (which may be AC, DC, etc.), and/or causing a reaction to occur that causes the fluid to have an electric charge.

The electric field, in some embodiments, is generated from an electric field generator, i.e., a device or system able to create an electric field that can be applied to the fluid. The electric field generator may produce an AC field (i.e., one that varies periodically with respect to time, for example, sinusoidally, sawtooth, square, etc.), a DC field (i.e., one that is constant with respect to time), a pulsed field, etc. Techniques for producing a suitable electric field (which may be AC, DC, etc.) are known to those of ordinary skill in the art. For example, in one embodiment, an electric field is produced by applying voltage across a pair of electrodes, which may be positioned proximate a channel such that at least a portion of the electric field interacts with the channel. The electrodes can be fashioned from any suitable electrode material or materials known to those of ordinary skill in the art, including, but not limited to, silver, gold, copper, carbon, platinum, copper, tungsten, tin, cadmium, nickel, indium tin oxide ("ITO"), etc., as well as combinations thereof.

In another set of embodiments, droplets of fluid can be created from a fluid surrounded by a liquid within a channel by altering the channel dimensions in a manner that is able to induce the fluid to form individual droplets. The channel may, for example, be a channel that expands relative to the direction of flow, e.g., such that the fluid does not adhere to the channel walls and forms individual droplets instead, or a channel that narrows relative to the direction of flow, e.g., such that the fluid is forced to coalesce into individual droplets. In some cases, the channel dimensions may be altered with respect to time (for example, mechanically or electromechanically, pneumatically, etc.) in such a manner as to cause the formation of individual droplets to occur. For example, the channel may be mechanically contracted ("squeezed") to cause droplet formation, or a fluid stream may be mechanically disrupted to cause droplet formation, for example, through the use of moving baffles, rotating blades, or the like.

Certain embodiments are generally directed to systems and methods for splitting a droplet into two or more droplets. For example, a droplet can be split using an applied electric field. The droplet may have a greater electrical conductivity than the surrounding liquid, and, in some cases, the droplet may be neutrally charged. In certain embodiments, in an applied electric field, electric charge may be urged to migrate from the interior of the droplet to the surface to be distributed thereon, which may thereby cancel the electric field experienced in the interior of the droplet. In some embodiments, the electric charge on the surface of the droplet may also experience a force due to the applied electric field, which causes charges having opposite polarities to migrate in opposite directions. The charge migration may, in some cases, cause the drop to be pulled apart into two separate droplets.

Some embodiments of the description generally relate to systems and methods for fusing or coalescing two or more droplets into one droplet, e.g., where the two or more droplets ordinarily are unable to fuse or coalesce, for example, due to composition, surface tension, droplet size, the presence or absence of surfactants, etc. In certain cases, the surface tension of the droplets, relative to the size of the droplets, may also prevent fusion or coalescence of the droplets from occurring.

As a non-limiting example, two droplets can be given opposite electric charges (i.e., positive and negative charges, not necessarily of the same magnitude), which can increase the electrical interaction of the two droplets such that fusion or coalescence of the droplets can occur due to their opposite electric charges. For instance, an electric field may be applied to the droplets, the droplets may be passed through a capacitor, a chemical reaction may cause the droplets to become charged, etc. The droplets, in some cases, may not be able to fuse even if a surfactant is applied to lower the surface tension of the droplets. However, if the droplets are electrically charged with opposite charges (which can be, but are not necessarily of, the same magnitude), the droplets may be able to fuse or coalesce. As another example, the droplets may not necessarily be given opposite electric charges (and, in some cases, may not be given any electric charge), and are fused through the use of dipoles induced in the droplets that causes the droplets to coalesce. Also, the two or more droplets allowed to coalesce are not necessarily required to meet "head-on." Any angle of contact, so long as at least some fusion of the droplets initially occurs, is sufficient. See also, e.g., U.S. Patent Application Serial No. 11/698,298, filed January 24, 2007, entitled "Fluidic Droplet Coalescence," by Ahn, *et al.,* published as U.S. Patent Application Publication No. 2007/0195127 on August 23, 2007,

In one set of embodiments, a fluid may be injected into a droplet. The fluid may be microinjected into the droplet in some cases, e.g., using a microneedle or other such device. In other cases, the fluid may be injected directly into a droplet using a fluidic channel as the droplet comes into contact with the fluidic channel. Other techniques of fluid injection are disclosed in, e.g., International Patent Application No. PCT/US2010/040006, filed June 25, 2010, entitled "Fluid Injection," by Weitz, *et al.,* published as WO 2010/151776 on December 29, 2010; or International Patent Application No. PCT/US2009/006649, filed December 18, 2009, entitled "Particle-Assisted Nucleic Acid Sequencing," by Weitz, *et al.,* published as WO 2010/080134 on July 15, 2010.

A variety of materials and methods, according to certain aspects of the description, can be used to form articles or components such as those described herein, e.g., channels such as microfluidic channels, chambers, etc. For example, various articles or components can be formed from solid materials, in which the channels can be formed via micromachining, film deposition processes such as spin coating and chemical vapor deposition, laser fabrication, photolithographic techniques, etching methods including wet chemical or plasma processes, and the like. See, for example, Scientific American, 248:44-55, 1983 (Angell, *et al).*

In one set of embodiments, various structures or components of the articles described herein can be formed of a polymer, for example, an elastomeric polymer such as polydimethylsiloxane ("PDMS"), polytetrafluoroethylene ("PTFE" or Teflon®), or the like. For instance, according to one embodiment, a microfluidic channel may be implemented by fabricating the fluidic system separately using PDMS or other soft lithography techniques (details of soft lithography techniques suitable for this embodiment are discussed in the references entitled "Soft Lithography," by Younan Xia and George M. Whitesides, published in the Annual Review of Material Science, 1998, Vol. 28, pages 153-184, and "Soft Lithography in Biology and Biochemistry," by George M. Whitesides, Emanuele Ostuni, Shuichi Takayama, Xingyu Jiang and Donald E. Ingber, published in the Annual Review of Biomedical Engineering, 2001, Vol. 3, pages 335-373.

Other examples of potentially suitable polymers include, but are not limited to, polyethylene terephthalate (PET), polyacrylate, polymethacrylate, polycarbonate, polystyrene, polyethylene, polypropylene, polyvinylchloride, cyclic olefin copolymer (COC), polytetrafluoroethylene, a fluorinated polymer, a silicone such as polydimethylsiloxane, polyvinylidene chloride, bis-benzocyclobutene ("BCB"), a polyimide, a fluorinated derivative of a polyimide, or the like. Combinations, copolymers, or blends involving polymers including those described above are also envisioned. The device may also be formed from composite materials, for example, a composite of a polymer and a semiconductor material.

In some embodiments, various structures or components of the article are fabricated from polymeric and/or flexible and/or elastomeric materials, and can be conveniently formed of a hardenable fluid, facilitating fabrication via molding (e.g. replica molding, injection molding, cast molding, etc.). The hardenable fluid can be essentially any fluid that can be induced to solidify, or that spontaneously solidifies, into a solid capable of containing and/or transporting fluids contemplated for use in and with the fluidic network. In one embodiment, the hardenable fluid comprises a polymeric liquid or a liquid polymeric precursor (i.e. a "prepolymer"). Suitable polymeric liquids can include, for example, thermoplastic polymers, thermoset polymers, waxes, metals, or mixtures or composites thereof heated above their melting point. As another example, a suitable polymeric liquid may include a solution of one or more polymers in a suitable solvent, which solution forms a solid polymeric material upon removal of the solvent, for example, by evaporation. Such polymeric materials, which can be solidified from, for example, a melt state or by solvent evaporation, are well known to those of ordinary skill in the art. A variety of polymeric materials, many of which are elastomeric, are suitable, and are also suitable for forming molds or mold masters, for embodiments where one or both of the mold masters is composed of an elastomeric material. A non-limiting list of examples of such polymers includes polymers of the general classes of silicone polymers, epoxy polymers, and acrylate polymers. Epoxy polymers are characterized by the presence of a three-membered cyclic ether group commonly referred to as an epoxy group, 1,2-epoxide, or oxirane. For example, diglycidyl ethers of bisphenol A can be used, in addition to compounds based on aromatic amine, triazine, and cycloaliphatic backbones. Another example includes the well-known Novolac polymers. Non-limiting examples of silicone elastomers suitable for use according to the invention include those formed from precursors including the chlorosilanes such as methylchlorosilanes, ethylchlorosilanes, phenylchlorosilanes, dodecyltrichlorosilanes, etc.

Silicone polymers are used in certain embodiments, for example, the silicone elastomer polydimethylsiloxane. Non-limiting examples of PDMS polymers include those sold under the trademark Sylgard by Dow Chemical Co., Midland, MI, and particularly Sylgard 182, Sylgard 184, and Sylgard 186. Silicone polymers including PDMS have several beneficial properties simplifying fabrication of various structures of the description. For instance, such materials are inexpensive, readily available, and can be solidified from a prepolymeric liquid via curing with heat. For example, PDMSs are typically curable by exposure of the prepolymeric liquid to temperatures of about, for example, about 65 °C to about 75 °C for exposure times of, for example, about an hour. Also, silicone polymers, such as PDMS, can be elastomeric and thus may be useful for forming very small features with relatively high aspect ratios, necessary in certain embodiments of the invention. Flexible (e.g., elastomeric) molds or masters can be advantageous in this regard.

One advantage of forming structures such as microfluidic structures or channels from silicone polymers, such as PDMS, is the ability of such polymers to be oxidized, for example by exposure to an oxygen-containing plasma such as an air plasma, so that the oxidized structures contain, at their surface, chemical groups capable of cross-linking to other oxidized silicone polymer surfaces or to the oxidized surfaces of a variety of other polymeric and non-polymeric materials. Thus, structures can be fabricated and then oxidized and essentially irreversibly sealed to other silicone polymer surfaces, or to the surfaces of other substrates reactive with the oxidized silicone polymer surfaces, without the need for separate adhesives or other sealing means. In most cases, sealing can be completed simply by contacting an oxidized silicone surface to another surface without the need to apply auxiliary pressure to form the seal. That is, the pre-oxidized silicone surface acts as a contact adhesive against suitable mating surfaces. Specifically, in addition to being irreversibly sealable to itself, oxidized silicone such as oxidized PDMS can also be sealed irreversibly to a range of oxidized materials other than itself including, for example, glass, silicon, silicon oxide, quartz, silicon nitride, polyethylene, polystyrene, glassy carbon, and epoxy polymers, which have been oxidized in a similar fashion to the PDMS surface (for example, via exposure to an oxygen-containing plasma). Oxidation and sealing methods useful in the context of the present invention, as well as overall molding techniques, are described in the art, for example, in an article entitled "Rapid Prototyping of Microfluidic Systems and Polydimethylsiloxane," Anal. Chem., 70:474-480, 1998 (Duffy *et al.),*

Thus, in certain embodiments, the design and/or fabrication of the article may be relatively simple, e.g., by using relatively well-known soft lithography and other techniques such as those described herein. In addition, in some embodiments, rapid and/or customized design of the article is possible, for example, in terms of geometry. In one set of embodiments, the article may be produced to be disposable, for example, in embodiments where the article is used with substances that are radioactive, toxic, poisonous, reactive, biohazardous, etc., and/or where the profile of the substance (e.g., the toxicology profile, the radioactivity profile, etc.) is unknown. Another advantage to forming channels or other structures (or interior, fluid-contacting surfaces) from oxidized silicone polymers is that these surfaces can be much more hydrophilic than the surfaces of typical elastomeric polymers (where a hydrophilic interior surface is desired). Such hydrophilic channel surfaces can thus be more easily filled and wetted with aqueous solutions than can structures comprised of typical, unoxidized elastomeric polymers or other hydrophobic materials.

The following documents are referred to in their entirety for all purposes: Int. Pat. Apl. Pub. No. WO 2004/091763, entitled "Formation and Control of Fluidic Species," by Link *et al.;* Int. Pat. Apl. Pub. No. WO 2004/002627, entitled "Method and Apparatus for Fluid Dispersion," by Stone *et al.;* Int. Pat. Apl. Pub. No. WO 2006/096571, entitled "Method and Apparatus for Forming Multiple Emulsions," by Weitz *et al.;* Int. Pat. Apl. Pub. No. WO 2005/021151, entitled "Electronic Control of Fluidic Species," by Link *et al.;* Int. Pat. Apl. Pub. No. WO 2011/056546, entitled "Droplet Creation Techniques," by Weitz, *et al.;* Int. Pat. Apl. Pub. No. WO 2010/033200, entitled "Creation of Libraries of Droplets and Related Species," by Weitz, *et al.;* U.S. Pat. Apl. Pub. No. 2012-0132288, entitled "Fluid Injection," by Weitz, *et al.;* Int. Pat. Apl. Pub. No. WO 2008/109176, entitled "Assay And Other Reactions Involving Droplets," by Agresti, *et al.;* and Int. Pat. Apl. Pub. No. WO 2010/151776, entitled "Fluid Injection," by Weitz, *et al.* In addition, U.S. Provisional Patent Application Serial No. 61/981,123, filed April 17, 2014, entitled "Systems and Methods for Droplet Tagging," by Nicol, *et al.,* and U.S. Provisional Patent Application Serial No. 61/981,108, filed April 17, 2014, entitled "Methods and Systems for Droplet Tagging and Amplification," by Weitz, *et al.* are each referred to in its entirety

In addition, the following are referred to in their entireties: U.S. Pat. Apl. Ser. No. 61/981,123 filed April 17, 2014; a PCT application filed April 17, 2015, entitled "Systems and Methods for Droplet Tagging"; U.S. Pat. Apl. Ser. No. 61/981,108 filed April 17, 2014; a U.S. patent application filed on April 17, 2015, entitled "Immobilization-Based Systems and Methods for Genetic Analysis and Other Applications"; a U.S. patent application filed on April 17, 2015, entitled "Barcoding Systems and Methods for Gene Sequencing and Other Applications"; U.S. Pat. Apl. Ser. No. 62/072,944, filed October 30, 2014; and a PCT application filed on April 17, 2015, entitled "Systems and Methods for Barcoding Nucleic Acids."

The following examples are intended to illustrate certain embodiments of the present invention, but do not exemplify the full scope of the invention.

### EXAMPLE 1

High-throughput screening (HTS) is a method for drug discovery. Using robotics, data processing and control software, liquid handling devices, and sensitive detectors, a researcher can conduct large scale of pharmacological tests, or identify active compounds, antibodies, or genes that modulate a particular biomolecular pathway. The key labware of HTS is the microtiter plate, which can have, e.g., 384, 1536, or 3456 wells, and current robots can often test up to 100,000 compounds per day. However, this technology is approaching its physical limit; below the 1-microliter-volumes of 1,536-well plates, evaporation and capillary forces become significant.

Developments on microwell-based microfluidic technology have significantly improved screening capabilities, increased the speed by 10-fold and decreased the reaction volume by 1,000-fold. The use of water-in-oil drops eliminates solid wells used in microtiter plates; this can simplify engineering and/or expand the capacity of drug screening within an acceptable time and cost scale. This is demonstrated in this example, where droplets are multi-functionalizd to demonstrate drug screening with a high level of combinations, e.g., to test their synergistic effects on cells.

To construct massive drug combinations, three groups of relatively monodisperse picoliter drops were first individually generated in 96-parallel microfluidic drop-makers. Each group included 96 kinds of drops with different drugs and their different concentrations, along with a unique pre-mixed oligonucleotide index in the solution (96 was used here as an illustrative example, although other numbers of drops could have been used in other embodiments). These three groups of drops were then merged using a microfluidic drop-merger in a random combination of different drugs and different concentrations. Single K562 chronic myeloid leukemia cells were introduced to the drug combinations by picoinjecting a cell suspension to the merged drops at a concentration known to obtain a Poisson distribution with rate λ (lambda) = 0.1, and incubated at 37 °C for 24 hours. See, e.g., U.S. Pat. Apl. Pub. No. 2012/0132288, entitled "Fluid Injection,-"

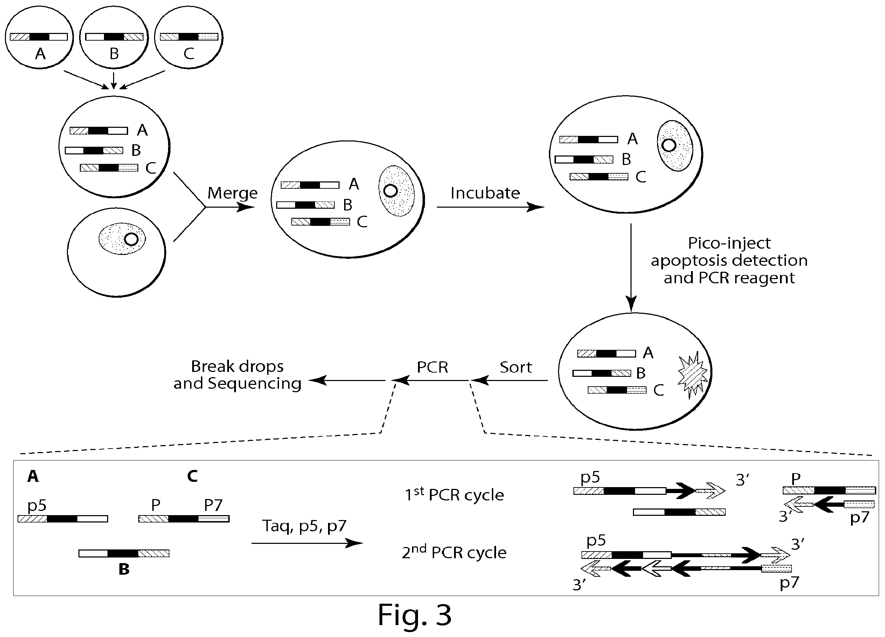

By adding a fluorogenic substrate, caspalux6-J1D2, which is specifically cleaved by increased caspase 3 and caspase 3-like activities during apoptosis, apoptosis of cells in drops can be determined. In this apoptosis assay solution, a PCR cocktail was included to link the oligonucleotide indexes to a full-length double-stranded DNA barcode through PCR amplification. After incubation at 37 °C for half an hour, the drops containing apoptotic cells that suggest effective drug combinations were sorted according to fluorescence intensity, followed by PCR amplification and next-generation sequencing (NGS) to decode the double-stranded DNA barcodes in each sorted drops, which were used to reveal the optimal drug combinations. The schema of this large-scale drug combination screening system is shown in Fig. 3, showing large-scale drug combination screening in drop-based microfluidics.

The strategy to create a double-stranded DNA "barcode" representing three oligos/three drugs and their different concentrations is presented in the Fig. 3 inset. To form this DNA barcode, three families of oligonucleotide indexes are used, a left oligonucleotide (A), a center oligonucleotide (B) and a right oligonucleotide (C). The left (A) and center (B) partially overlap, and the center (B) and right (C) partially overlap. These overlaps allow the three oligonucleotides to anneal to each other when they are present in a single drop, as discussed below. The drug defining unique barcode is encoded in the non-overlapping parts of the left, center and right oligonucleotides. After two rounds of PCR, these three oligonucleotides result in a double stranded "ABC" DNA "barcode." To allow the DNA barcode to be sequenced through NGS, common sequencing primers P5 and P7 are integrated on the 5' end of the left (A) oligonucleotide and the 3' end of the right (C) oligonucleotide, respectively. The annealing and PCR are performed within individual droplets, e.g., to make sure the 3 barcodes are linked together to allow subsequent sequence analysis to reveal what 3 drugs were combined based on the oligonucleotides within the "barcode." A bioinformatics pipeline to decode the DNA barcodes from NGS reads has been developed.

An even annealing and amplification of combinations of four A, four B and four C oligos in bulk, 64 barcode combinations in total, is shown in Fig. 4; this property allows for quantitatively analyzing how many cells have been induced to undergo apoptosis by counting the unique barcode reads. Furthermore, another advantage for this drop-based platform to perform quantitative apoptosis detection is that the loss of apoptotic cells was minimized compared with bulk assays, in which several staining and washing steps diminish the accuracy for apoptosis detection.

Fig. 4 shows even amplification of 64 barcode combinations in bulk decoded by deep sequencing, representing three oligonucleotides/three drugs and their different concentration combinations. It should be noted that each "barcode" was amplified by substantially the same amount, i.e., the amplification was "even," e.g., rather than favoring one or two barcodes at the expense of the other barcodes.

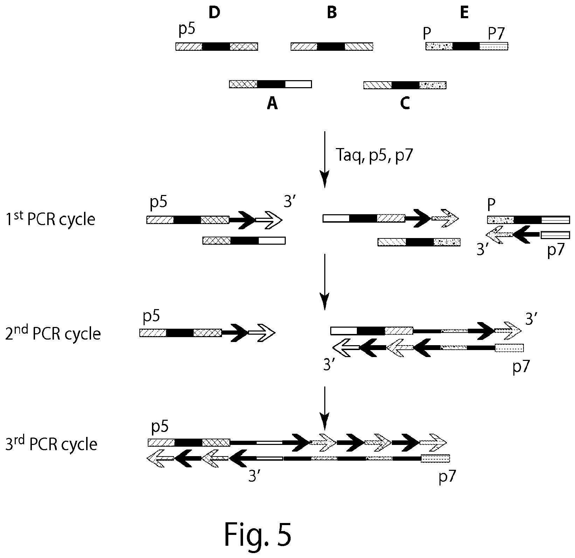

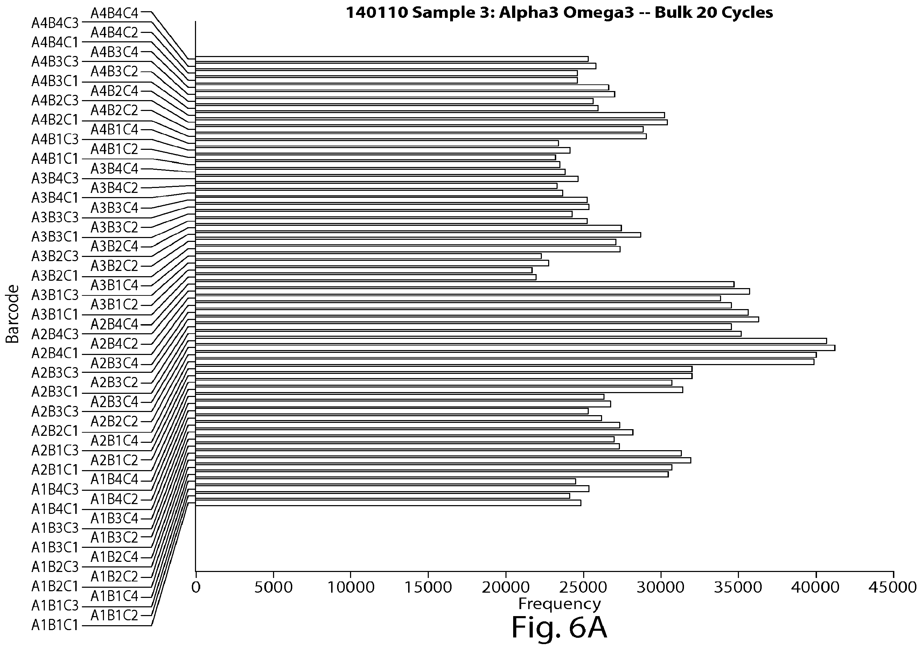

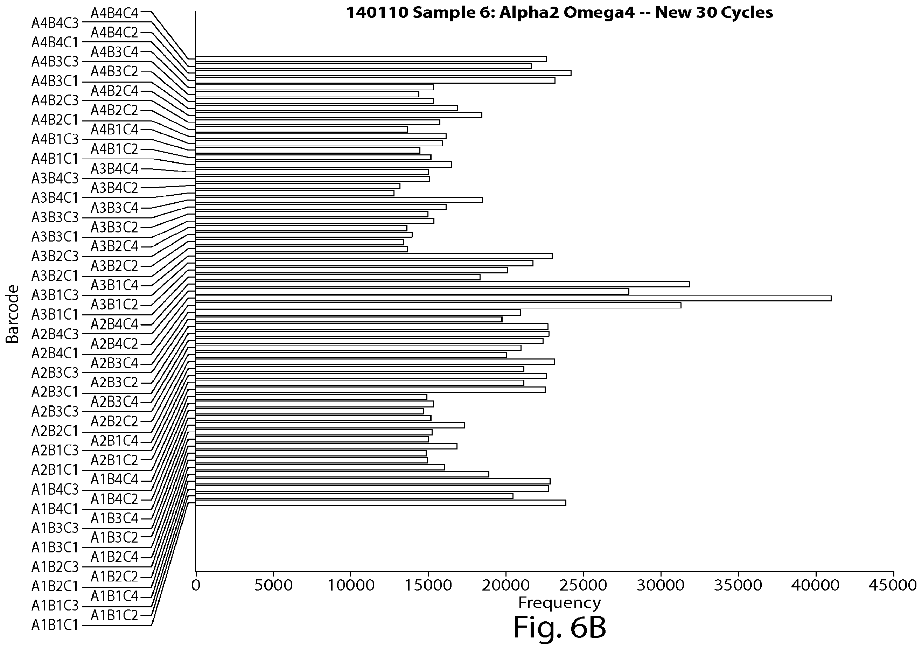

Given that each group of drops had at most 96 kinds of drops with different drugs and their different concentrations, and there are a total of three groups of drops, nearly 1 million drug combinations could be obtained (96x96x96). To further scale up the screening in terms of multiple cell lines without increasing the deep-sequencing run, two oligonucleotide indexes D and E were added into the solution containing the PCR cocktail in another set of experiments. The formation of double stranded DNA barcodes shared a similar mechanism as that described above. Briefly, the newly added oligo indexes D and E integrate P5 had P7 sequences, and partially overlap with oligo A and C, respectively. Instead of in two cycles of PCR, the final barcode DNA was constructed in three cycles of PCR, as shown in Fig. 5, showing a strategy to create a double-stranded DNA barcode combining three oligonucleotide tagging drugs and two more barcodes tagging the cell lines. Even amplifications of 64 barcode combinations with two more barcodes to tag the cell lines is shown in both bulk and drop-based amplification (Fig. 6). This strategy allowed screening of drug combinations and cell lines in a high-throughput and cost- and time-effective way in this example.

Fig. 6 shows even amplification of 64 barcode combinations with 2 more barcodes to tag the samples is shown by deep sequencing. Fig. 6A shows bulk amplification, while Fig. 6B shows drop-based amplification.

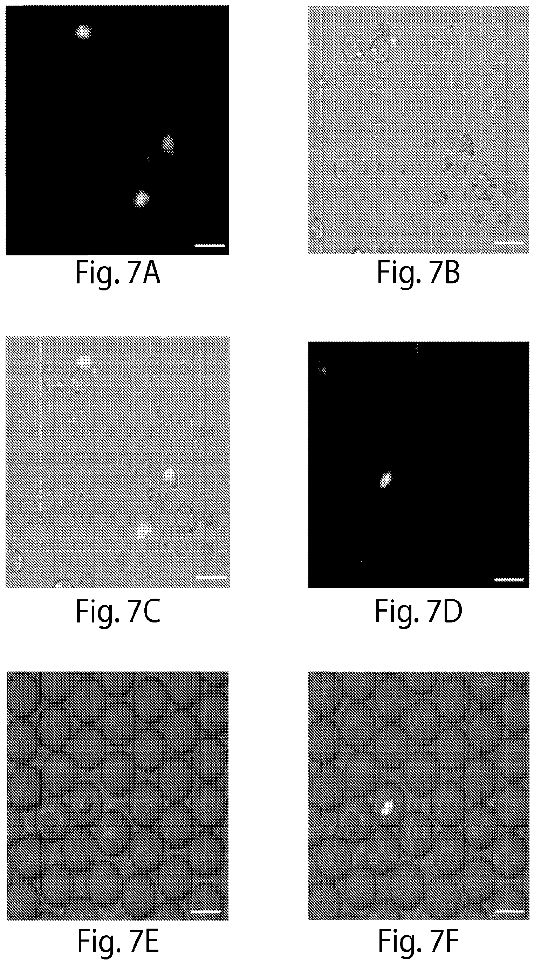

In this example, apoptosis was selected as an evaluation marker for drug combination efficiency; this is because apoptosis is deregulated in many cancers, making it difficult to kill tumors, and drugs that restore the normal apoptotic pathways have the potential for effectively treating cancers that depend on aberrations of the apoptotic pathway to stay alive. However, in other cases, other evaluation markers could also have been studied. Since in drop-based apoptosis detection, the dye is not washed out, a wash-free dye was used here, which not only distinctively stains apoptotic cells, but shows sufficient contrast between cells and background. Caspalux6-J1D2 met these requirements, and showed a clear apoptotic signal in the Imatinib-treated K562 cells in both bulk and drop experiment; this result is shown in Fig. 7.

Fig. 7 shows apoptosis detection of K562 cells in bulk and drop experiment after being treated with Imatinib for 24 hours. Figs. 7A-7C, bulk experiment: fluorescence field (Fig. 7A), bright field (Fig. 7B), and merging (Fig. 7C). Figs. 7D-7F, drop experiment: fluorescence field (Fig. 7D), bright field (Fig. 7E), and merging (Fig. 7F).

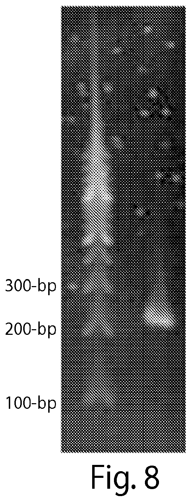

One consideration about the dye used is that it potentially could inhibit the amplification and the construction of the double-stranded DNA barcode. In these examples, full-length DNA barcodes was amplified with the presence of an apoptosis assay reagent, as shown is Fig. 8, thus showing that this is less of a concern. Fig. 8 shows successful amplification of 64 barcode combinations with two more barcodes in drops in the presence of apoptosis assay reagent.

These examples describe a drop-based microfluidic system for quantitative drug combination screening. Although the current example describes a three-group oligo merging, the assay can also be updated to allow for five, seven or more combinations of oligonucleotides in single droplets. This assay screens for cell killing using a fluorescent apoptosis signal, but can also work for other biomarkers. Furthermore, the assay may work for any biologically active agent to be screened not just drugs, but other agents such as siRNA, shRNA, TALENs, CRISPR-Cas and retroviral libraries, etc.

### EXAMPLE 2

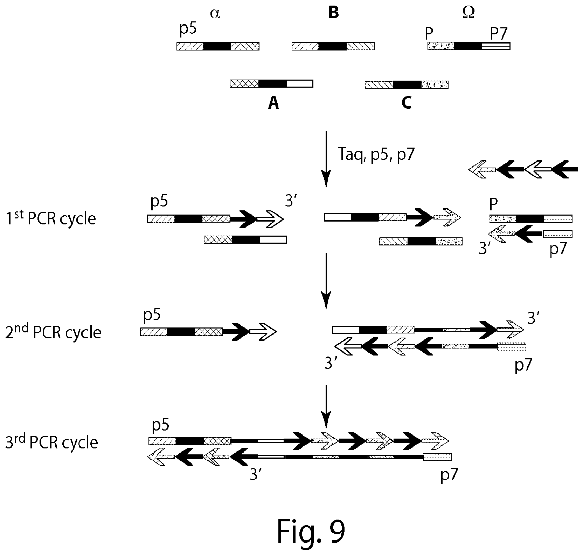

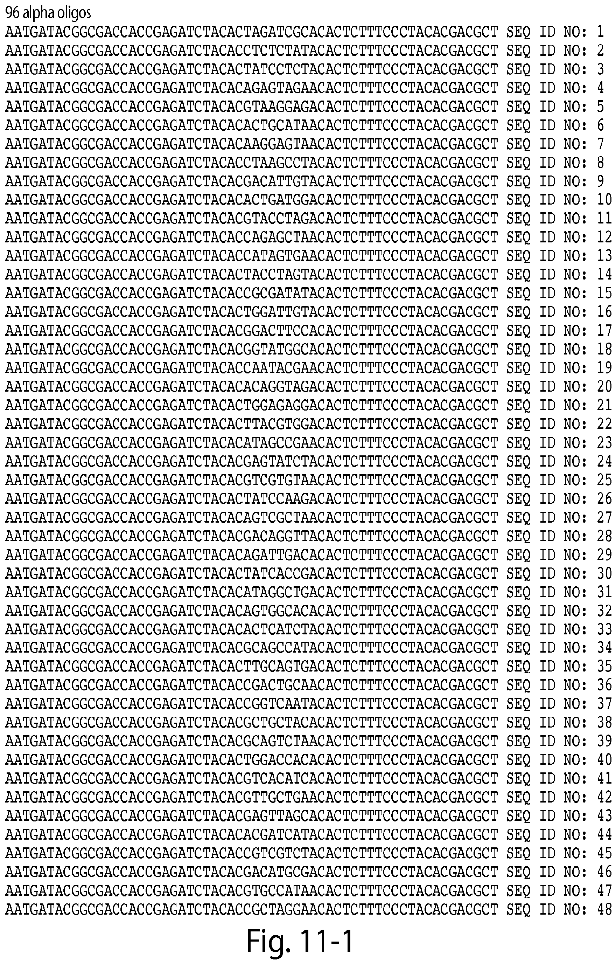

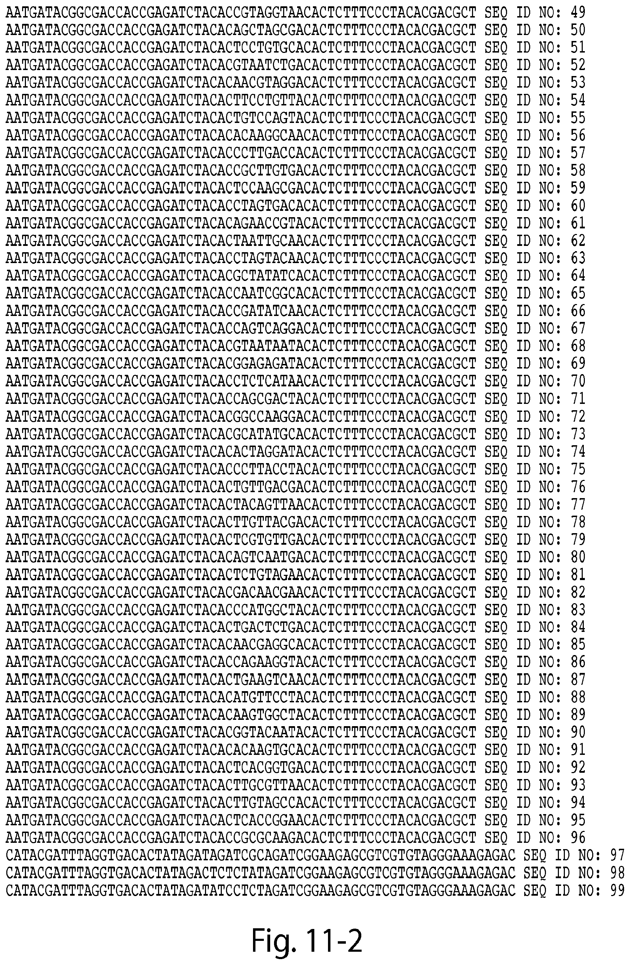

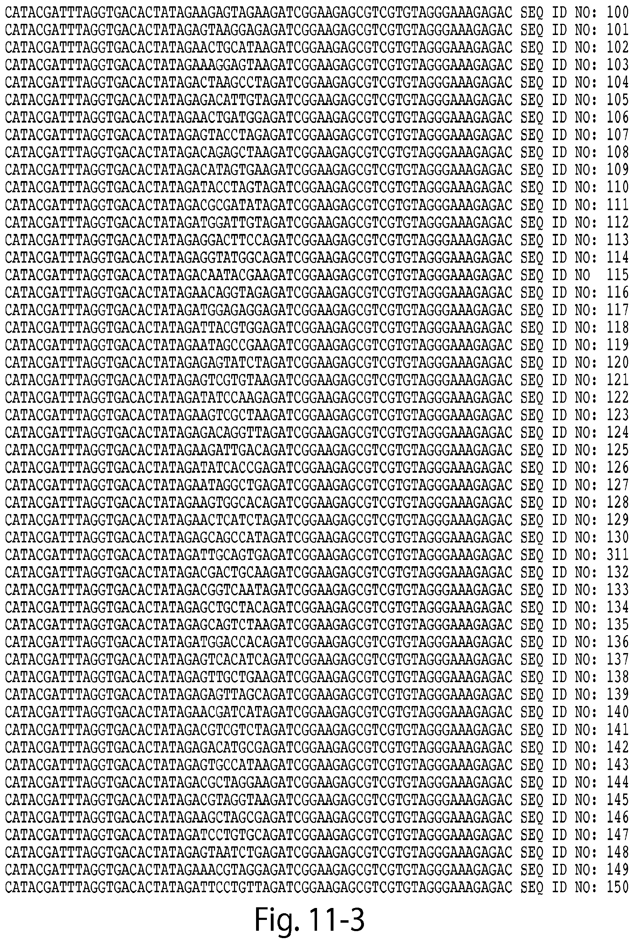

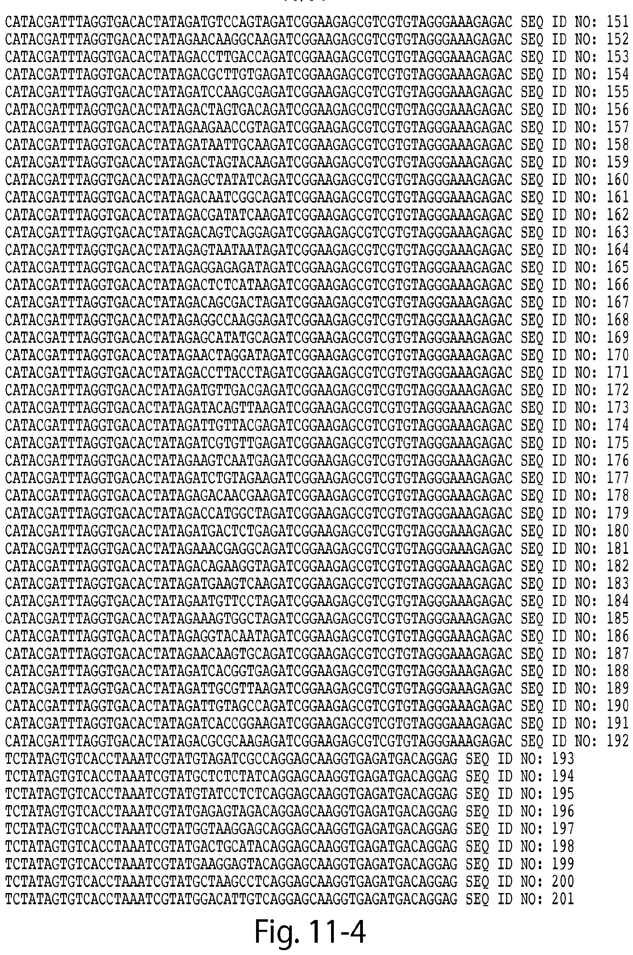

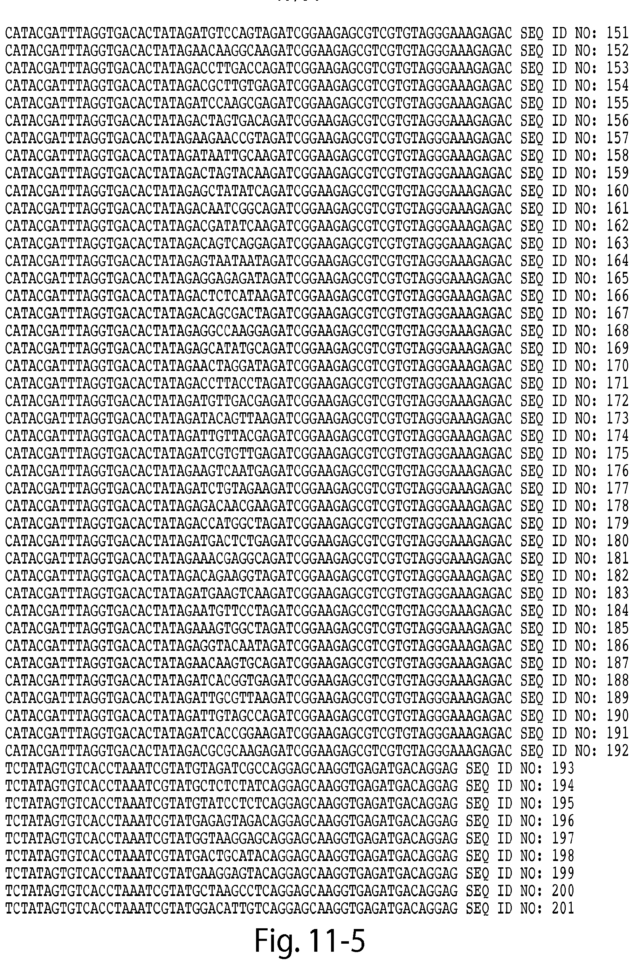

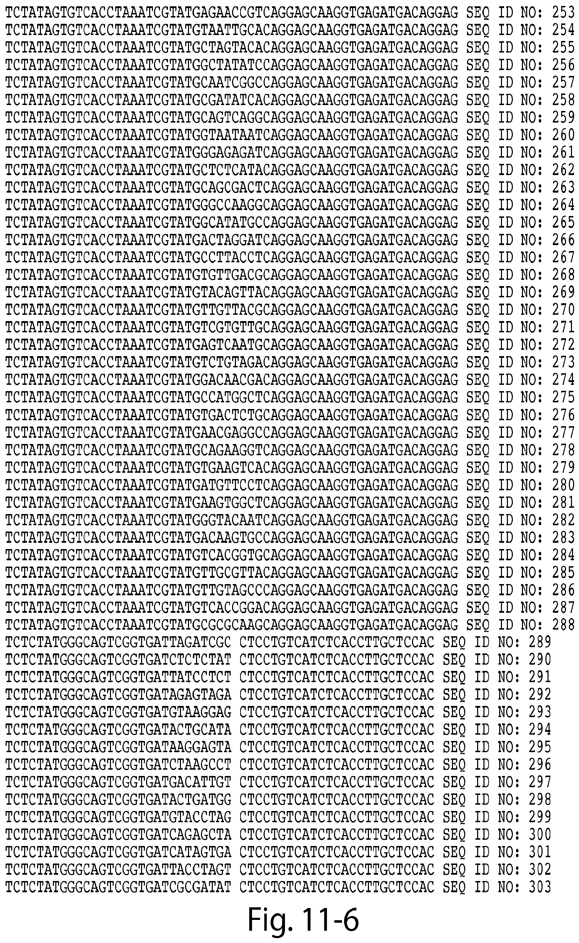

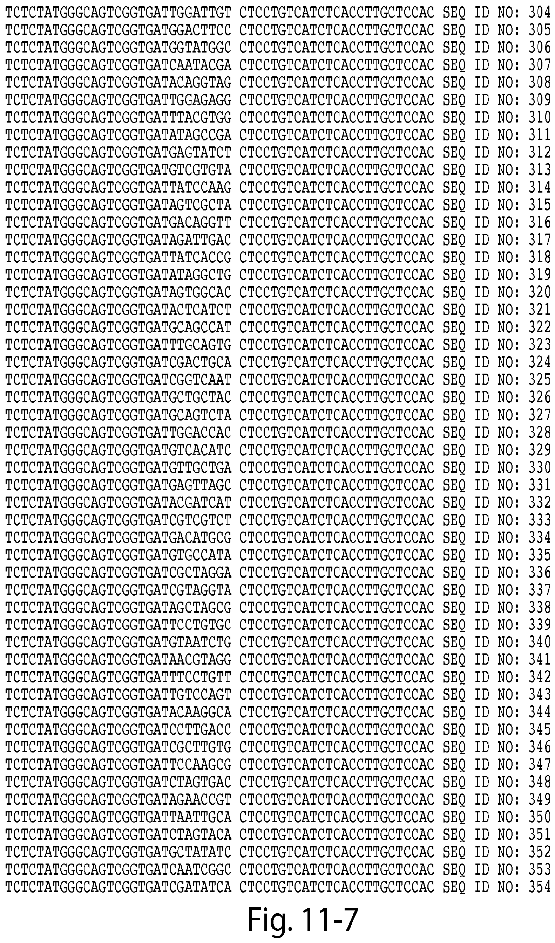

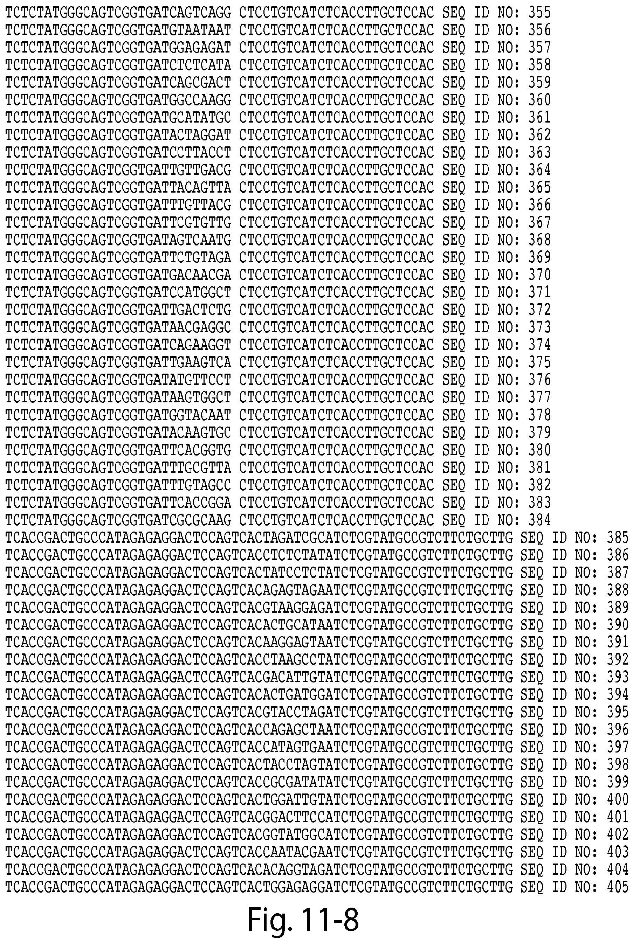

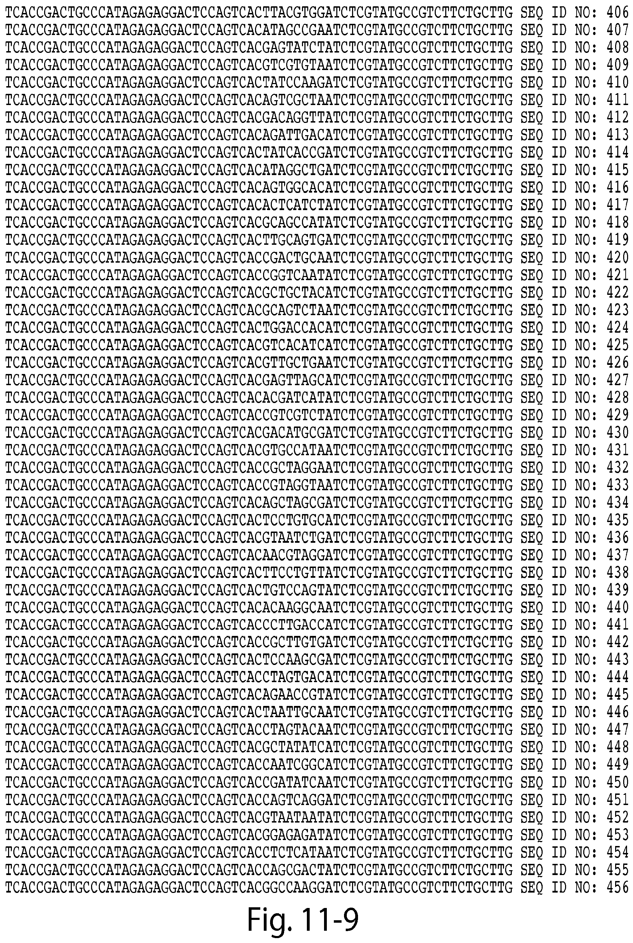

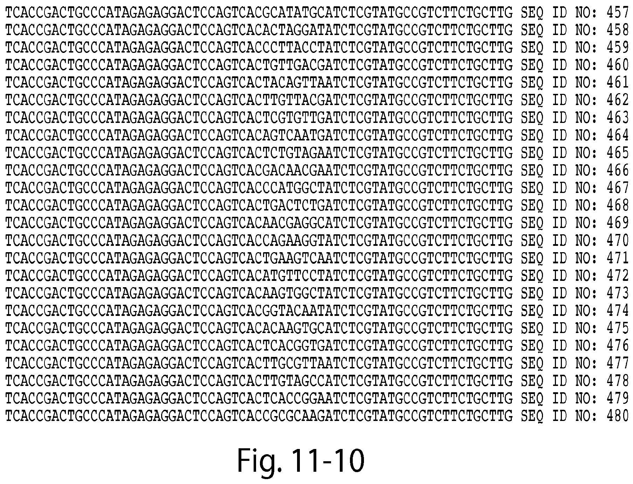

This example illustrates the design of drug-barcoding method by an example of testing 3-drug interaction from a set of 24 drugs, each with 4 concentrations (0, IC25, IC50 and IC75). 96 different oligonucleotides were designed that contain different barcodes for labeling 96 (= 24 x 4) drug-concentrations. Every oligonucleotide contained linker sequences flanking both sides of its barcode. To test up to 3 drug interactions, 3 different pairs of linkers (A, B, and C) were used to generate 3 sets of oligonucleotides for labeling the same 96 drug-concentrations 3 times, redundantly. The linkers allowed for linker-linker connection via PCR to yield A-B-C combinations (total n = 96 x 96 x 96), and for addition of downstream sequencing adaptor primers (denoted as α, alpha and Ω, omega), resulting in full-length α-A-β-C-Ω PCR products that were ready for sequencing (Fig. 9). 96 different alpha oligonucleotides were used for barcoding experiment conditions such as incubation time, technical replications etc. 96 different omega oligonucleotides for barcoding cell lines to be tested could have also be used. 8-nt random mers were used here as barcodes, although these could have also been shorter or longer. Alpha and omega were designed for, but not limited to, the Illumina Miseq platform. Fig. 11 lists oligonucleotide sequences for alpha, A, B, C and omega, each with 96 different barcodes (480 sequences). The full-length of the oligonucleotide barcode PCR product was 212 base pairs, among which only 8, 74, and 8 base pairs need to be sequenced and contain barcode for alpha, A-B-C, and omega, respectively. This simple non-limiting example is capable of barcoding nearly 1 billion (n = 96 x 96 x 96) "drug-concentration/experiment condition/cell line" combinations, with a capacity of up to 3 drug interactions. The 96 barcodes could also be used for testing 48 drugs with 2 concentrations. Further, this example is easily scaled up to higher levels of drug interactions (5 or 10 if necessary) by increasing the number of replicate plates and higher number of testing drugs by using more oligo barcodes (for example, 8-nt random mers could provide 65,536 possible barcodes).

Fig. 9 shows the strategy used in this example to create a double-stranded DNA barcode combining three oligonucelotides (A, B, and C) tagging drugs and two more barcodes (alpha and omega) tagging the cell lines.

Fabrication of microfluidics devices. Polydimethylsiloxane (PDMS) devices were fabricated using replica molding with SU8 photo resist as the mold master. The PDMS devices were rendered more hydrophobic by coating them with Aquapel (Rider, MA, USA). Aquapel was injected into the devices and then the devices were dried by blowing air into them and baking at 65 °C for 15 minutes. Electrodes were fabricated on chip using low melting temperature solder.

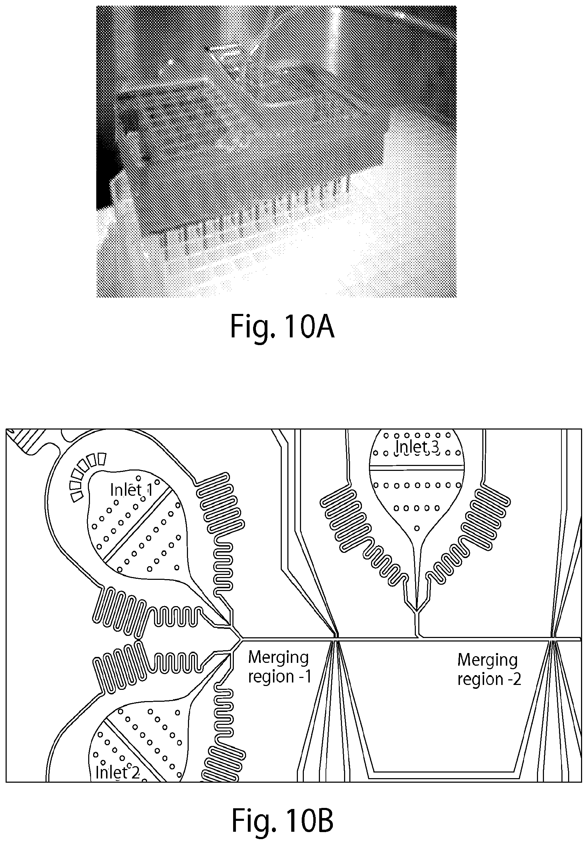

Encapsulation of the oligo-drug library emulsion. Three 96-well plates (A, B and C), each contained 24 different drugs with 4 different concentrations, thus these experiments used 96 drug-concentrations per plate, 3 replicate plates. To plate A, 96 different A oligonucleotides were added. Similarly, 96 B oligonucleotides and 96 C oligonucleotides are added to plate B and C, respectively. To encapsulate the oligonucleotides in drops, 96 parallel drop-makers were designed on a single microfluidic chip, so that the aqueous inlets of each drop-maker (22 gauge stainless steel capillaries, New England Small Tube) fit one quarter of a 384 well-plate and were immersed in 96 different wells of a 96-well plate, each containing a unique drug at certain concentration and a unique oligonucleotide. See Fig. 10A.

Oil with 1% w/w surfactant was distributed to all drop-makers via a common inlet that was connected to a pressurized oil reservoir. The plate and the microfluidic parallel device were placed in a pressure chamber while a common outlet for all 96 drop-makers was located outside the pressure chamber. Upon pressurizing the chamber, each of the 96 oligonucleotide-drug solutions was forced through its own drop-maker, thereby forming an emulsion at junction of the oil and oligonucleotide-drug solution. The oil reservoir was pressurized to 9 psi (1 psi ~ 6894,757 Pa) and the pressure chamber to 6 psi, producing -40 micrometer drops at a rate of about 500 microliters/min from 96 wells. This encapsulation process was performed two more times using two new 96 parallel drop-makers from the other two 96-well plates. These two drop-makers had a smaller junction size to produce -25 micrometer drops.

Generation of drug combination library emulsion. To obtain a random drug combination library of different drugs and different concentrations, a three-drop merging microfluidic chip was designed to merge the three groups of drops from the three 96-well plates (Fig. 10B). Large drops and one group of the small drops was injected to the inlet 1 and inlet 2; drops in each group were spaced by flowing in oil with 1% w/w surfactant. By adjusting the flow rates of drops and oil, the two groups of drops were synchronized to flow in the same channel. The small drops flowed faster than the large drops; this allowed the small drops to chase the large drops to form a pair. The droplets were then merged together when a pair of the large drop and small drop passed an electric field in the emerging region-1. The electric field was generated by the electrodes connected with a high-voltage amplifier (Trek). The other group of small drops were injected into the inlet 3, spaced by oil with 1% w/w surfactant as well. These drops were synchronized with the merged first two groups of drops and merge them at the merging region-2. Finally, the drops were collected at the outlet. Each successfully merged drop from these three groups of drops contained a random drug combination and an oligonucleotide combination of A, B, and C.

Pico-injection of target cells and incubation. The merged droplets were injected into inlet-1 of a microfluidic pico-injector, spaced by oil with 1% w/w surfactant. A K562 cell solution was prepared and injected into the inlet-s. When the electric filed was applied, the cell solution was injected to the drops containing drugs and oligonucleotides. By calculating the concentration of the cells, this resulted in around one cell per ten drops. To prevent sedimentation in the syringe of cells facing encapsulated, a custom-built, motorized cell stirring setup was used. A 1.5 mm x 8 mm magnetic stirrer bar (VWR, USA) was placed in the syringe containing the cell solution. A small bar magnet was mounted on a DC gear motor (Firgelli Automations, USA) that rotates the magnet, thus reversing the polarity of the magnetic field in its vicinity. This external setup was placed close to the syringe containing the cells to be encapsulated. Continuous rotation of the motor resulted in the rotation of the magnetic field of the bar magnet mounted on the motor. This rotating magnetic field forces the magnetic stirrer bar inside the syringe to rotate continuously, thus stirring the cell solution. The drops were collected in a 1.5 ml Eppendorf tube with cap open, and the tube sealed with one thin strip of stretched Parafilm; this allowed efficient oxygen and CO₂ exchange, and simultaneously prevented evaporating. The drops were incubated at 37 °C in a carbon dioxide cell incubator for 24 h.

Pico-injection of apoptosis assay reagent and PCR cocktail. To add an apoptosis assay reagent to detect apoptotic cells, and to link the three oligos in one drop to a full-length barcode and amplify the linked barcode, apoptosis assay reagent and PCR cocktail was mixed together, and pico-injected into the incubated drops. The component of the mixture is listed in Table 1. Here, alpha and omega were used as a indexes for different samples; this allowed performance of deep-sequencing on different samples together, and further increased the throughput. Drops were collected at the outlet, and incubated at 37 °C for half an hour.

**Table 1. Component of the mixture of apoptosis assay reagent and PCR cocktail**

| Alpha-Omega Master Mix | Volume (microliters) |
|---|---|
| Buffer for Taq, 10X | 24 |
| Mg²⁺, 50 mM | 7.2 |
| dNTP, 10mM | 4.8 |
| P7, 10 micromolar | 6 |
| P5, 10 micromolar | 6 |
| α (alpha) #, 8.8 micromolar | 2.4 |
| Ω (omega) #, 8.8 micromolar | 2.4 |
| 10 mg/ml BSA | 4.8 |
| 10% Tween 20 | 4.8 |
| Platinum Taq Polymerase | 2.4 |
| 10 micromolar substrate solution | 48 |
| Fetal bovine serum (FCS) | 12 |
| TOTAL | 120 |

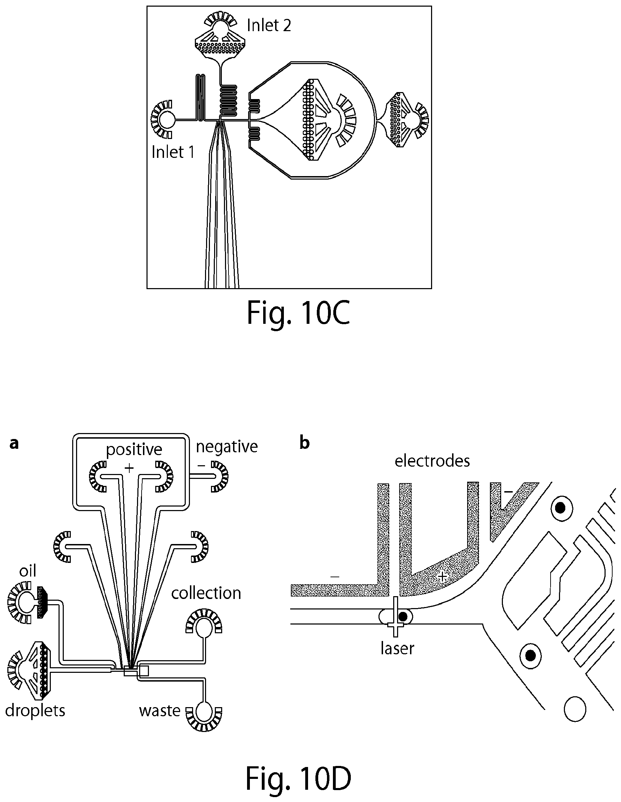

Detection and sorting of apoptotic cells and PCR. The incubated drops were injected into a sorting microfluidic device at a flow rate of 40 microliters/h and evenly spaced by HFE-7500 oil with 1% surfactant flowing (Fig. 10C). The detection and sorting region was aligned to a laser induced fluorescence system. When a drop passes by the laser, its fluorescence generated from an apoptotic cell is collected by a microscope objective and focused on a photomultiplier tube (Hammamatsu) which is connected to a custom computer LabView program running on a real-time field-programmable gate array card (National Instruments). All drops were gated based on detector peak width to exclude outliers, such as doublets and triplets. The counting of bright drops represents the number of apoptotic cells. Drops then flowed to the asymmetric "Y" sorting junction, where they can take one of two paths. In the absence of sorting, droplets preferentially flow to the waste channel, as it has a lower fluidic resistance. When a drop is bright enough to cross the voltage threshold set in the program, the software sends several cycles of a 20 kHz single-ended square wave to the sorting electrodes after being amplified by a factor of 1,000 by a high-voltage amplifier. The bright drops were sorted to 20 microliters of empty drops. Then, the drops were flowed and washed into a PCR tube, the surface covered with mineral oil, and the tube placed in a PCR machine. The thermocycling conditions were 95 °C for 5 min, 10 cycles of 95 °C for 30 sec and 60 °C for 2 min, 10 cycles of 95 °C 30 sec, and 60 °C for 45 sec.

Fig. 10 shows the design of the microfluidic device. Fig. 10A shows 96 drop-makers. Fig. 10B shows an example device for there-drop merging. Fig. 10C shows a pico-injector. Fig. 10D shows a microfluidic sorter (left, overall design; right, zoom-in at sorting junction).

Break the drops and prepare sequencing library. To break or burst the drops, 20% of 1H,1H,2H,2H-perfluorooctanol (PFO) (Alfa Aesar, Ward Hill, MA) was added, vortexed, and centrifuged for 5 min at 5,000 rpm, followed by a cleanup step using SPRI beads (1.0X) cleanup immediately and eluted in 20 microliters of nuclease-free water. To remove remaining single-strand oligonucelotides, the sample was treated with exonuclease (ExoSAP-IT, Affymetrix, CA) at 37 °C for 15 min, followed by 80 °C for 15 min to inactivate exonuclease. The samples were then subjected to a PCR amplification to yield full-length and sufficient library molecule for sequencing. The PCR reaction contained alpha and omega oligonucleotides. The thermocycling conditions were 95 °C for 5 min, 20 cycles of 95 °C for 30 sec, 60 °C for 30 sec, and 72 °C for 60 sec.

Deep sequencing. The library was quantified using a qPCR kit (KAPA Library Quantification Kit - Illumina, MA). A total of 12 billions of pooled library molecules from different experiment conditions were loaded on the Miseq sequencer and sequenced using a 300-cycles V2 kit according to the Nextera dual-index sequencing protocol.

Data analysis. Raw data in BCL format from the Miseq was converted to FASTQ format using the CASAVA v1.8.2 software from Illumina. A custom script was used to annotate alpha, A, B, C, and omega in each sequence read. Briefly, linker sequences were masked. An array of A barcodes (n = 96) were queried in the position window of the read where A barcodes are expected given the design of full-length oligonucleotides. Similarly, B and C barcodes were annotated. The barcode for alpha and omega comes from index read 2 and index read 1, respectively, and was annotated by a custom de-multiplexing script.

A merger of three droplets could contain a single drug (merger of the same drug droplets from replicate wells from plates A, B and C; or one non-zero concentration drug droplet merged with two zero-concentration droplets; etc.), a two-drug combination, or a three-drug combination. Experimental drug concentrations were calculated using input drug quantities, from the decoding of A-B-C barcodes, divided by the calculated average size of cell incubation droplet.

Single agent effect could be evaluated using apoptosis percentage derived from single drug droplet experiments. Synergy and additive effects of two or more drugs are evaluated by a number of statistical models such as Loewe additivity and Bliss independence models using the SAS software.

### EXAMPLE 3

To allow for improved cell viability during prolonged drug treatments in droplets, and to allow for simpler manipulation of cells following drug treatment, this example illustrates embedding cells in agarose gel prior to drug treatment. Manipulation is simplified since magnetic streptavidin nanobeads can be incorporated into the gels that both bind the drug barcodes and allow for magnetic bead washing and separation, and the post-drug step can be performed outside of lipid droplets in bulk, after destabilizing droplets, allowing for staining and washing.

In this example, first cells are mixed with molten low melt agarose at 37 °C and together incorporated into droplets. See, e.g., Int. Pat. Apl. Pub. No. WO 2008/109176, An UltraPure low melting point agarose is used in this experiment, which melts at 65.5 °C, remains fluid at 37 °C and becomes solid rapidly at temperatures below 25 °C. In addition, its gel structure allows for better handling and less breakage. In this example, agarose powder was dissolved into PBS and autoclaved to prepare a sterilized 2% (w/v) agarose solution. Cells were counted and suspend in a DMEM culture media with 40% fetal bovine serum, and the cell suspension was mixed with agarose solution at 1:1 volume ratio. The mixture of cells and agarose gel was flowed into the sample inlet of a microfluidic drop-making device, and HFE7500 oil with 2% surfactant was flowed into the oil inlet to cause the cell-containing agarose gel to produce 80 micrometer drops. The generated gel-in-oil drops were collected into a pre-cooled eppendorf tube in an ice bath.

To further simply the microfluidic manipulation and minimize the time for cells to be exposed to an anaerobic environment when they are put in syringes during encapsulation and re-injection, this example uses a one-step procedure to inject the cell/gel mixture into drops already containing drugs. The drops containing agarose gel particles were collected into a pre-cooled eppendorf tube, after which the injected cell-containing agarose gel remained spherical. In the following steps, the agarose gel particles were injected into the drop containing drug combination and drug barcodes, followed by incubation, destabilization, staining, washing and FACS to obtain the gel particles containing positive cells. To remove oil and surfactant, a drop destabilizer was added to the emulsion and the agarose gel particles were collected by centrifuge.

Leukemia K562 cells having abnormally high activity of oncogene tyrosine kinase(s) and a kinase inhibitor drug, Imatinib, were used as a model, where Imatinib can induce K562 cell apoptosis. The cells were mixed with the agarose gel solution and injected into the mixture into the drops containing 20 micrograms/mL Imatinib at a 1:1 ratio. After 48 hr, the emulsions were burst and the gel particles stained with propidium iodide (PI) and Annexin V to determine the apoptosis and necrosis through differences in plasma membrane integrity and permeability, followed by three rounds of washing.

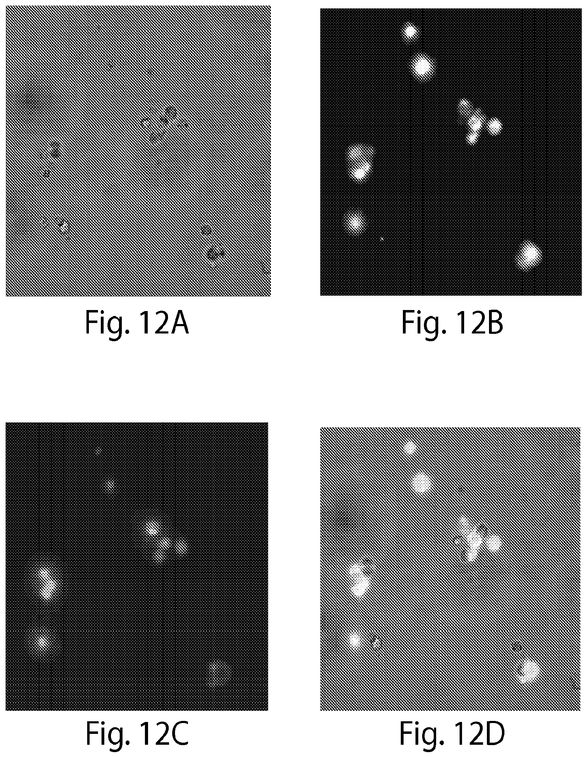

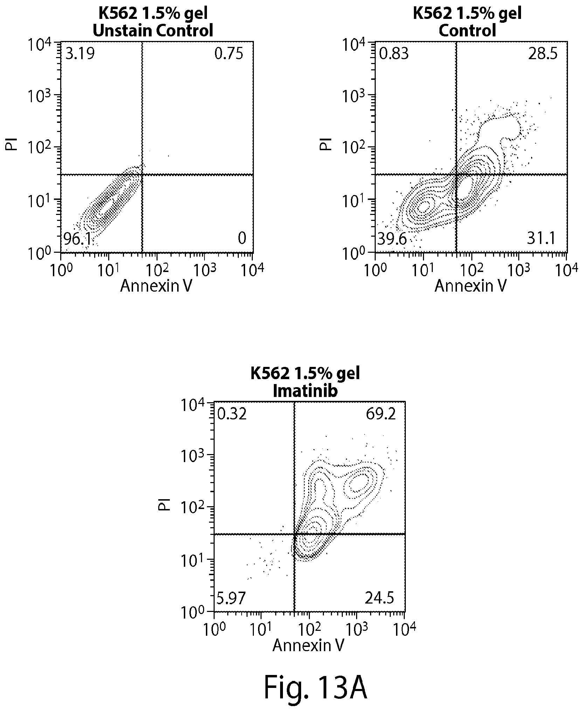

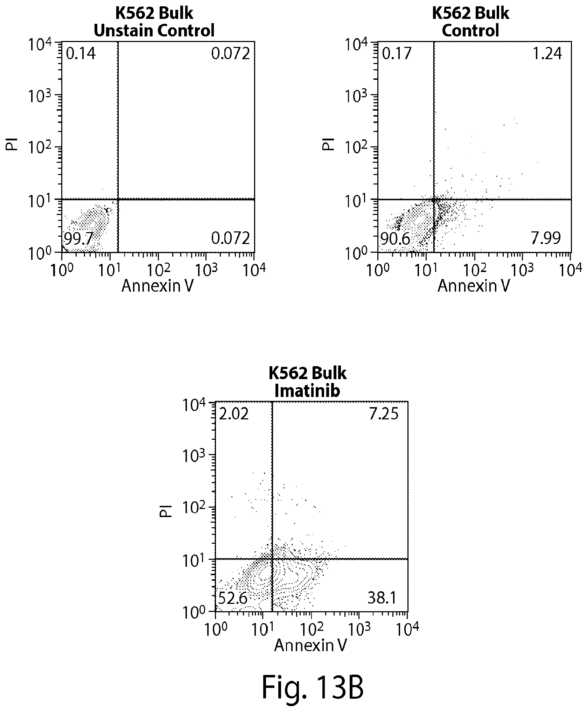

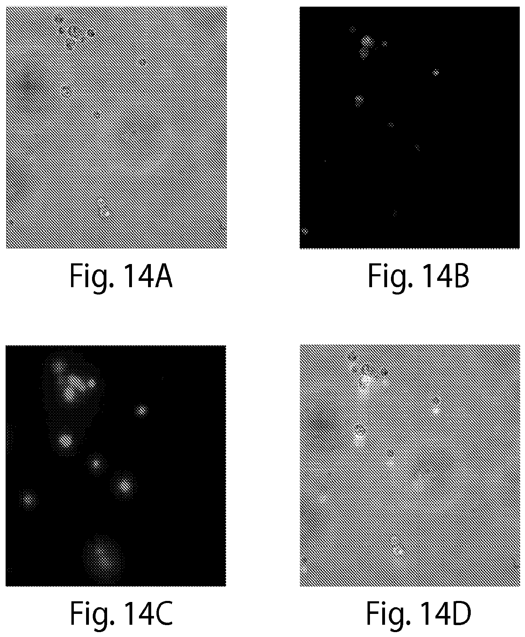

About 70% apoptotic and necrotic cells were observed as shown in Fig. 12. The flow cytometry detection showed more quantitative results (Fig. 13). The cells were also stained with PI and Calcein AM to directly determine the viable and dead cells, as shown in Fig. 14. Thus, this procedure was used to encapsulate cells, culture them in drops, stain them, wash them, and detect them using flow cytometry. Subsequently, FACS or other techniques may be used to sort for positive cells.

Fig. 12 shows fluorescence microscope images of leukemia K562 cells in 1.5% agarose particles after 48 hr of incubation with the presence of 10 micrograms/mL Imatinib. Fig. 12A is bright field; Fig. 12B is the Annexin V channel (green channel); Fig. 12C is the PI channel (red channel); and Fig. 12D is a merged image of Figs. 12B and 12C.

Fig. 13 shows flow cytometry detection results. Fig. 13A shows leukemia K562 cells in 1.5% agarose particles after 48 hr of incubation with the presence of 10 microgram/mL Imatinib. Fig. 13B shows leukemia K562 cells after 48 hr of incubation with the presence of 10 microgram/mL Imatinib.

Fig. 14 shows fluorescence microscope images of leukemia K562 cells in 1.5% agarose particles after 48 hr of incubation with the presence of 10 microgram/mL Imatinib. Fig. 14A is bright field; Fig. 14B is the calcein AM channel (green channel); Fig. 14C is the PI channel (red channel); and Fig. 14D is a merged image of Fig. 14B and 14C.

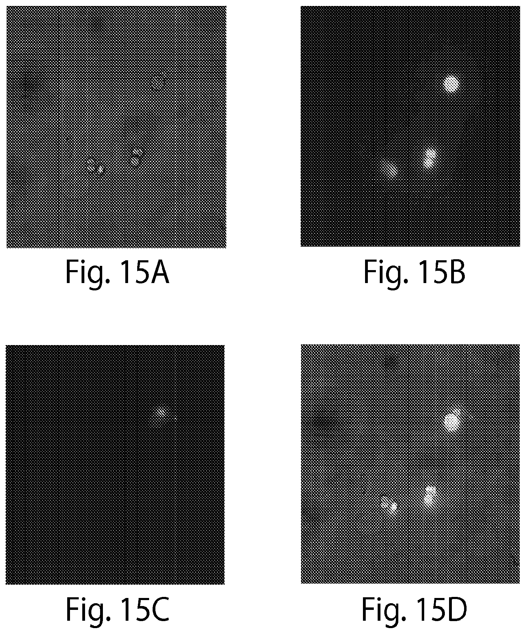

Fig. 15 shows fluorescence microscope images of leukemia K562 cells after 48 hr of incubation with the presence of 10 microgram/mL Imatinib. Fig. 15A is bright field; Fig. 15B is the calcein AM channel (green channel); Fig. 15C is the PI channel (red channel); and Fig. 15D is a merged image of Fig. 15B and 15C.

### EXAMPLE 4

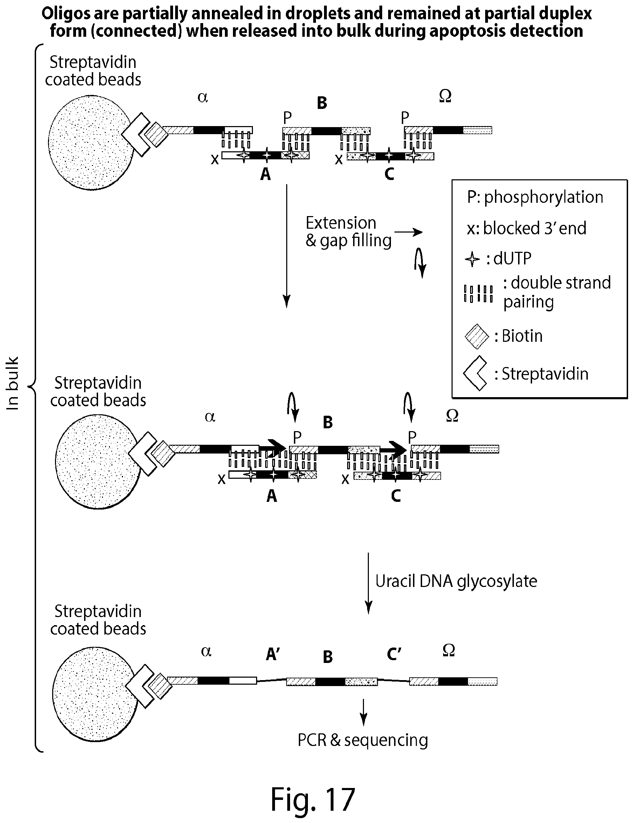

This example illustrates a streptavidin-biotin-based method to establish the linkage between drugs and cells. See also Fig. 17. During drug encapsulation, three barcodes A, B, and C are added into three sets of 96-well plate containing different drugs, respectively, followed by merging the barcodes together. Therefore, each resulting drop contains its unique combinations of both drugs and barcodes. As mentioned, to capture these barcodes on agarose gel particles, nanometer-sized streptavidin magnetic beads are added into agarose gel solution. These beads have been pre-incubated with a biotinylated linker sequence which carries the complementary sequence of barcode A. To test the capture efficiency, agarose gel containing magnetic beads was injected into the drops containing barcodes A, B and C, the drops were collected, the agarose gel was solidified by cooling in ice bath and the droplets incubated at 37 °C for 12 hr. The emulsions were burst and the gel particles collected using a magnetic plate.

With respect to Fig. 17, the extension-and-filling step is achieved in this example using a polymerase without strand displacement property (e.g. Phusion DNA polymerase, NEB) and a gap-filling enzyme that is active (e.g. Ampligase, USB) at the same temperature as the polymerase. The oligonucleotides B and omega (Ω) are 5' phosphorylated. Oligonucleotides A and C contain dUTP and can be dissolved using uracil DNA glycosylate, to avoid oligonucleotides A and C serving as templates for PCR which would confound the oligonucleotide composition on a same full-length oligonucleotide. After uracil DNA glycosylate treatment, the remaining full-length oligonucleotides are PCR amplified for sequencing.

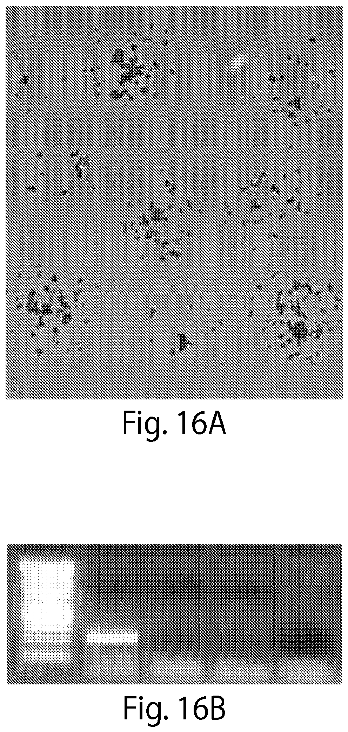

The gel particles are shown in Fig. 16A. These gel particles were used to perform PCR. An expected amplification was observed at 212-bp, which suggested all the barcodes were captured on beads, as shown in Fig. 16B.

Fig. 16A shows an image of nanosized (160-750 nm) streptavidin magnetic beads in agarose gel particles. Fig. 16B shows amplification results of the gel particles. Lane 1, Biotinylated alpha+SA beads, incubated for 5 min, washed and put into agarose gel; lane 2, Biotinylated alpha +SA beads, directly added to agarose gel; lane 3, non-biotinylated alpha +SA beads, incubated for 5 min, washed and put into agarose gel; lane 4, non-biotinylated alpha +SA beads, directly added to agarose gel.

While several embodiments of the present invention have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present description. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used. Those skilled in the art will recognize, or be able to ascertain using no more than routine experimentation, many equivalents to the specific embodiments of the invention described herein. It is, therefore, to be understood that the foregoing embodiments are presented by way of example only and that, within the scope of the appended claims and equivalents thereto, the invention may be practiced otherwise than as specifically described and claimed. The present descriptionis directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present description.

All definitions, as defined and used herein, should be understood to control over dictionary definitions, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

When the word "about" is used herein in reference to a number, it should be understood that still another embodiment of the invention includes that number not modified by the presence of the word "about."

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively, as set forth in the United States Patent Office Manual of Patent Examining Procedures, Section 2111.03.

What is claimed is:

### SEQUENCE LISTING

<110> President and Fellows of Harvard College
   The General Hospital Corporation d/b/a Massachusetts
   General Hospital
<120> METHODS AND SYSTEMS FOR DROPLET TAGGING AND AMPLIFICATION
<130> H0498.70503WO00
<140> Not yet assigned
   <141> Concurrently herewith
<150> US 61/981,108
   <151> 2014-04-17
<160> 480
<170> PatentIn version 3.5
<210> 1
   <211> 60
   <212> DNA
   <213> Artificial Sequence
<220>
   <223> Synthetic Polynucleotide
<400> 1
   aatgatacgg cgaccaccga gatctacact agatcgcaca ctctttccct acacgacgct 60
<210> 2
   <211> 60
   <212> DNA
   <213> Artificial Sequence
<220>
   <223> Synthetic Polynucleotide
<400> 2
   aatgatacgg cgaccaccga gatctacacc tctctataca ctctttccct acacgacgct 60
<210> 3
   <211> 60
   <212> DNA
   <213> Artificial Sequence
<220>
   <223> Synthetic Polynucleotide
<400> 3
   aatgatacgg cgaccaccga gatctacact atcctctaca ctctttccct acacgacgct 60
<210> 4
   <211> 60
   <212> DNA
   <213> Artificial Sequence
<220>
   <223> Synthetic Polynucleotide
<400> 4
   aatgatacgg cgaccaccga gatctacaca gagtagaaca ctctttccct acacgacgct 60
<210> 5
   <211> 60
   <212> DNA
   <213> Artificial Sequence
<220>
   <223> Synthetic Polynucleotide
<400> 5
   aatgatacgg cgaccaccga gatctacacg taaggagaca ctctttccct acacgacgct 60
<210> 6
   <211> 60
   <212> DNA
   <213> Artificial Sequence
<220>
   <223> Synthetic Polynucleotide
<400> 6
   aatgatacgg cgaccaccga gatctacaca ctgcataaca ctctttccct acacgacgct 60
<210> 7
   <211> 60
   <212> DNA
   <213> Artificial Sequence
<220>
   <223> Synthetic Polynucleotide
<400> 7
   aatgatacgg cgaccaccga gatctacaca aggagtaaca ctctttccct acacgacgct 60
<210> 8
   <211> 60
   <212> DNA
   <213> Artificial Sequence
<220>
   <223> Synthetic Polynucleotide
<400> 8
   aatgatacgg cgaccaccga gatctacacc taagcctaca ctctttccct acacgacgct 60
<210> 9
   <211> 60
   <212> DNA
   <213> Artificial Sequence
<220>
   <223> Synthetic Polynucleotide
<400> 9
   aatgatacgg cgaccaccga gatctacacg acattgtaca ctctttccct acacgacgct 60
<210> 10
   <211> 60
   <212> DNA
   <213> Artificial Sequence
<220>
   <223> Synthetic Polynucleotide
<400> 10
   aatgatacgg cgaccaccga gatctacaca ctgatggaca ctctttccct acacgacgct 60
<210> 11
   <211> 60
   <212> DNA
   <213> Artificial Sequence
<220>
   <223> Synthetic Polynucleotide
<400> 11
   aatgatacgg cgaccaccga gatctacacg tacctagaca ctctttccct acacgacgct 60
<210> 12
   <211> 60
   <212> DNA
   <213> Artificial Sequence
<220>
   <223> Synthetic Polynucleotide
<400> 12
   aatgatacgg cgaccaccga gatctacacc agagctaaca ctctttccct acacgacgct 60
<210> 13
   <211> 60
   <212> DNA
   <213> Artificial Sequence
<220>
   <223> Synthetic Polynucleotide
<400> 13
   aatgatacgg cgaccaccga gatctacacc atagtgaaca ctctttccct acacgacgct 60
<210> 14
   <211> 60
   <212> DNA
   <213> Artificial Sequence
<220>
   <223> Synthetic Polynucleotide
<400> 14
   aatgatacgg cgaccaccga gatctacact acctagtaca ctctttccct acacgacgct 60
<210> 15
   <211> 60
   <212> DNA
   <213> Artificial Sequence
<220>
   <223> Synthetic Polynucleotide
<400> 15
   aatgatacgg cgaccaccga gatctacacc gcgatataca ctctttccct acacgacgct 60
<210> 16
   <211> 60
   <212> DNA
   <213> Artificial Sequence
<220>
   <223> Synthetic Polynucleotide
<400> 16
   aatgatacgg cgaccaccga gatctacact ggattgtaca ctctttccct acacgacgct 60
<210> 17
   <211> 60
   <212> DNA
   <213> Artificial Sequence
<220>
   <223> Synthetic Polynucleotide
<400> 17
   aatgatacgg cgaccaccga gatctacacg gacttccaca ctctttccct acacgacgct 60
<210> 18
   <211> 60
   <212> DNA
   <213> Artificial Sequence
<220>
   <223> Synthetic Polynucleotide
<400> 18
   aatgatacgg cgaccaccga gatctacacg gtatggcaca ctctttccct acacgacgct 60
<210> 19
   <211> 60
   <212> DNA
   <213> Artificial Sequence
<220>
   <223> Synthetic Polynucleotide
<400> 19
   aatgatacgg cgaccaccga gatctacacc aatacgaaca ctctttccct acacgacgct 60
<210> 20
   <211> 60
   <212> DNA
   <213> Artificial Sequence
<220>
   <223> Synthetic Polynucleotide
<400> 20
   aatgatacgg cgaccaccga gatctacaca caggtagaca ctctttccct acacgacgct 60
<210> 21
   <211> 60
   <212> DNA
   <213> Artificial Sequence
<220>
   <223> Synthetic Polynucleotide
<400> 21
   aatgatacgg cgaccaccga gatctacact ggagaggaca ctctttccct acacgacgct 60
<210> 22
   <211> 60
   <212> DNA
   <213> Artificial Sequence
<220>
   <223> Synthetic Polynucleotide
<400> 22
   aatgatacgg cgaccaccga gatctacact tacgtggaca ctctttccct acacgacgct 60
<210> 23
   <211> 60
   <212> DNA
   <213> Artificial Sequence
<220>
   <223> Synthetic Polynucleotide
<400> 23
   aatgatacgg cgaccaccga gatctacaca tagccgaaca ctctttccct acacgacgct 60
<210> 24
   <211> 60
   <212> DNA
   <213> Artificial Sequence
<220>
   <223> Synthetic Polynucleotide
<400> 24
   aatgatacgg cgaccaccga gatctacacg agtatctaca ctctttccct acacgacgct 60
<210> 25
   <211> 60
   <212> DNA
   <213> Artificial Sequence
<220>
   <223> Synthetic Polynucleotide
<400> 25
   aatgatacgg cgaccaccga gatctacacg tcgtgtaaca ctctttccct acacgacgct 60
<210> 26
   <211> 60
   <212> DNA
   <213> Artificial Sequence
<220>
   <223> Synthetic Polynucleotide
<400> 26
   aatgatacgg cgaccaccga gatctacact atccaagaca ctctttccct acacgacgct 60
<210> 27
   <211> 60
   <212> DNA
   <213> Artificial Sequence
<220>
   <223> Synthetic Polynucleotide
<400> 27
   aatgatacgg cgaccaccga gatctacaca gtcgctaaca ctctttccct acacgacgct 60
<210> 28
   <211> 60
   <212> DNA
   <213> Artificial Sequence
<220>
   <223> Synthetic Polynucleotide
<400> 28
   aatgatacgg cgaccaccga gatctacacg acaggttaca ctctttccct acacgacgct 60
<210> 29
   <211> 60
   <212> DNA
   <213> Artificial Sequence
<220>
   <223> Synthetic Polynucleotide
<400> 29
   aatgatacgg cgaccaccga gatctacaca gattgacaca ctctttccct acacgacgct 60
<210> 30
   <211> 60
   <212> DNA
   <213> Artificial Sequence
<220>
   <223> Synthetic Polynucleotide
<400> 30
   aatgatacgg cgaccaccga gatctacact atcaccgaca ctctttccct acacgacgct 60
<210> 31
   <211> 60
   <212> DNA
   <213> Artificial Sequence
<220>
   <223> Synthetic Polynucleotide
<400> 31
   aatgatacgg cgaccaccga gatctacaca taggctgaca ctctttccct acacgacgct 60
<210> 32
   <211> 60
   <212> DNA
   <213> Artificial Sequence
<220>
   <223> Synthetic Polynucleotide
<400> 32
   aatgatacgg cgaccaccga gatctacaca gtggcacaca ctctttccct acacgacgct 60
<210> 33
   <211> 60
   <212> DNA
   <213> Artificial Sequence
<220>
   <223> Synthetic Polynucleotide
<400> 33
   aatgatacgg cgaccaccga gatctacaca ctcatctaca ctctttccct acacgacgct 60
<210> 34
   <211> 60
   <212> DNA
   <213> Artificial Sequence
<220>
   <223> Synthetic Polynucleotide
<400> 34
   aatgatacgg cgaccaccga gatctacacg cagccataca ctctttccct acacgacgct 60
<210> 35
   <211> 60
   <212> DNA
   <213> Artificial Sequence
<220>
   <223> Synthetic Polynucleotide
<400> 35
   aatgatacgg cgaccaccga gatctacact tgcagtgaca ctctttccct acacgacgct 60
<210> 36
   <211> 60
   <212> DNA
   <213> Artificial Sequence
<220>
   <223> Synthetic Polynucleotide
<400> 36
   aatgatacgg cgaccaccga gatctacacc gactgcaaca ctctttccct acacgacgct 60
<210> 37
   <211> 60
   <212> DNA
   <213> Artificial Sequence
<220>
   <223> Synthetic Polynucleotide
<400> 37
   aatgatacgg cgaccaccga gatctacacc ggtcaataca ctctttccct acacgacgct 60
<210> 38
   <211> 60
   <212> DNA
   <213> Artificial Sequence
<220>
   <223> Synthetic Polynucleotide
<400> 38
   aatgatacgg cgaccaccga gatctacacg ctgctacaca ctctttccct acacgacgct 60
<210> 39
   <211> 60
   <212> DNA
   <213> Artificial Sequence
<220>
   <223> Synthetic Polynucleotide
<400> 39
   aatgatacgg cgaccaccga gatctacacg cagtctaaca ctctttccct acacgacgct 60
<210> 40
   <211> 60
   <212> DNA
   <213> Artificial Sequence
<220>
   <223> Synthetic Polynucleotide
<400> 40
   aatgatacgg cgaccaccga gatctacact ggaccacaca ctctttccct acacgacgct 60
<210> 41
   <211> 60
   <212> DNA
   <213> Artificial Sequence
<220>
   <223> Synthetic Polynucleotide
<400> 41
   aatgatacgg cgaccaccga gatctacacg tcacatcaca ctctttccct acacgacgct 60
<210> 42
   <211> 60
   <212> DNA
   <213> Artificial Sequence
<220>
   <223> Synthetic Polynucleotide
<400> 42
   aatgatacgg cgaccaccga gatctacacg ttgctgaaca ctctttccct acacgacgct 60
<210> 43
   <211> 60
   <212> DNA
   <213> Artificial Sequence
<220>
   <223> Synthetic Polynucleotide
<400> 43
   aatgatacgg cgaccaccga gatctacacg agttagcaca ctctttccct acacgacgct 60
<210> 44
   <211> 60
   <212> DNA
   <213> Artificial Sequence
<220>
   <223> Synthetic Polynucleotide
<400> 44
   aatgatacgg cgaccaccga gatctacaca cgatcataca ctctttccct acacgacgct 60
<210> 45
   <211> 60
   <212> DNA
   <213> Artificial Sequence
<220>
   <223> Synthetic Polynucleotide
<400> 45
   aatgatacgg cgaccaccga gatctacacc gtcgtctaca ctctttccct acacgacgct 60
<210> 46
   <211> 60
   <212> DNA
   <213> Artificial Sequence
<220>
   <223> Synthetic Polynucleotide
<400> 46
   aatgatacgg cgaccaccga gatctacacg acatgcgaca ctctttccct acacgacgct 60
<210> 47
   <211> 60
   <212> DNA
   <213> Artificial Sequence
<220>
   <223> Synthetic Polynucleotide
<400> 47
   aatgatacgg cgaccaccga gatctacacg tgccataaca ctctttccct acacgacgct 60
<210> 48
   <211> 60
   <212> DNA
   <213> Artificial Sequence
<220>
   <223> Synthetic Polynucleotide
<400> 48
   aatgatacgg cgaccaccga gatctacacc gctaggaaca ctctttccct acacgacgct 60
<210> 49
   <211> 60
   <212> DNA
   <213> Artificial Sequence
<220>
   <223> Synthetic Polynucleotide
<400> 49
   aatgatacgg cgaccaccga gatctacacc gtaggtaaca ctctttccct acacgacgct 60
<210> 50
   <211> 60
   <212> DNA
   <213> Artificial Sequence
<220>
   <223> Synthetic Polynucleotide
<400> 50
   aatgatacgg cgaccaccga gatctacaca gctagcgaca ctctttccct acacgacgct 60
<210> 51
   <211> 60
   <212> DNA
   <213> Artificial Sequence
<220>
   <223> Synthetic Polynucleotide
<400> 51
   aatgatacgg cgaccaccga gatctacact cctgtgcaca ctctttccct acacgacgct 60
<210> 52
   <211> 60
   <212> DNA
   <213> Artificial Sequence
<220>
   <223> Synthetic Polynucleotide
<400> 52
   aatgatacgg cgaccaccga gatctacacg taatctgaca ctctttccct acacgacgct 60
<210> 53
   <211> 60
   <212> DNA
   <213> Artificial Sequence
<220>
   <223> Synthetic Polynucleotide
<400> 53
   aatgatacgg cgaccaccga gatctacaca acgtaggaca ctctttccct acacgacgct 60
<210> 54
   <211> 60
   <212> DNA
   <213> Artificial Sequence
<220>
   <223> Synthetic Polynucleotide
<400> 54
   aatgatacgg cgaccaccga gatctacact tcctgttaca ctctttccct acacgacgct 60
<210> 55
   <211> 60
   <212> DNA
   <213> Artificial Sequence
<220>
   <223> Synthetic Polynucleotide
<400> 55
   aatgatacgg cgaccaccga gatctacact gtccagtaca ctctttccct acacgacgct 60
<210> 56
   <211> 60
   <212> DNA
   <213> Artificial Sequence
<220>
   <223> Synthetic Polynucleotide
<400> 56
   aatgatacgg cgaccaccga gatctacaca caaggcaaca ctctttccct acacgacgct 60
<210> 57
   <211> 60
   <212> DNA
   <213> Artificial Sequence
<220>
   <223> Synthetic Polynucleotide
<400> 57
   aatgatacgg cgaccaccga gatctacacc cttgaccaca ctctttccct acacgacgct 60
<210> 58
   <211> 60
   <212> DNA
   <213> Artificial Sequence
<220>
   <223> Synthetic Polynucleotide
<400> 58
   aatgatacgg cgaccaccga gatctacacc gcttgtgaca ctctttccct acacgacgct 60
<210> 59
   <211> 60
   <212> DNA
   <213> Artificial Sequence
<220>
   <223> Synthetic Polynucleotide
<400> 59
   aatgatacgg cgaccaccga gatctacact ccaagcgaca ctctttccct acacgacgct 60
<210> 60
   <211> 60
   <212> DNA
   <213> Artificial Sequence
<220>
   <223> Synthetic Polynucleotide
<400> 60
   aatgatacgg cgaccaccga gatctacacc tagtgacaca ctctttccct acacgacgct 60
<210> 61
   <211> 60
   <212> DNA
   <213> Artificial Sequence
<220>
   <223> Synthetic Polynucleotide
<400> 61
   aatgatacgg cgaccaccga gatctacaca gaaccgtaca ctctttccct acacgacgct 60
<210> 62
   <211> 60
   <212> DNA
   <213> Artificial Sequence
<220>
   <223> Synthetic Polynucleotide
<400> 62
   aatgatacgg cgaccaccga gatctacact aattgcaaca ctctttccct acacgacgct 60
<210> 63
   <211> 60
   <212> DNA
   <213> Artificial Sequence
<220>
   <223> Synthetic Polynucleotide
<400> 63
   aatgatacgg cgaccaccga gatctacacc tagtacaaca ctctttccct acacgacgct 60
<210> 64
   <211> 60
   <212> DNA
   <213> Artificial Sequence
<220>
   <223> Synthetic Polynucleotide
<400> 64
   aatgatacgg cgaccaccga gatctacacg ctatatcaca ctctttccct acacgacgct 60
<210> 65
   <211> 60
   <212> DNA
   <213> Artificial Sequence
<220>
   <223> Synthetic Polynucleotide
<400> 65
   aatgatacgg cgaccaccga gatctacacc aatcggcaca ctctttccct acacgacgct 60
<210> 66
   <211> 60
   <212> DNA
   <213> Artificial Sequence
<220>
   <223> Synthetic Polynucleotide
<400> 66
   aatgatacgg cgaccaccga gatctacacc gatatcaaca ctctttccct acacgacgct 60
<210> 67
   <211> 60
   <212> DNA
   <213> Artificial Sequence
<220>
   <223> Synthetic Polynucleotide
<400> 67
   aatgatacgg cgaccaccga gatctacacc agtcaggaca ctctttccct acacgacgct 60
<210> 68
   <211> 60
   <212> DNA
   <213> Artificial Sequence
<220>
   <223> Synthetic Polynucleotide
<400> 68
   aatgatacgg cgaccaccga gatctacacg taataataca ctctttccct acacgacgct 60
<210> 69
   <211> 60
   <212> DNA
   <213> Artificial Sequence
<220>
   <223> Synthetic Polynucleotide
<400> 69
   aatgatacgg cgaccaccga gatctacacg gagagataca ctctttccct acacgacgct 60
<210> 70
   <211> 60
   <212> DNA
   <213> Artificial Sequence
<220>
   <223> Synthetic Polynucleotide
<400> 70
   aatgatacgg cgaccaccga gatctacacc tctcataaca ctctttccct acacgacgct 60
<210> 71
   <211> 60
   <212> DNA
   <213> Artificial Sequence
<220>
   <223> Synthetic Polynucleotide
<400> 71
   aatgatacgg cgaccaccga gatctacacc agcgactaca ctctttccct acacgacgct 60
<210> 72
   <211> 60
   <212> DNA
   <213> Artificial Sequence
<220>
   <223> Synthetic Polynucleotide
<400> 72
   aatgatacgg cgaccaccga gatctacacg gccaaggaca ctctttccct acacgacgct 60
<210> 73
   <211> 60
   <212> DNA
   <213> Artificial Sequence
<220>
   <223> Synthetic Polynucleotide
<400> 73
   aatgatacgg cgaccaccga gatctacacg catatgcaca ctctttccct acacgacgct 60
<210> 74
   <211> 60
   <212> DNA
   <213> Artificial Sequence
<220>
   <223> Synthetic Polynucleotide
<400> 74
   aatgatacgg cgaccaccga gatctacaca ctaggataca ctctttccct acacgacgct 60
<210> 75
   <211> 60
   <212> DNA
   <213> Artificial Sequence
<220>
   <223> Synthetic Polynucleotide
<400> 75
   aatgatacgg cgaccaccga gatctacacc cttacctaca ctctttccct acacgacgct 60
<210> 76
   <211> 60
   <212> DNA
   <213> Artificial Sequence
<220>
   <223> Synthetic Polynucleotide
<400> 76
   aatgatacgg cgaccaccga gatctacact gttgacgaca ctctttccct acacgacgct 60
<210> 77
   <211> 60
   <212> DNA
   <213> Artificial Sequence
<220>
   <223> Synthetic Polynucleotide
<400> 77
   aatgatacgg cgaccaccga gatctacact acagttaaca ctctttccct acacgacgct 60
<210> 78
   <211> 60
   <212> DNA
   <213> Artificial Sequence
<220>
   <223> Synthetic Polynucleotide
<400> 78
   aatgatacgg cgaccaccga gatctacact tgttacgaca ctctttccct acacgacgct 60
<210> 79
   <211> 60
   <212> DNA
   <213> Artificial Sequence
<220>
   <223> Synthetic Polynucleotide
<400> 79
   aatgatacgg cgaccaccga gatctacact cgtgttgaca ctctttccct acacgacgct 60
<210> 80
   <211> 60
   <212> DNA
   <213> Artificial Sequence
<220>
   <223> Synthetic Polynucleotide
<400> 80
   aatgatacgg cgaccaccga gatctacaca gtcaatgaca ctctttccct acacgacgct 60
<210> 81
   <211> 60
   <212> DNA
   <213> Artificial Sequence
<220>
   <223> Synthetic Polynucleotide
<400> 81
   aatgatacgg cgaccaccga gatctacact ctgtagaaca ctctttccct acacgacgct 60
<210> 82
   <211> 60
   <212> DNA
   <213> Artificial Sequence
<220>
   <223> Synthetic Polynucleotide
<400> 82
   aatgatacgg cgaccaccga gatctacacg acaacgaaca ctctttccct acacgacgct 60
<210> 83
   <211> 60
   <212> DNA
   <213> Artificial Sequence
<220>
   <223> Synthetic Polynucleotide
<400> 83
   aatgatacgg cgaccaccga gatctacacc catggctaca ctctttccct acacgacgct 60
<210> 84
   <211> 60
   <212> DNA
   <213> Artificial Sequence
<220>
   <223> Synthetic Polynucleotide
<400> 84
   aatgatacgg cgaccaccga gatctacact gactctgaca ctctttccct acacgacgct 60
<210> 85
   <211> 60
   <212> DNA
   <213> Artificial Sequence
<220>
   <223> Synthetic Polynucleotide
<400> 85
   aatgatacgg cgaccaccga gatctacaca acgaggcaca ctctttccct acacgacgct 60
<210> 86
   <211> 60
   <212> DNA
   <213> Artificial Sequence
<220>
   <223> Synthetic Polynucleotide
<400> 86
   aatgatacgg cgaccaccga gatctacacc agaaggtaca ctctttccct acacgacgct 60
<210> 87
   <211> 60
   <212> DNA
   <213> Artificial Sequence
<220>
   <223> Synthetic Polynucleotide
<400> 87
   aatgatacgg cgaccaccga gatctacact gaagtcaaca ctctttccct acacgacgct 60
<210> 88
   <211> 60
   <212> DNA
   <213> Artificial Sequence
<220>
   <223> Synthetic Polynucleotide
<400> 88
   aatgatacgg cgaccaccga gatctacaca tgttcctaca ctctttccct acacgacgct 60
<210> 89
   <211> 60
   <212> DNA
   <213> Artificial Sequence
<220>
   <223> Synthetic Polynucleotide
<400> 89
   aatgatacgg cgaccaccga gatctacaca agtggctaca ctctttccct acacgacgct 60
<210> 90
   <211> 60
   <212> DNA
   <213> Artificial Sequence
<220>
   <223> Synthetic Polynucleotide
<400> 90
   aatgatacgg cgaccaccga gatctacacg gtacaataca ctctttccct acacgacgct 60
<210> 91
   <211> 60
   <212> DNA
   <213> Artificial Sequence
<220>
   <223> Synthetic Polynucleotide
<400> 91
   aatgatacgg cgaccaccga gatctacaca caagtgcaca ctctttccct acacgacgct 60
<210> 92
   <211> 60
   <212> DNA
   <213> Artificial Sequence
<220>
   <223> Synthetic Polynucleotide
<400> 92
   aatgatacgg cgaccaccga gatctacact cacggtgaca ctctttccct acacgacgct 60
<210> 93
   <211> 60
   <212> DNA
   <213> Artificial Sequence
<220>
   <223> Synthetic Polynucleotide
<400> 93
   aatgatacgg cgaccaccga gatctacact tgcgttaaca ctctttccct acacgacgct 60
<210> 94
   <211> 60
   <212> DNA
   <213> Artificial Sequence
<220>
   <223> Synthetic Polynucleotide
<400> 94
   aatgatacgg cgaccaccga gatctacact tgtagccaca ctctttccct acacgacgct 60
<210> 95
   <211> 60
   <212> DNA
   <213> Artificial Sequence
<220>
   <223> Synthetic Polynucleotide
<400> 95
   aatgatacgg cgaccaccga gatctacact caccggaaca ctctttccct acacgacgct 60
<210> 96
   <211> 60
   <212> DNA
   <213> Artificial Sequence
<220>
   <223> Synthetic Polynucleotide
<400> 96
   aatgatacgg cgaccaccga gatctacacc gcgcaagaca ctctttccct acacgacgct 60
<210> 97
   <211> 65
   <212> DNA
   <213> Artificial Sequence
<220>
   <223> Synthetic Polynucleotide
<400> 97
   catacgattt aggtgacact atagatagat cgcagatcgg aagagcgtcg tgtagggaaa 60
   gagac 65
<210> 98
   <211> 65
   <212> DNA
   <213> Artificial Sequence
<220>
   <223> Synthetic Polynucleotide
<400> 98
   catacgattt aggtgacact atagactctc tatagatcgg aagagcgtcg tgtagggaaa 60
gagac 65
<210> 99
   <211> 65
   <212> DNA
   <213> Artificial Sequence
<220>
   <223> Synthetic Polynucleotide
<400> 99
   catacgattt aggtgacact atagatatcc tctagatcgg aagagcgtcg tgtagggaaa 60
   gagac 65
<210> 100
   <211> 65
   <212> DNA
   <213> Artificial Sequence
<220>
   <223> Synthetic Polynucleotide
<400> 100
   catacgattt aggtgacact atagaagagt agaagatcgg aagagcgtcg tgtagggaaa 60
   gagac 65
<210> 101
   <211> 65
   <212> DNA
   <213> Artificial Sequence
<220>
   <223> Synthetic Polynucleotide
<400> 101
   catacgattt aggtgacact atagagtaag gagagatcgg aagagcgtcg tgtagggaaa 60
   gagac 65
<210> 102
   <211> 65
   <212> DNA
   <213> Artificial Sequence
<220>
   <223> Synthetic Polynucleotide
<400> 102
   catacgattt aggtgacact atagaactgc ataagatcgg aagagcgtcg tgtagggaaa 60
   gagac 65
<210> 103
   <211> 65
   <212> DNA
   <213> Artificial Sequence
<220>
   <223> Synthetic Polynucleotide
<400> 103
   catacgattt aggtgacact atagaaagga gtaagatcgg aagagcgtcg tgtagggaaa 60
   gagac 65
<210> 104
   <211> 65
   <212> DNA
   <213> Artificial Sequence
<220>
   <223> Synthetic Polynucleotide
<400> 104
   catacgattt aggtgacact atagactaag cctagatcgg aagagcgtcg tgtagggaaa 60
   gagac 65
<210> 105
   <211> 65
   <212> DNA
   <213> Artificial Sequence
<220>
   <223> Synthetic Polynucleotide
<400> 105
   catacgattt aggtgacact atagagacat tgtagatcgg aagagcgtcg tgtagggaaa 60
   gagac 65
<210> 106
   <211> 65
   <212> DNA
   <213> Artificial Sequence
<220>
   <223> Synthetic Polynucleotide
<400> 106
   catacgattt aggtgacact atagaactga tggagatcgg aagagcgtcg tgtagggaaa 60
   gagac 65
<210> 107
   <211> 65
   <212> DNA
   <213> Artificial Sequence
<220>
   <223> Synthetic Polynucleotide
<400> 107
   catacgattt aggtgacact atagagtacc tagagatcgg aagagcgtcg tgtagggaaa 60
   gagac 65
<210> 108
   <211> 65
   <212> DNA
   <213> Artificial Sequence
<220>
   <223> Synthetic Polynucleotide
<400> 108
   catacgattt aggtgacact atagacagag ctaagatcgg aagagcgtcg tgtagggaaa 60
   gagac 65
<210> 109
   <211> 65
   <212> DNA
   <213> Artificial Sequence
<220>
   <223> Synthetic Polynucleotide
<400> 109
   catacgattt aggtgacact atagacatag tgaagatcgg aagagcgtcg tgtagggaaa 60
   gagac 65
<210> 110
   <211> 65
   <212> DNA
   <213> Artificial Sequence
<220>
   <223> Synthetic Polynucleotide
<400> 110
   catacgattt aggtgacact atagatacct agtagatcgg aagagcgtcg tgtagggaaa 60
   gagac 65
<210> 111
   <211> 65
   <212> DNA
   <213> Artificial Sequence
<220>
   <223> Synthetic Polynucleotide
<400> 111
   catacgattt aggtgacact atagacgcga tatagatcgg aagagcgtcg tgtagggaaa 60
   gagac 65
<210> 112
   <211> 65
   <212> DNA
   <213> Artificial Sequence
<220>
   <223> Synthetic Polynucleotide
<400> 112
   catacgattt aggtgacact atagatggat tgtagatcgg aagagcgtcg tgtagggaaa 60
   gagac 65
<210> 113
   <211> 65
   <212> DNA
   <213> Artificial Sequence
<220>
   <223> Synthetic Polynucleotide
<400> 113
   catacgattt aggtgacact atagaggact tccagatcgg aagagcgtcg tgtagggaaa 60
   gagac 65
<210> 114
   <211> 65
   <212> DNA
   <213> Artificial Sequence
<220>
   <223> Synthetic Polynucleotide
<400> 114
   catacgattt aggtgacact atagaggtat ggcagatcgg aagagcgtcg tgtagggaaa 60
   gagac 65
<210> 115
   <211> 65
   <212> DNA
   <213> Artificial Sequence
<220>
   <223> Synthetic Polynucleotide
<400> 115
   catacgattt aggtgacact atagacaata cgaagatcgg aagagcgtcg tgtagggaaa 60
   gagac 65
<210> 116
   <211> 65
   <212> DNA
   <213> Artificial Sequence
<220>
   <223> Synthetic Polynucleotide
<400> 116
   catacgattt aggtgacact atagaacagg tagagatcgg aagagcgtcg tgtagggaaa 60
   gagac 65
<210> 117
   <211> 65
   <212> DNA
   <213> Artificial Sequence
<220>
   <223> Synthetic Polynucleotide
<400> 117
   catacgattt aggtgacact atagatggag aggagatcgg aagagcgtcg tgtagggaaa 60
   gagac 65
<210> 118
   <211> 65
   <212> DNA
   <213> Artificial Sequence
<220>
   <223> Synthetic Polynucleotide
<400> 118
   catacgattt aggtgacact atagattacg tggagatcgg aagagcgtcg tgtagggaaa 60
   gagac 65
<210> 119
   <211> 65
   <212> DNA
   <213> Artificial Sequence
<220>
   <223> Synthetic Polynucleotide
<400> 119
   catacgattt aggtgacact atagaatagc cgaagatcgg aagagcgtcg tgtagggaaa 60
   gagac 65
<210> 120
   <211> 65
   <212> DNA
   <213> Artificial Sequence
<220>
   <223> Synthetic Polynucleotide
<400> 120
   catacgattt aggtgacact atagagagta tctagatcgg aagagcgtcg tgtagggaaa 60
   gagac 65
<210> 121
   <211> 65
   <212> DNA
   <213> Artificial Sequence
<220>
   <223> Synthetic Polynucleotide
<400> 121
   catacgattt aggtgacact atagagtcgt gtaagatcgg aagagcgtcg tgtagggaaa 60
   gagac 65
<210> 122
   <211> 65
   <212> DNA
   <213> Artificial Sequence
<220>
   <223> Synthetic Polynucleotide
<400> 122
   catacgattt aggtgacact atagatatcc aagagatcgg aagagcgtcg tgtagggaaa 60
   gagac 65
<210> 123
   <211> 65
   <212> DNA
   <213> Artificial Sequence
<220>
   <223> Synthetic Polynucleotide
<400> 123
   catacgattt aggtgacact atagaagtcg ctaagatcgg aagagcgtcg tgtagggaaa 60
   gagac 65
<210> 124
   <211> 65
   <212> DNA
   <213> Artificial Sequence
<220>
   <223> Synthetic Polynucleotide
<400> 124
   catacgattt aggtgacact atagagacag gttagatcgg aagagcgtcg tgtagggaaa 60
   gagac 65
<210> 125
   <211> 65
   <212> DNA
   <213> Artificial Sequence
<220>
   <223> Synthetic Polynucleotide
<400> 125
   catacgattt aggtgacact atagaagatt gacagatcgg aagagcgtcg tgtagggaaa 60
   gagac 65
<210> 126
   <211> 65
   <212> DNA
   <213> Artificial Sequence
<220>
   <223> Synthetic Polynucleotide
<400> 126
   catacgattt aggtgacact atagatatca ccgagatcgg aagagcgtcg tgtagggaaa 60
   gagac 65
<210> 127
   <211> 65
   <212> DNA
   <213> Artificial Sequence
<220>
   <223> Synthetic Polynucleotide
<400> 127
   catacgattt aggtgacact atagaatagg ctgagatcgg aagagcgtcg tgtagggaaa 60
   gagac 65
<210> 128
   <211> 65
   <212> DNA
   <213> Artificial Sequence
<220>
   <223> Synthetic Polynucleotide
<400> 128
   catacgattt aggtgacact atagaagtgg cacagatcgg aagagcgtcg tgtagggaaa 60
   gagac 65
<210> 129
   <211> 65
   <212> DNA
   <213> Artificial Sequence
<220>
   <223> Synthetic Polynucleotide
<400> 129
   catacgattt aggtgacact atagaactca tctagatcgg aagagcgtcg tgtagggaaa 60
   gagac 65
<210> 130
   <211> 65
   <212> DNA
   <213> Artificial Sequence
<220>
   <223> Synthetic Polynucleotide
<400> 130
   catacgattt aggtgacact atagagcagc catagatcgg aagagcgtcg tgtagggaaa 60
   gagac 65
<210> 131
   <211> 65
   <212> DNA
   <213> Artificial Sequence
<220>
   <223> Synthetic Polynucleotide
<400> 131
   catacgattt aggtgacact atagattgca gtgagatcgg aagagcgtcg tgtagggaaa 60
   gagac 65
<210> 132
   <211> 65
   <212> DNA
   <213> Artificial Sequence
<220>
   <223> Synthetic Polynucleotide
<400> 132
   catacgattt aggtgacact atagacgact gcaagatcgg aagagcgtcg tgtagggaaa      60
   gagac                                                                  65
<210> 133
   <211> 65
   <212> DNA
   <213> Artificial Sequence
<220>
   <223> Synthetic Polynucleotide
<400> 133
   catacgattt aggtgacact atagacggtc aatagatcgg aagagcgtcg tgtagggaaa      60
   gagac                                                                  65
<210> 134
   <211> 65
   <212> DNA
   <213> Artificial Sequence
<220>
   <223> Synthetic Polynucleotide
<400> 134
   catacgattt aggtgacact atagagctgc tacagatcgg aagagcgtcg tgtagggaaa      60
   gagac                                                                  65
<210> 135
   <211> 65
   <212> DNA
   <213> Artificial Sequence
<220>
   <223> Synthetic Polynucleotide
<400> 135
   catacgattt aggtgacact atagagcagt ctaagatcgg aagagcgtcg tgtagggaaa      60
   gagac                                                                  65
<210> 136
   <211> 65
   <212> DNA
   <213> Artificial Sequence
<220>
   <223> Synthetic Polynucleotide
<400> 136
   catacgattt aggtgacact atagatggac cacagatcgg aagagcgtcg tgtagggaaa 60
   gagac 65
<210> 137
   <211> 65
   <212> DNA
   <213> Artificial Sequence
<220>
   <223> Synthetic Polynucleotide
<400> 137
   catacgattt aggtgacact atagagtcac atcagatcgg aagagcgtcg tgtagggaaa 60
   gagac 65
<210> 138
   <211> 65
   <212> DNA
   <213> Artificial Sequence
<220>
   <223> Synthetic Polynucleotide
<400> 138
   catacgattt aggtgacact atagagttgc tgaagatcgg aagagcgtcg tgtagggaaa 60
   gagac 65
<210> 139
   <211> 65
   <212> DNA
   <213> Artificial Sequence
<220>
   <223> Synthetic Polynucleotide
<400> 139
   catacgattt aggtgacact atagagagtt agcagatcgg aagagcgtcg tgtagggaaa 60
   gagac 65
<210> 140
   <211> 65
   <212> DNA
   <213> Artificial Sequence
<220>
   <223> Synthetic Polynucleotide
<400> 140
   catacgattt aggtgacact atagaacgat catagatcgg aagagcgtcg tgtagggaaa 60
   gagac 65
<210> 141
   <211> 65
   <212> DNA
   <213> Artificial Sequence
<220>
   <223> Synthetic Polynucleotide
<400> 141
   catacgattt aggtgacact atagacgtcg tctagatcgg aagagcgtcg tgtagggaaa 60
   gagac 65
<210> 142
   <211> 65
   <212> DNA
   <213> Artificial Sequence
<220>
   <223> Synthetic Polynucleotide
<400> 142
   catacgattt aggtgacact atagagacat gcgagatcgg aagagcgtcg tgtagggaaa 60
   gagac 65
<210> 143
   <211> 65
   <212> DNA
   <213> Artificial Sequence
<220>
   <223> Synthetic Polynucleotide
<400> 143
   catacgattt aggtgacact atagagtgcc ataagatcgg aagagcgtcg tgtagggaaa 60
   gagac 65
<210> 144
   <211> 65
   <212> DNA
   <213> Artificial Sequence
<220>
   <223> Synthetic Polynucleotide
<400> 144
   catacgattt aggtgacact atagacgcta ggaagatcgg aagagcgtcg tgtagggaaa 60
   gagac 65
<210> 145
   <211> 65
   <212> DNA
   <213> Artificial Sequence
<220>
   <223> Synthetic Polynucleotide
<400> 145
   catacgattt aggtgacact atagacgtag gtaagatcgg aagagcgtcg tgtagggaaa 60
   gagac 65
<210> 146
   <211> 65
   <212> DNA
   <213> Artificial Sequence
<220>
   <223> Synthetic Polynucleotide
<400> 146
   catacgattt aggtgacact atagaagcta gcgagatcgg aagagcgtcg tgtagggaaa 60
   gagac 65
<210> 147
   <211> 65
   <212> DNA
   <213> Artificial Sequence
<220>
   <223> Synthetic Polynucleotide
<400> 147
   catacgattt aggtgacact atagatcctg tgcagatcgg aagagcgtcg tgtagggaaa 60
   gagac 65
<210> 148
   <211> 65
   <212> DNA
   <213> Artificial Sequence
<220>
   <223> Synthetic Polynucleotide
<400> 148
   catacgattt aggtgacact atagagtaat ctgagatcgg aagagcgtcg tgtagggaaa 60
   gagac 65
<210> 149
   <211> 65
   <212> DNA
   <213> Artificial Sequence
<220>
   <223> Synthetic Polynucleotide
<400> 149
   catacgattt aggtgacact atagaaacgt aggagatcgg aagagcgtcg tgtagggaaa 60
   gagac 65
<210> 150
   <211> 65
   <212> DNA
   <213> Artificial Sequence
<220>
   <223> Synthetic Polynucleotide
<400> 150
   catacgattt aggtgacact atagattcct gttagatcgg aagagcgtcg tgtagggaaa 60
   gagac 65
<210> 151
   <211> 65
   <212> DNA
   <213> Artificial Sequence
<220>
   <223> Synthetic Polynucleotide
<400> 151
   catacgattt aggtgacact atagatgtcc agtagatcgg aagagcgtcg tgtagggaaa 60
   gagac 65
<210> 152
   <211> 65
   <212> DNA
   <213> Artificial Sequence
<220>
   <223> Synthetic Polynucleotide
<400> 152
   catacgattt aggtgacact atagaacaag gcaagatcgg aagagcgtcg tgtagggaaa 60
   gagac 65
<210> 153
   <211> 65
   <212> DNA
   <213> Artificial Sequence
<220>
   <223> Synthetic Polynucleotide
<400> 153
   catacgattt aggtgacact atagaccttg accagatcgg aagagcgtcg tgtagggaaa 60
   gagac 65
<210> 154
   <211> 65
   <212> DNA
   <213> Artificial Sequence
<220>
   <223> Synthetic Polynucleotide
<400> 154
   catacgattt aggtgacact atagacgctt gtgagatcgg aagagcgtcg tgtagggaaa 60
   gagac 65
<210> 155
   <211> 65
   <212> DNA
   <213> Artificial Sequence
<220>
   <223> Synthetic Polynucleotide
<400> 155
   catacgattt aggtgacact atagatccaa gcgagatcgg aagagcgtcg tgtagggaaa 60
   gagac 65
<210> 156
   <211> 65
   <212> DNA
   <213> Artificial Sequence
<220>
   <223> Synthetic Polynucleotide
<400> 156
   catacgattt aggtgacact atagactagt gacagatcgg aagagcgtcg tgtagggaaa 60
   gagac 65
<210> 157
   <211> 65
   <212> DNA
   <213> Artificial Sequence
<220>
   <223> Synthetic Polynucleotide
<400> 157
   catacgattt aggtgacact atagaagaac cgtagatcgg aagagcgtcg tgtagggaaa 60
   gagac 65
<210> 158
   <211> 65
   <212> DNA
   <213> Artificial Sequence
<220>
   <223> Synthetic Polynucleotide
<400> 158
   catacgattt aggtgacact atagataatt gcaagatcgg aagagcgtcg tgtagggaaa 60
   gagac 65
<210> 159
   <211> 65
   <212> DNA
   <213> Artificial Sequence
<220>
   <223> Synthetic Polynucleotide
<400> 159
   catacgattt aggtgacact atagactagt acaagatcgg aagagcgtcg tgtagggaaa 60
   gagac 65
<210> 160
   <211> 65
   <212> DNA
   <213> Artificial Sequence
<220>
   <223> Synthetic Polynucleotide
<400> 160
   catacgattt aggtgacact atagagctat atcagatcgg aagagcgtcg tgtagggaaa 60
   gagac 65
<210> 161
   <211> 65
   <212> DNA
   <213> Artificial Sequence
<220>
   <223> Synthetic Polynucleotide
<400> 161
   catacgattt aggtgacact atagacaatc ggcagatcgg aagagcgtcg tgtagggaaa 60
   gagac 65
<210> 162
   <211> 65
   <212> DNA
   <213> Artificial Sequence
<220>
   <223> Synthetic Polynucleotide
<400> 162
   catacgattt aggtgacact atagacgata tcaagatcgg aagagcgtcg tgtagggaaa 60
   gagac 65
<210> 163
   <211> 65
   <212> DNA
   <213> Artificial Sequence
<220>
   <223> Synthetic Polynucleotide
<400> 163
   catacgattt aggtgacact atagacagtc aggagatcgg aagagcgtcg tgtagggaaa 60
   gagac 65
<210> 164
   <211> 65
   <212> DNA
   <213> Artificial Sequence
<220>
   <223> Synthetic Polynucleotide
<400> 164
   catacgattt aggtgacact atagagtaat aatagatcgg aagagcgtcg tgtagggaaa 60
   gagac 65
<210> 165
   <211> 65
   <212> DNA
   <213> Artificial Sequence
<220>
   <223> Synthetic Polynucleotide
<400> 165
   catacgattt aggtgacact atagaggaga gatagatcgg aagagcgtcg tgtagggaaa 60
   gagac 65
<210> 166
   <211> 65
   <212> DNA
   <213> Artificial Sequence
<220>
   <223> Synthetic Polynucleotide
<400> 166
   catacgattt aggtgacact atagactctc ataagatcgg aagagcgtcg tgtagggaaa 60
   gagac 65
<210> 167
   <211> 65
   <212> DNA
   <213> Artificial Sequence
<220>
   <223> Synthetic Polynucleotide
<400> 167
   catacgattt aggtgacact atagacagcg actagatcgg aagagcgtcg tgtagggaaa 60
   gagac 65
<210> 168
   <211> 65
   <212> DNA
   <213> Artificial Sequence
<220>
   <223> Synthetic Polynucleotide
<400> 168
   catacgattt aggtgacact atagaggcca aggagatcgg aagagcgtcg tgtagggaaa 60
   gagac 65
<210> 169
   <211> 65
   <212> DNA
   <213> Artificial Sequence
<220>
   <223> Synthetic Polynucleotide
<400> 169
   catacgattt aggtgacact atagagcata tgcagatcgg aagagcgtcg tgtagggaaa 60
   gagac 65
<210> 170
   <211> 65
   <212> DNA
   <213> Artificial Sequence
<220>
   <223> Synthetic Polynucleotide
<400> 170
   catacgattt aggtgacact atagaactag gatagatcgg aagagcgtcg tgtagggaaa 60
   gagac 65
<210> 171
   <211> 65
   <212> DNA
   <213> Artificial Sequence
<220>
   <223> Synthetic Polynucleotide
<400> 171
   catacgattt aggtgacact atagacctta cctagatcgg aagagcgtcg tgtagggaaa 60
   gagac 65
<210> 172
   <211> 65
   <212> DNA
   <213> Artificial Sequence
<220>
   <223> Synthetic Polynucleotide
<400> 172
   catacgattt aggtgacact atagatgttg acgagatcgg aagagcgtcg tgtagggaaa 60
   gagac 65
<210> 173
   <211> 65
   <212> DNA
   <213> Artificial Sequence
<220>
   <223> Synthetic Polynucleotide
<400> 173
   catacgattt aggtgacact atagatacag ttaagatcgg aagagcgtcg tgtagggaaa 60
   gagac 65
<210> 174
   <211> 65
   <212> DNA
   <213> Artificial Sequence
<220>
   <223> Synthetic Polynucleotide
<400> 174
   catacgattt aggtgacact atagattgtt acgagatcgg aagagcgtcg tgtagggaaa 60
   gagac 65
<210> 175
   <211> 65
   <212> DNA
   <213> Artificial Sequence
<220>
   <223> Synthetic Polynucleotide
<400> 175
   catacgattt aggtgacact atagatcgtg ttgagatcgg aagagcgtcg tgtagggaaa 60
   gagac 65
<210> 176
   <211> 65
   <212> DNA
   <213> Artificial Sequence
<220>
   <223> Synthetic Polynucleotide
<400> 176
   catacgattt aggtgacact atagaagtca atgagatcgg aagagcgtcg tgtagggaaa 60
   gagac 65
<210> 177
   <211> 65
   <212> DNA
   <213> Artificial Sequence
<220>
   <223> Synthetic Polynucleotide
<400> 177
   catacgattt aggtgacact atagatctgt agaagatcgg aagagcgtcg tgtagggaaa 60
   gagac 65
<210> 178
   <211> 65
   <212> DNA
   <213> Artificial Sequence
<220>
   <223> Synthetic Polynucleotide
<400> 178
   catacgattt aggtgacact atagagacaa cgaagatcgg aagagcgtcg tgtagggaaa 60
   gagac 65
<210> 179
   <211> 65
   <212> DNA
   <213> Artificial Sequence
<220>
   <223> Synthetic Polynucleotide
<400> 179
   catacgattt aggtgacact atagaccatg gctagatcgg aagagcgtcg tgtagggaaa 60
   gagac 65
<210> 180
   <211> 65
   <212> DNA
   <213> Artificial Sequence
<220>
   <223> Synthetic Polynucleotide
<400> 180
   catacgattt aggtgacact atagatgact ctgagatcgg aagagcgtcg tgtagggaaa 60
   gagac 65
<210> 181
   <211> 65
   <212> DNA
   <213> Artificial Sequence
<220>
   <223> Synthetic Polynucleotide
<400> 181
   catacgattt aggtgacact atagaaacga ggcagatcgg aagagcgtcg tgtagggaaa 60
   gagac 65
<210> 182
   <211> 65
   <212> DNA
   <213> Artificial Sequence
<220>
   <223> Synthetic Polynucleotide
<400> 182
   catacgattt aggtgacact atagacagaa ggtagatcgg aagagcgtcg tgtagggaaa 60
   gagac 65
<210> 183
   <211> 65
   <212> DNA
   <213> Artificial Sequence
<220>
   <223> Synthetic Polynucleotide
<400> 183
   catacgattt aggtgacact atagatgaag tcaagatcgg aagagcgtcg tgtagggaaa 60
   gagac 65
<210> 184
   <211> 65
   <212> DNA
   <213> Artificial Sequence
<220>
   <223> Synthetic Polynucleotide
<400> 184
   catacgattt aggtgacact atagaatgtt cctagatcgg aagagcgtcg tgtagggaaa 60
   gagac 65
<210> 185
   <211> 65
   <212> DNA
   <213> Artificial Sequence
<220>
   <223> Synthetic Polynucleotide
<400> 185
   catacgattt aggtgacact atagaaagtg gctagatcgg aagagcgtcg tgtagggaaa 60
   gagac 65
<210> 186
   <211> 65
   <212> DNA
   <213> Artificial Sequence
<220>
   <223> Synthetic Polynucleotide
<400> 186
   catacgattt aggtgacact atagaggtac aatagatcgg aagagcgtcg tgtagggaaa 60
   gagac 65
<210> 187
   <211> 65
   <212> DNA
   <213> Artificial Sequence
<220>
   <223> Synthetic Polynucleotide
<400> 187
   catacgattt aggtgacact atagaacaag tgcagatcgg aagagcgtcg tgtagggaaa 60
   gagac 65
<210> 188
   <211> 65
   <212> DNA
   <213> Artificial Sequence
<220>
   <223> Synthetic Polynucleotide
<400> 188 gagac 65
<210> 189
   <211> 65
   <212> DNA
   <213> Artificial Sequence
<220>
   <223> Synthetic Polynucleotide
<400> 189
   catacgattt aggtgacact atagattgcg ttaagatcgg aagagcgtcg tgtagggaaa 60
   gagac 65
<210> 190
   <211> 65
   <212> DNA
   <213> Artificial Sequence
<220>
   <223> Synthetic Polynucleotide
<400> 190
   catacgattt aggtgacact atagattgta gccagatcgg aagagcgtcg tgtagggaaa 60
   gagac 65
<210> 191
   <211> 65
   <212> DNA
   <213> Artificial Sequence
<220>
   <223> Synthetic Polynucleotide
<400> 191
   catacgattt aggtgacact atagatcacc ggaagatcgg aagagcgtcg tgtagggaaa 60
   gagac 65
<210> 192
   <211> 65
   <212> DNA
   <213> Artificial Sequence
<220>
   <223> Synthetic Polynucleotide
<400> 192
   catacgattt aggtgacact atagacgcgc aagagatcgg aagagcgtcg tgtagggaaa 60
   gagac 65
<210> 193
   <211> 58
   <212> DNA
   <213> Artificial Sequence
<220>
   <223> Synthetic Polynucleotide
<400> 193
   tctatagtgt cacctaaatc gtatgtagat cgccaggagc aaggtgagat gacaggag 58
<210> 194
   <211> 58
   <212> DNA
   <213> Artificial Sequence
<220>
   <223> Synthetic Polynucleotide
<400> 194
   tctatagtgt cacctaaatc gtatgctctc tatcaggagc aaggtgagat gacaggag 58
<210> 195
   <211> 58
   <212> DNA
   <213> Artificial Sequence
<220>
   <223> Synthetic Polynucleotide
<400> 195
   tctatagtgt cacctaaatc gtatgtatcc tctcaggagc aaggtgagat gacaggag 58
<210> 196
   <211> 58
   <212> DNA
   <213> Artificial Sequence
<220>
   <223> Synthetic Polynucleotide
<400> 196
   tctatagtgt cacctaaatc gtatgagagt agacaggagc aaggtgagat gacaggag 58
<210> 197
   <211> 58
   <212> DNA
   <213> Artificial Sequence
<220>
   <223> Synthetic Polynucleotide
<400> 197
   tctatagtgt cacctaaatc gtatggtaag gagcaggagc aaggtgagat gacaggag 58
<210> 198
   <211> 58
   <212> DNA
   <213> Artificial Sequence
<220>
   <223> Synthetic Polynucleotide
<400> 198
   tctatagtgt cacctaaatc gtatgactgc atacaggagc aaggtgagat gacaggag 58
<210> 199
   <211> 58
   <212> DNA
   <213> Artificial Sequence
<220>
   <223> Synthetic Polynucleotide
<400> 199
   tctatagtgt cacctaaatc gtatgaagga gtacaggagc aaggtgagat gacaggag 58
<210> 200
   <211> 58
   <212> DNA
   <213> Artificial Sequence
<220>
   <223> Synthetic Polynucleotide
<400> 200
   tctatagtgt cacctaaatc gtatgctaag cctcaggagc aaggtgagat gacaggag 58
<210> 201
   <211> 58
   <212> DNA
   <213> Artificial Sequence
<220>
   <223> Synthetic Polynucleotide
<400> 201
   tctatagtgt cacctaaatc gtatggacat tgtcaggagc aaggtgagat gacaggag 58
<210> 202
   <211> 58
   <212> DNA
   <213> Artificial Sequence
<220>
   <223> Synthetic Polynucleotide
<400> 202
   tctatagtgt cacctaaatc gtatgactga tggcaggagc aaggtgagat gacaggag 58
<210> 203
   <211> 58
   <212> DNA
   <213> Artificial Sequence
<220>
   <223> Synthetic Polynucleotide
<400> 203
   tctatagtgt cacctaaatc gtatggtacc tagcaggagc aaggtgagat gacaggag 58
<210> 204
   <211> 58
   <212> DNA
   <213> Artificial Sequence
<220>
   <223> Synthetic Polynucleotide
<400> 204
   tctatagtgt cacctaaatc gtatgcagag ctacaggagc aaggtgagat gacaggag 58
<210> 205
   <211> 58
   <212> DNA
   <213> Artificial Sequence
<220>
   <223> Synthetic Polynucleotide
<400> 205
   tctatagtgt cacctaaatc gtatgcatag tgacaggagc aaggtgagat gacaggag 58
<210> 206
   <211> 58
   <212> DNA
   <213> Artificial Sequence
<220>
   <223> Synthetic Polynucleotide
<400> 206
   tctatagtgt cacctaaatc gtatgtacct agtcaggagc aaggtgagat gacaggag 58
<210> 207
   <211> 58
   <212> DNA
   <213> Artificial Sequence
<220>
   <223> Synthetic Polynucleotide
<400> 207
   tctatagtgt cacctaaatc gtatgcgcga tatcaggagc aaggtgagat gacaggag 58
<210> 208
   <211> 58
   <212> DNA
   <213> Artificial Sequence
<220>
   <223> Synthetic Polynucleotide
<400> 208
   tctatagtgt cacctaaatc gtatgtggat tgtcaggagc aaggtgagat gacaggag 58
<210> 209
   <211> 58
   <212> DNA
   <213> Artificial Sequence
<220>
   <223> Synthetic Polynucleotide
<400> 209
   tctatagtgt cacctaaatc gtatgggact tcccaggagc aaggtgagat gacaggag 58
<210> 210
   <211> 58
   <212> DNA
   <213> Artificial Sequence
<220>
   <223> Synthetic Polynucleotide
<400> 210
   tctatagtgt cacctaaatc gtatgggtat ggccaggagc aaggtgagat gacaggag 58
<210> 211
   <211> 58
   <212> DNA
   <213> Artificial Sequence
<220>
   <223> Synthetic Polynucleotide
<400> 211
   tctatagtgt cacctaaatc gtatgcaata cgacaggagc aaggtgagat gacaggag 58
<210> 212
   <211> 58
   <212> DNA
   <213> Artificial Sequence
<220>
   <223> Synthetic Polynucleotide
<400> 212
   tctatagtgt cacctaaatc gtatgacagg tagcaggagc aaggtgagat gacaggag 58
<210> 213
   <211> 58
   <212> DNA
   <213> Artificial Sequence
<220>
   <223> Synthetic Polynucleotide
<400> 213
   tctatagtgt cacctaaatc gtatgtggag aggcaggagc aaggtgagat gacaggag 58
<210> 214
   <211> 58
   <212> DNA
   <213> Artificial Sequence
<220>
   <223> Synthetic Polynucleotide
<400> 214
   tctatagtgt cacctaaatc gtatgttacg tggcaggagc aaggtgagat gacaggag 58
<210> 215
   <211> 58
   <212> DNA
   <213> Artificial Sequence
<220>
   <223> Synthetic Polynucleotide
<400> 215
   tctatagtgt cacctaaatc gtatgatagc cgacaggagc aaggtgagat gacaggag 58
<210> 216
   <211> 58
   <212> DNA
   <213> Artificial Sequence
<220>
   <223> Synthetic Polynucleotide
<400> 216
   tctatagtgt cacctaaatc gtatggagta tctcaggagc aaggtgagat gacaggag 58
<210> 217
   <211> 58
   <212> DNA
   <213> Artificial Sequence
<220>
   <223> Synthetic Polynucleotide
<400> 217
   tctatagtgt cacctaaatc gtatggtcgt gtacaggagc aaggtgagat gacaggag 58
<210> 218
   <211> 58
   <212> DNA
   <213> Artificial Sequence
<220>
   <223> Synthetic Polynucleotide
<400> 218
   tctatagtgt cacctaaatc gtatgtatcc aagcaggagc aaggtgagat gacaggag 58
<210> 219
   <211> 58
   <212> DNA
   <213> Artificial Sequence
<220>
   <223> Synthetic Polynucleotide
<400> 219
   tctatagtgt cacctaaatc gtatgagtcg ctacaggagc aaggtgagat gacaggag 58
<210> 220
   <211> 58
   <212> DNA
   <213> Artificial Sequence
<220>
   <223> Synthetic Polynucleotide
<400> 220
   tctatagtgt cacctaaatc gtatggacag gttcaggagc aaggtgagat gacaggag 58
<210> 221
   <211> 58
   <212> DNA
   <213> Artificial Sequence
<220>
   <223> Synthetic Polynucleotide
<400> 221
   tctatagtgt cacctaaatc gtatgagatt gaccaggagc aaggtgagat gacaggag 58
<210> 222
   <211> 58
   <212> DNA
   <213> Artificial Sequence
<220>
   <223> Synthetic Polynucleotide
<400> 222
   tctatagtgt cacctaaatc gtatgtatca ccgcaggagc aaggtgagat gacaggag 58
<210> 223
   <211> 58
   <212> DNA
   <213> Artificial Sequence
<220>
   <223> Synthetic Polynucleotide
<400> 223
   tctatagtgt cacctaaatc gtatgatagg ctgcaggagc aaggtgagat gacaggag 58
<210> 224
   <211> 58
   <212> DNA
   <213> Artificial Sequence
<220>
   <223> Synthetic Polynucleotide
<400> 224
   tctatagtgt cacctaaatc gtatgagtgg caccaggagc aaggtgagat gacaggag 58
<210> 225
   <211> 58
   <212> DNA
   <213> Artificial Sequence
<220>
   <223> Synthetic Polynucleotide
<400> 225
   tctatagtgt cacctaaatc gtatgactca tctcaggagc aaggtgagat gacaggag 58
<210> 226
   <211> 58
   <212> DNA
   <213> Artificial Sequence
<220>
   <223> Synthetic Polynucleotide
<400> 226
   tctatagtgt cacctaaatc gtatggcagc catcaggagc aaggtgagat gacaggag 58
<210> 227
   <211> 58
   <212> DNA
   <213> Artificial Sequence
<220>
   <223> Synthetic Polynucleotide
<400> 227
   tctatagtgt cacctaaatc gtatgttgca gtgcaggagc aaggtgagat gacaggag 58
<210> 228
   <211> 58
   <212> DNA
   <213> Artificial Sequence
<220>
   <223> Synthetic Polynucleotide
<400> 228
   tctatagtgt cacctaaatc gtatgcgact gcacaggagc aaggtgagat gacaggag 58
<210> 229
   <211> 58
   <212> DNA
   <213> Artificial Sequence
<220>
   <223> Synthetic Polynucleotide
<400> 229
   tctatagtgt cacctaaatc gtatgcggtc aatcaggagc aaggtgagat gacaggag 58
<210> 230
   <211> 58
   <212> DNA
   <213> Artificial Sequence
<220>
   <223> Synthetic Polynucleotide
<400> 230
   tctatagtgt cacctaaatc gtatggctgc taccaggagc aaggtgagat gacaggag 58
<210> 231
   <211> 58
   <212> DNA
   <213> Artificial Sequence
<220>
   <223> Synthetic Polynucleotide
<400> 231
   tctatagtgt cacctaaatc gtatggcagt ctacaggagc aaggtgagat gacaggag 58
<210> 232
   <211> 58
   <212> DNA
   <213> Artificial Sequence
<220>
   <223> Synthetic Polynucleotide
<400> 232
   tctatagtgt cacctaaatc gtatgtggac caccaggagc aaggtgagat gacaggag 58
<210> 233
   <211> 58
   <212> DNA
   <213> Artificial Sequence
<220>
   <223> Synthetic Polynucleotide
<400> 233
   tctatagtgt cacctaaatc gtatggtcac atccaggagc aaggtgagat gacaggag 58
<210> 234
   <211> 58
   <212> DNA
   <213> Artificial Sequence
<220>
   <223> Synthetic Polynucleotide
<400> 234
   tctatagtgt cacctaaatc gtatggttgc tgacaggagc aaggtgagat gacaggag 58
<210> 235
   <211> 58
   <212> DNA
   <213> Artificial Sequence
<220>
   <223> Synthetic Polynucleotide
<400> 235
   tctatagtgt cacctaaatc gtatggagtt agccaggagc aaggtgagat gacaggag 58
<210> 236
   <211> 58
   <212> DNA
   <213> Artificial Sequence
<220>
   <223> Synthetic Polynucleotide
<400> 236
   tctatagtgt cacctaaatc gtatgacgat catcaggagc aaggtgagat gacaggag 58
<210> 237
   <211> 58
   <212> DNA
   <213> Artificial Sequence
<220>
   <223> Synthetic Polynucleotide
<400> 237
   tctatagtgt cacctaaatc gtatgcgtcg tctcaggagc aaggtgagat gacaggag 58
<210> 238
   <211> 58
   <212> DNA
   <213> Artificial Sequence
<220>
   <223> Synthetic Polynucleotide
<400> 238
   tctatagtgt cacctaaatc gtatggacat gcgcaggagc aaggtgagat gacaggag 58
<210> 239
   <211> 58
   <212> DNA
   <213> Artificial Sequence
<220>
   <223> Synthetic Polynucleotide
<400> 239
   tctatagtgt cacctaaatc gtatggtgcc atacaggagc aaggtgagat gacaggag 58
<210> 240
   <211> 58
   <212> DNA
   <213> Artificial Sequence
<220>
   <223> Synthetic Polynucleotide
<400> 240
   tctatagtgt cacctaaatc gtatgcgcta ggacaggagc aaggtgagat gacaggag 58
<210> 241
   <211> 58
   <212> DNA
   <213> Artificial Sequence
<220>
   <223> Synthetic Polynucleotide
<400> 241
   tctatagtgt cacctaaatc gtatgcgtag gtacaggagc aaggtgagat gacaggag 58
<210> 242
   <211> 58
   <212> DNA
   <213> Artificial Sequence
<220>
   <223> Synthetic Polynucleotide
<400> 242
   tctatagtgt cacctaaatc gtatgagcta gcgcaggagc aaggtgagat gacaggag 58
<210> 243
   <211> 58
   <212> DNA
   <213> Artificial Sequence
<220>
   <223> Synthetic Polynucleotide
<400> 243
   tctatagtgt cacctaaatc gtatgtcctg tgccaggagc aaggtgagat gacaggag 58
<210> 244
   <211> 58
   <212> DNA
   <213> Artificial Sequence
<220>
   <223> Synthetic Polynucleotide
<400> 244
   tctatagtgt cacctaaatc gtatggtaat ctgcaggagc aaggtgagat gacaggag 58
<210> 245
   <211> 58
   <212> DNA
   <213> Artificial Sequence
<220>
   <223> Synthetic Polynucleotide
<400> 245
   tctatagtgt cacctaaatc gtatgaacgt aggcaggagc aaggtgagat gacaggag 58
<210> 246
   <211> 58
   <212> DNA
   <213> Artificial Sequence
<220>
   <223> Synthetic Polynucleotide
<400> 246
   tctatagtgt cacctaaatc gtatgttcct gttcaggagc aaggtgagat gacaggag 58
<210> 247
   <211> 58
   <212> DNA
   <213> Artificial Sequence
<220>
   <223> Synthetic Polynucleotide
<400> 247
   tctatagtgt cacctaaatc gtatgtgtcc agtcaggagc aaggtgagat gacaggag 58
<210> 248
   <211> 58
   <212> DNA
   <213> Artificial Sequence
<220>
   <223> Synthetic Polynucleotide
<400> 248
   tctatagtgt cacctaaatc gtatgacaag gcacaggagc aaggtgagat gacaggag 58
<210> 249
   <211> 58
   <212> DNA
   <213> Artificial Sequence
<220>
   <223> Synthetic Polynucleotide
<400> 249
   tctatagtgt cacctaaatc gtatgccttg acccaggagc aaggtgagat gacaggag 58
<210> 250
   <211> 58
   <212> DNA
   <213> Artificial Sequence
<220>
   <223> Synthetic Polynucleotide
<400> 250
   tctatagtgt cacctaaatc gtatgcgctt gtgcaggagc aaggtgagat gacaggag 58
<210> 251
   <211> 58
   <212> DNA
   <213> Artificial Sequence
<220>
   <223> Synthetic Polynucleotide
<400> 251
   tctatagtgt cacctaaatc gtatgtccaa gcgcaggagc aaggtgagat gacaggag 58
<210> 252
   <211> 58
   <212> DNA
   <213> Artificial Sequence
<220>
   <223> Synthetic Polynucleotide
<400> 252
   tctatagtgt cacctaaatc gtatgctagt gaccaggagc aaggtgagat gacaggag 58
<210> 253
   <211> 58
   <212> DNA
   <213> Artificial Sequence
<220>
   <223> Synthetic Polynucleotide
<400> 253
   tctatagtgt cacctaaatc gtatgagaac cgtcaggagc aaggtgagat gacaggag 58
<210> 254
   <211> 58
   <212> DNA
   <213> Artificial Sequence
<220>
   <223> Synthetic Polynucleotide
<400> 254
   tctatagtgt cacctaaatc gtatgtaatt gcacaggagc aaggtgagat gacaggag 58
<210> 255
   <211> 58
   <212> DNA
   <213> Artificial Sequence
<220>
   <223> Synthetic Polynucleotide
<400> 255
   tctatagtgt cacctaaatc gtatgctagt acacaggagc aaggtgagat gacaggag 58
<210> 256
   <211> 58
   <212> DNA
   <213> Artificial Sequence
<220>
   <223> Synthetic Polynucleotide
<400> 256
   tctatagtgt cacctaaatc gtatggctat atccaggagc aaggtgagat gacaggag 58
<210> 257
   <211> 58
   <212> DNA
   <213> Artificial Sequence
<220>
   <223> Synthetic Polynucleotide
<400> 257
   tctatagtgt cacctaaatc gtatgcaatc ggccaggagc aaggtgagat gacaggag 58
<210> 258
   <211> 58
   <212> DNA
   <213> Artificial Sequence
<220>
   <223> Synthetic Polynucleotide
<400> 258
   tctatagtgt cacctaaatc gtatgcgata tcacaggagc aaggtgagat gacaggag 58
<210> 259
   <211> 58
   <212> DNA
   <213> Artificial Sequence
<220>
   <223> Synthetic Polynucleotide
<400> 259
   tctatagtgt cacctaaatc gtatgcagtc aggcaggagc aaggtgagat gacaggag 58
<210> 260
   <211> 58
   <212> DNA
   <213> Artificial Sequence
<220>
   <223> Synthetic Polynucleotide
<400> 260
   tctatagtgt cacctaaatc gtatggtaat aatcaggagc aaggtgagat gacaggag 58
<210> 261
   <211> 58
   <212> DNA
   <213> Artificial Sequence
<220>
   <223> Synthetic Polynucleotide
<400> 261
   tctatagtgt cacctaaatc gtatgggaga gatcaggagc aaggtgagat gacaggag 58
<210> 262
   <211> 58
   <212> DNA
   <213> Artificial Sequence
<220>
   <223> Synthetic Polynucleotide
<400> 262
   tctatagtgt cacctaaatc gtatgctctc atacaggagc aaggtgagat gacaggag 58
<210> 263
   <211> 58
   <212> DNA
   <213> Artificial Sequence
<220>
   <223> Synthetic Polynucleotide
<400> 263
   tctatagtgt cacctaaatc gtatgcagcg actcaggagc aaggtgagat gacaggag 58
<210> 264
   <211> 58
   <212> DNA
   <213> Artificial Sequence
<220>
   <223> Synthetic Polynucleotide
<400> 264
   tctatagtgt cacctaaatc gtatgggcca aggcaggagc aaggtgagat gacaggag 58
<210> 265
   <211> 58
   <212> DNA
   <213> Artificial Sequence
<220>
   <223> Synthetic Polynucleotide
<400> 265
   tctatagtgt cacctaaatc gtatggcata tgccaggagc aaggtgagat gacaggag 58
<210> 266
   <211> 58
   <212> DNA
   <213> Artificial Sequence
<220>
   <223> Synthetic Polynucleotide
<400> 266
   tctatagtgt cacctaaatc gtatgactag gatcaggagc aaggtgagat gacaggag 58
<210> 267
   <211> 58
   <212> DNA
   <213> Artificial Sequence
<220>
   <223> Synthetic Polynucleotide
<400> 267
   tctatagtgt cacctaaatc gtatgcctta cctcaggagc aaggtgagat gacaggag 58
<210> 268
   <211> 58
   <212> DNA
   <213> Artificial Sequence
<220>
   <223> Synthetic Polynucleotide
<400> 268
   tctatagtgt cacctaaatc gtatgtgttg acgcaggagc aaggtgagat gacaggag 58
<210> 269
   <211> 58
   <212> DNA
   <213> Artificial Sequence
<220>
   <223> Synthetic Polynucleotide
<400> 269
   tctatagtgt cacctaaatc gtatgtacag ttacaggagc aaggtgagat gacaggag 58
<210> 270
   <211> 58
   <212> DNA
   <213> Artificial Sequence
<220>
   <223> Synthetic Polynucleotide
<400> 270
   tctatagtgt cacctaaatc gtatgttgtt acgcaggagc aaggtgagat gacaggag 58
<210> 271
   <211> 58
   <212> DNA
   <213> Artificial Sequence
<220>
   <223> Synthetic Polynucleotide
<400> 271
   tctatagtgt cacctaaatc gtatgtcgtg ttgcaggagc aaggtgagat gacaggag 58
<210> 272
   <211> 58
   <212> DNA
   <213> Artificial Sequence
<220>
   <223> Synthetic Polynucleotide
<400> 272
   tctatagtgt cacctaaatc gtatgagtca atgcaggagc aaggtgagat gacaggag 58
<210> 273
   <211> 58
   <212> DNA
   <213> Artificial Sequence
<220>
   <223> Synthetic Polynucleotide
<400> 273
   tctatagtgt cacctaaatc gtatgtctgt agacaggagc aaggtgagat gacaggag 58
<210> 274
   <211> 58
   <212> DNA
   <213> Artificial Sequence
<220>
   <223> Synthetic Polynucleotide
<400> 274
   tctatagtgt cacctaaatc gtatggacaa cgacaggagc aaggtgagat gacaggag 58
<210> 275
   <211> 58
   <212> DNA
   <213> Artificial Sequence
<220>
   <223> Synthetic Polynucleotide
<400> 275
   tctatagtgt cacctaaatc gtatgccatg gctcaggagc aaggtgagat gacaggag 58
<210> 276
   <211> 58
   <212> DNA
   <213> Artificial Sequence
<220>
   <223> Synthetic Polynucleotide
<400> 276
   tctatagtgt cacctaaatc gtatgtgact ctgcaggagc aaggtgagat gacaggag 58
<210> 277
   <211> 58
   <212> DNA
   <213> Artificial Sequence
<220>
   <223> Synthetic Polynucleotide
<400> 277
   tctatagtgt cacctaaatc gtatgaacga ggccaggagc aaggtgagat gacaggag 58
<210> 278
   <211> 58
   <212> DNA
   <213> Artificial Sequence
<220>
   <223> Synthetic Polynucleotide
<400> 278
   tctatagtgt cacctaaatc gtatgcagaa ggtcaggagc aaggtgagat gacaggag 58
<210> 279
   <211> 58
   <212> DNA
   <213> Artificial Sequence
<220>
   <223> Synthetic Polynucleotide
<400> 279
   tctatagtgt cacctaaatc gtatgtgaag tcacaggagc aaggtgagat gacaggag 58
<210> 280
   <211> 58
   <212> DNA
   <213> Artificial Sequence
<220>
   <223> Synthetic Polynucleotide
<400> 280
   tctatagtgt cacctaaatc gtatgatgtt cctcaggagc aaggtgagat gacaggag 58
<210> 281
   <211> 58
   <212> DNA
   <213> Artificial Sequence
<220>
   <223> Synthetic Polynucleotide
<400> 281
   tctatagtgt cacctaaatc gtatgaagtg gctcaggagc aaggtgagat gacaggag 58
<210> 282
   <211> 58
   <212> DNA
   <213> Artificial Sequence
<220>
   <223> Synthetic Polynucleotide
<400> 282
   tctatagtgt cacctaaatc gtatgggtac aatcaggagc aaggtgagat gacaggag 58
<210> 283
   <211> 58
   <212> DNA
   <213> Artificial Sequence
<220>
   <223> Synthetic Polynucleotide
<400> 283
   tctatagtgt cacctaaatc gtatgacaag tgccaggagc aaggtgagat gacaggag 58
<210> 284
   <211> 58
   <212> DNA
   <213> Artificial Sequence
<220>
   <223> Synthetic Polynucleotide
<400> 284
   tctatagtgt cacctaaatc gtatgtcacg gtgcaggagc aaggtgagat gacaggag 58
<210> 285
   <211> 58
   <212> DNA
   <213> Artificial Sequence
<220>
   <223> Synthetic Polynucleotide
<400> 285
   tctatagtgt cacctaaatc gtatgttgcg ttacaggagc aaggtgagat gacaggag 58
<210> 286
   <211> 58
   <212> DNA
   <213> Artificial Sequence
<220>
   <223> Synthetic Polynucleotide
<400> 286
   tctatagtgt cacctaaatc gtatgttgta gcccaggagc aaggtgagat gacaggag 58
<210> 287
   <211> 58
   <212> DNA
   <213> Artificial Sequence
<220>
   <223> Synthetic Polynucleotide
<400> 287
   tctatagtgt cacctaaatc gtatgtcacc ggacaggagc aaggtgagat gacaggag 58
<210> 288
   <211> 58
   <212> DNA
   <213> Artificial Sequence
<220>
   <223> Synthetic Polynucleotide
<400> 288
   tctatagtgt cacctaaatc gtatgcgcgc aagcaggagc aaggtgagat gacaggag 58
<210> 289
   <211> 54
   <212> DNA
   <213> Artificial Sequence
<220>
   <223> Synthetic Polynucleotide
<400> 289
   tctctatggg cagtcggtga ttagatcgcc tcctgtcatc tcaccttgct ccac 54
<210> 290
   <211> 54
   <212> DNA
   <213> Artificial Sequence
<220>
   <223> Synthetic Polynucleotide
<400> 290
   tctctatggg cagtcggtga tctctctatc tcctgtcatc tcaccttgct ccac 54
<210> 291
   <211> 54
   <212> DNA
   <213> Artificial Sequence
<220>
   <223> Synthetic Polynucleotide
<400> 291
   tctctatggg cagtcggtga ttatcctctc tcctgtcatc tcaccttgct ccac 54
<210> 292
   <211> 54
   <212> DNA
   <213> Artificial Sequence
<220>
   <223> Synthetic Polynucleotide
<400> 292
   tctctatggg cagtcggtga tagagtagac tcctgtcatc tcaccttgct ccac 54
<210> 293
   <211> 54
   <212> DNA
   <213> Artificial Sequence
<220>
   <223> Synthetic Polynucleotide
<400> 293
   tctctatggg cagtcggtga tgtaaggagc tcctgtcatc tcaccttgct ccac 54
<210> 294
   <211> 54
   <212> DNA
   <213> Artificial Sequence
<220>
   <223> Synthetic Polynucleotide
<400> 294
   tctctatggg cagtcggtga tactgcatac tcctgtcatc tcaccttgct ccac 54
<210> 295
   <211> 54
   <212> DNA
   <213> Artificial Sequence
<220>
   <223> Synthetic Polynucleotide
<400> 295
   tctctatggg cagtcggtga taaggagtac tcctgtcatc tcaccttgct ccac 54
<210> 296
   <211> 54
   <212> DNA
   <213> Artificial Sequence
<220>
   <223> Synthetic Polynucleotide
<400> 296
   tctctatggg cagtcggtga tctaagcctc tcctgtcatc tcaccttgct ccac 54
<210> 297
   <211> 54
   <212> DNA
   <213> Artificial Sequence
<220>
   <223> Synthetic Polynucleotide
<400> 297
   tctctatggg cagtcggtga tgacattgtc tcctgtcatc tcaccttgct ccac 54
<210> 298
   <211> 54
   <212> DNA
   <213> Artificial Sequence
<220>
   <223> Synthetic Polynucleotide
<400> 298
   tctctatggg cagtcggtga tactgatggc tcctgtcatc tcaccttgct ccac 54
<210> 299
   <211> 54
   <212> DNA
   <213> Artificial Sequence
<220>
   <223> Synthetic Polynucleotide
<400> 299
   tctctatggg cagtcggtga tgtacctagc tcctgtcatc tcaccttgct ccac 54
<210> 300
   <211> 54
   <212> DNA
   <213> Artificial Sequence
<220>
   <223> Synthetic Polynucleotide
<400> 300
   tctctatggg cagtcggtga tcagagctac tcctgtcatc tcaccttgct ccac 54
<210> 301
   <211> 54
   <212> DNA
   <213> Artificial Sequence
<220>
   <223> Synthetic Polynucleotide
<400> 301
   tctctatggg cagtcggtga tcatagtgac tcctgtcatc tcaccttgct ccac 54
<210> 302
   <211> 54
   <212> DNA
   <213> Artificial Sequence
<220>
   <223> Synthetic Polynucleotide
<400> 302
   tctctatggg cagtcggtga ttacctagtc tcctgtcatc tcaccttgct ccac 54
<210> 303
   <211> 54
   <212> DNA
   <213> Artificial Sequence
<220>
   <223> Synthetic Polynucleotide
<400> 303
   tctctatggg cagtcggtga tcgcgatatc tcctgtcatc tcaccttgct ccac 54
<210> 304
   <211> 54
   <212> DNA
   <213> Artificial Sequence
<220>
   <223> Synthetic Polynucleotide
<400> 304
   tctctatggg cagtcggtga ttggattgtc tcctgtcatc tcaccttgct ccac 54
<210> 305
   <211> 54
   <212> DNA
   <213> Artificial Sequence
<220>
   <223> Synthetic Polynucleotide
<400> 305
   tctctatggg cagtcggtga tggacttccc tcctgtcatc tcaccttgct ccac 54
<210> 306
   <211> 54
   <212> DNA
   <213> Artificial Sequence
<220>
   <223> Synthetic Polynucleotide
<400> 306
   tctctatggg cagtcggtga tggtatggcc tcctgtcatc tcaccttgct ccac 54
<210> 307
   <211> 54
   <212> DNA
   <213> Artificial Sequence
<220>
   <223> Synthetic Polynucleotide
<400> 307
   tctctatggg cagtcggtga tcaatacgac tcctgtcatc tcaccttgct ccac 54
<210> 308
   <211> 54
   <212> DNA
   <213> Artificial Sequence
<220>
   <223> Synthetic Polynucleotide
<400> 308
   tctctatggg cagtcggtga tacaggtagc tcctgtcatc tcaccttgct ccac 54
<210> 309
   <211> 54
   <212> DNA
   <213> Artificial Sequence
<220>
   <223> Synthetic Polynucleotide
<400> 309
   tctctatggg cagtcggtga ttggagaggc tcctgtcatc tcaccttgct ccac 54
<210> 310
   <211> 54
   <212> DNA
   <213> Artificial Sequence
<220>
   <223> Synthetic Polynucleotide
<400> 310
   tctctatggg cagtcggtga tttacgtggc tcctgtcatc tcaccttgct ccac 54
<210> 311
   <211> 54
   <212> DNA
   <213> Artificial Sequence
<220>
   <223> Synthetic Polynucleotide
<400> 311
   tctctatggg cagtcggtga tatagccgac tcctgtcatc tcaccttgct ccac 54
<210> 312
   <211> 54
   <212> DNA
   <213> Artificial Sequence
<220>
   <223> Synthetic Polynucleotide
<400> 312
   tctctatggg cagtcggtga tgagtatctc tcctgtcatc tcaccttgct ccac 54
<210> 313
   <211> 54
   <212> DNA
   <213> Artificial Sequence
<220>
   <223> Synthetic Polynucleotide
<400> 313
   tctctatggg cagtcggtga tgtcgtgtac tcctgtcatc tcaccttgct ccac 54
<210> 314
   <211> 54
   <212> DNA
   <213> Artificial Sequence
<220>
   <223> Synthetic Polynucleotide
<400> 314
   tctctatggg cagtcggtga ttatccaagc tcctgtcatc tcaccttgct ccac 54
<210> 315
   <211> 54
   <212> DNA
   <213> Artificial Sequence
<220>
   <223> Synthetic Polynucleotide
<400> 315
   tctctatggg cagtcggtga tagtcgctac tcctgtcatc tcaccttgct ccac 54
<210> 316
   <211> 54
   <212> DNA
   <213> Artificial Sequence
<220>
   <223> Synthetic Polynucleotide
<400> 316
   tctctatggg cagtcggtga tgacaggttc tcctgtcatc tcaccttgct ccac 54
<210> 317
   <211> 54
   <212> DNA
   <213> Artificial Sequence
<220>
   <223> Synthetic Polynucleotide
<400> 317
   tctctatggg cagtcggtga tagattgacc tcctgtcatc tcaccttgct ccac 54
<210> 318
   <211> 54
   <212> DNA
   <213> Artificial Sequence
<220>
   <223> Synthetic Polynucleotide
<400> 318
   tctctatggg cagtcggtga ttatcaccgc tcctgtcatc tcaccttgct ccac 54
<210> 319
   <211> 54
   <212> DNA
   <213> Artificial Sequence
<220>
   <223> Synthetic Polynucleotide
<400> 319
   tctctatggg cagtcggtga tataggctgc tcctgtcatc tcaccttgct ccac 54
<210> 320
   <211> 54
   <212> DNA
   <213> Artificial Sequence
<220>
   <223> Synthetic Polynucleotide
<400> 320
   tctctatggg cagtcggtga tagtggcacc tcctgtcatc tcaccttgct ccac 54
<210> 321
   <211> 54
   <212> DNA
   <213> Artificial Sequence
<220>
   <223> Synthetic Polynucleotide
<400> 321
   tctctatggg cagtcggtga tactcatctc tcctgtcatc tcaccttgct ccac 54
<210> 322
   <211> 54
   <212> DNA
   <213> Artificial Sequence
<220>
   <223> Synthetic Polynucleotide
<400> 322
   tctctatggg cagtcggtga tgcagccatc tcctgtcatc tcaccttgct ccac 54
<210> 323
   <211> 54
   <212> DNA
   <213> Artificial Sequence
<220>
   <223> Synthetic Polynucleotide
<400> 323
   tctctatggg cagtcggtga tttgcagtgc tcctgtcatc tcaccttgct ccac 54
<210> 324
   <211> 54
   <212> DNA
   <213> Artificial Sequence
<220>
   <223> Synthetic Polynucleotide
<400> 324
   tctctatggg cagtcggtga tcgactgcac tcctgtcatc tcaccttgct ccac 54
<210> 325
   <211> 54
   <212> DNA
   <213> Artificial Sequence
<220>
   <223> Synthetic Polynucleotide
<400> 325
   tctctatggg cagtcggtga tcggtcaatc tcctgtcatc tcaccttgct ccac 54
<210> 326
   <211> 54
   <212> DNA
   <213> Artificial Sequence
<220>
   <223> Synthetic Polynucleotide
<400> 326
   tctctatggg cagtcggtga tgctgctacc tcctgtcatc tcaccttgct ccac 54
<210> 327
   <211> 54
   <212> DNA
   <213> Artificial Sequence
<220>
   <223> Synthetic Polynucleotide
<400> 327
   tctctatggg cagtcggtga tgcagtctac tcctgtcatc tcaccttgct ccac 54
<210> 328
   <211> 54
   <212> DNA
   <213> Artificial Sequence
<220>
   <223> Synthetic Polynucleotide
<400> 328
   tctctatggg cagtcggtga ttggaccacc tcctgtcatc tcaccttgct ccac 54
<210> 329
   <211> 54
   <212> DNA
   <213> Artificial Sequence
<220>
   <223> Synthetic Polynucleotide
<400> 329
   tctctatggg cagtcggtga tgtcacatcc tcctgtcatc tcaccttgct ccac 54
<210> 330
   <211> 54
   <212> DNA
   <213> Artificial Sequence
<220>
   <223> Synthetic Polynucleotide
<400> 330
   tctctatggg cagtcggtga tgttgctgac tcctgtcatc tcaccttgct ccac 54
<210> 331
   <211> 54
   <212> DNA
   <213> Artificial Sequence
<220>
   <223> Synthetic Polynucleotide
<400> 331
   tctctatggg cagtcggtga tgagttagcc tcctgtcatc tcaccttgct ccac 54
<210> 332
   <211> 54
   <212> DNA
   <213> Artificial Sequence
<220>
   <223> Synthetic Polynucleotide
<400> 332
   tctctatggg cagtcggtga tacgatcatc tcctgtcatc tcaccttgct ccac 54
<210> 333
   <211> 54
   <212> DNA
   <213> Artificial Sequence
<220>
   <223> Synthetic Polynucleotide
<400> 333
   tctctatggg cagtcggtga tcgtcgtctc tcctgtcatc tcaccttgct ccac 54
<210> 334
   <211> 54
   <212> DNA
   <213> Artificial Sequence
<220>
   <223> Synthetic Polynucleotide
<400> 334
   tctctatggg cagtcggtga tgacatgcgc tcctgtcatc tcaccttgct ccac 54
<210> 335
   <211> 54
   <212> DNA
   <213> Artificial Sequence
<220>
   <223> Synthetic Polynucleotide
<400> 335
   tctctatggg cagtcggtga tgtgccatac tcctgtcatc tcaccttgct ccac 54
<210> 336
   <211> 54
   <212> DNA
   <213> Artificial Sequence
<220>
   <223> Synthetic Polynucleotide
<400> 336
   tctctatggg cagtcggtga tcgctaggac tcctgtcatc tcaccttgct ccac 54
<210> 337
   <211> 54
   <212> DNA
   <213> Artificial Sequence
<220>
   <223> Synthetic Polynucleotide
<400> 337
   tctctatggg cagtcggtga tcgtaggtac tcctgtcatc tcaccttgct ccac 54
<210> 338
   <211> 54
   <212> DNA
   <213> Artificial Sequence
<220>
   <223> Synthetic Polynucleotide
<400> 338
   tctctatggg cagtcggtga tagctagcgc tcctgtcatc tcaccttgct ccac 54
<210> 339
   <211> 54
   <212> DNA
   <213> Artificial Sequence
<220>
   <223> Synthetic Polynucleotide
<400> 339
   tctctatggg cagtcggtga ttcctgtgcc tcctgtcatc tcaccttgct ccac 54
<210> 340
   <211> 54
   <212> DNA
   <213> Artificial Sequence
<220>
   <223> Synthetic Polynucleotide
<400> 340
   tctctatggg cagtcggtga tgtaatctgc tcctgtcatc tcaccttgct ccac 54
<210> 341
   <211> 54
   <212> DNA
   <213> Artificial Sequence
<220>
   <223> Synthetic Polynucleotide
<400> 341
   tctctatggg cagtcggtga taacgtaggc tcctgtcatc tcaccttgct ccac 54
<210> 342
   <211> 54
   <212> DNA
   <213> Artificial Sequence
<220>
   <223> Synthetic Polynucleotide
<400> 342
   tctctatggg cagtcggtga tttcctgttc tcctgtcatc tcaccttgct ccac 54
<210> 343
   <211> 54
   <212> DNA
   <213> Artificial Sequence
<220>
   <223> Synthetic Polynucleotide
<400> 343
   tctctatggg cagtcggtga ttgtccagtc tcctgtcatc tcaccttgct ccac 54
<210> 344
   <211> 54
   <212> DNA
   <213> Artificial Sequence
<220>
   <223> Synthetic Polynucleotide
<400> 344
   tctctatggg cagtcggtga tacaaggcac tcctgtcatc tcaccttgct ccac 54
<210> 345
   <211> 54
   <212> DNA
   <213> Artificial Sequence
<220>
   <223> Synthetic Polynucleotide
<400> 345
   tctctatggg cagtcggtga tccttgaccc tcctgtcatc tcaccttgct ccac 54
<210> 346
   <211> 54
   <212> DNA
   <213> Artificial Sequence
<220>
   <223> Synthetic Polynucleotide
<400> 346
   tctctatggg cagtcggtga tcgcttgtgc tcctgtcatc tcaccttgct ccac 54
<210> 347
   <211> 54
   <212> DNA
   <213> Artificial Sequence
<220>
   <223> Synthetic Polynucleotide
<400> 347
   tctctatggg cagtcggtga ttccaagcgc tcctgtcatc tcaccttgct ccac 54
<210> 348
   <211> 54
   <212> DNA
   <213> Artificial Sequence
<220>
   <223> Synthetic Polynucleotide
<400> 348
   tctctatggg cagtcggtga tctagtgacc tcctgtcatc tcaccttgct ccac 54
<210> 349
   <211> 54
   <212> DNA
   <213> Artificial Sequence
<220>
   <223> Synthetic Polynucleotide
<400> 349
   tctctatggg cagtcggtga tagaaccgtc tcctgtcatc tcaccttgct ccac 54
<210> 350
   <211> 54
   <212> DNA
   <213> Artificial Sequence
<220>
   <223> Synthetic Polynucleotide
<400> 350
   tctctatggg cagtcggtga ttaattgcac tcctgtcatc tcaccttgct ccac 54
<210> 351
   <211> 54
   <212> DNA
   <213> Artificial Sequence
<220>
   <223> Synthetic Polynucleotide
<400> 351
   tctctatggg cagtcggtga tctagtacac tcctgtcatc tcaccttgct ccac 54
<210> 352
   <211> 54
   <212> DNA
   <213> Artificial Sequence
<220>
   <223> Synthetic Polynucleotide
<400> 352
   tctctatggg cagtcggtga tgctatatcc tcctgtcatc tcaccttgct ccac 54
<210> 353
   <211> 54
   <212> DNA
   <213> Artificial Sequence
<220>
   <223> Synthetic Polynucleotide
<400> 353
   tctctatggg cagtcggtga tcaatcggcc tcctgtcatc tcaccttgct ccac 54
<210> 354
   <211> 54
   <212> DNA
   <213> Artificial Sequence
<220>
   <223> Synthetic Polynucleotide
<400> 354
   tctctatggg cagtcggtga tcgatatcac tcctgtcatc tcaccttgct ccac 54
<210> 355
   <211> 54
   <212> DNA
   <213> Artificial Sequence
<220>
   <223> Synthetic Polynucleotide
<400> 355
   tctctatggg cagtcggtga tcagtcaggc tcctgtcatc tcaccttgct ccac 54
<210> 356
   <211> 54
   <212> DNA
   <213> Artificial Sequence
<220>
   <223> Synthetic Polynucleotide
<400> 356
   tctctatggg cagtcggtga tgtaataatc tcctgtcatc tcaccttgct ccac 54
<210> 357
   <211> 54
   <212> DNA
   <213> Artificial Sequence
<220>
   <223> Synthetic Polynucleotide
<400> 357
   tctctatggg cagtcggtga tggagagatc tcctgtcatc tcaccttgct ccac 54
<210> 358
   <211> 54
   <212> DNA
   <213> Artificial Sequence
<220>
   <223> Synthetic Polynucleotide
<400> 358
   tctctatggg cagtcggtga tctctcatac tcctgtcatc tcaccttgct ccac 54
<210> 359
   <211> 54
   <212> DNA
   <213> Artificial Sequence
<220>
   <223> Synthetic Polynucleotide
<400> 359
   tctctatggg cagtcggtga tcagcgactc tcctgtcatc tcaccttgct ccac 54
<210> 360
   <211> 54
   <212> DNA
   <213> Artificial Sequence
<220>
   <223> Synthetic Polynucleotide
<400> 360
   tctctatggg cagtcggtga tggccaaggc tcctgtcatc tcaccttgct ccac 54
<210> 361
   <211> 54
   <212> DNA
   <213> Artificial Sequence
<220>
   <223> Synthetic Polynucleotide
<400> 361
   tctctatggg cagtcggtga tgcatatgcc tcctgtcatc tcaccttgct ccac 54
<210> 362
   <211> 54
   <212> DNA
   <213> Artificial Sequence
<220>
   <223> Synthetic Polynucleotide
<400> 362
   tctctatggg cagtcggtga tactaggatc tcctgtcatc tcaccttgct ccac 54
<210> 363
   <211> 54
   <212> DNA
   <213> Artificial Sequence
<220>
   <223> Synthetic Polynucleotide
<400> 363
   tctctatggg cagtcggtga tccttacctc tcctgtcatc tcaccttgct ccac 54
<210> 364
   <211> 54
   <212> DNA
   <213> Artificial Sequence
<220>
   <223> Synthetic Polynucleotide
<400> 364
   tctctatggg cagtcggtga ttgttgacgc tcctgtcatc tcaccttgct ccac 54
<210> 365
   <211> 54
   <212> DNA
   <213> Artificial Sequence
<220>
   <223> Synthetic Polynucleotide
<400> 365
   tctctatggg cagtcggtga ttacagttac tcctgtcatc tcaccttgct ccac 54
<210> 366
   <211> 54
   <212> DNA
   <213> Artificial Sequence
<220>
   <223> Synthetic Polynucleotide
<400> 366
   tctctatggg cagtcggtga tttgttacgc tcctgtcatc tcaccttgct ccac 54
<210> 367
   <211> 54
   <212> DNA
   <213> Artificial Sequence
<220>
   <223> Synthetic Polynucleotide
<400> 367
   tctctatggg cagtcggtga ttcgtgttgc tcctgtcatc tcaccttgct ccac 54
<210> 368
   <211> 54
   <212> DNA
   <213> Artificial Sequence
<220>
   <223> Synthetic Polynucleotide
<400> 368
   tctctatggg cagtcggtga tagtcaatgc tcctgtcatc tcaccttgct ccac 54
<210> 369
   <211> 54
   <212> DNA
   <213> Artificial Sequence
<220>
   <223> Synthetic Polynucleotide
<400> 369
   tctctatggg cagtcggtga ttctgtagac tcctgtcatc tcaccttgct ccac 54
<210> 370
   <211> 54
   <212> DNA
   <213> Artificial Sequence
<220>
   <223> Synthetic Polynucleotide
<400> 370
   tctctatggg cagtcggtga tgacaacgac tcctgtcatc tcaccttgct ccac 54
<210> 371
   <211> 54
   <212> DNA
   <213> Artificial Sequence
<220>
   <223> Synthetic Polynucleotide
<400> 371
   tctctatggg cagtcggtga tccatggctc tcctgtcatc tcaccttgct ccac 54
<210> 372
   <211> 54
   <212> DNA
   <213> Artificial Sequence
<220>
   <223> Synthetic Polynucleotide
<400> 372
   tctctatggg cagtcggtga ttgactctgc tcctgtcatc tcaccttgct ccac 54
<210> 373
   <211> 54
   <212> DNA
   <213> Artificial Sequence
<220>
   <223> Synthetic Polynucleotide
<400> 373
   tctctatggg cagtcggtga taacgaggcc tcctgtcatc tcaccttgct ccac 54
<210> 374
   <211> 54
   <212> DNA
   <213> Artificial Sequence
<220>
   <223> Synthetic Polynucleotide
<400> 374
   tctctatggg cagtcggtga tcagaaggtc tcctgtcatc tcaccttgct ccac 54
<210> 375
   <211> 54
   <212> DNA
   <213> Artificial Sequence
<220>
   <223> Synthetic Polynucleotide
<400> 375
   tctctatggg cagtcggtga ttgaagtcac tcctgtcatc tcaccttgct ccac 54
<210> 376
   <211> 54
   <212> DNA
   <213> Artificial Sequence
<220>
   <223> Synthetic Polynucleotide
<400> 376
   tctctatggg cagtcggtga tatgttcctc tcctgtcatc tcaccttgct ccac 54
<210> 377
   <211> 54
   <212> DNA
   <213> Artificial Sequence
<220>
   <223> Synthetic Polynucleotide
<400> 377
   tctctatggg cagtcggtga taagtggctc tcctgtcatc tcaccttgct ccac 54
<210> 378
   <211> 54
   <212> DNA
   <213> Artificial Sequence
<220>
   <223> Synthetic Polynucleotide
<400> 378
   tctctatggg cagtcggtga tggtacaatc tcctgtcatc tcaccttgct ccac 54
<210> 379
   <211> 54
   <212> DNA
   <213> Artificial Sequence
<220>
   <223> Synthetic Polynucleotide
<400> 379
   tctctatggg cagtcggtga tacaagtgcc tcctgtcatc tcaccttgct ccac 54
<210> 380
   <211> 54
   <212> DNA
   <213> Artificial Sequence
<220>
   <223> Synthetic Polynucleotide
<400> 380
   tctctatggg cagtcggtga ttcacggtgc tcctgtcatc tcaccttgct ccac 54
<210> 381
   <211> 54
   <212> DNA
   <213> Artificial Sequence
<220>
   <223> Synthetic Polynucleotide
<400> 381
   tctctatggg cagtcggtga tttgcgttac tcctgtcatc tcaccttgct ccac 54
<210> 382
   <211> 54
   <212> DNA
   <213> Artificial Sequence
<220>
   <223> Synthetic Polynucleotide
<400> 382
   tctctatggg cagtcggtga tttgtagccc tcctgtcatc tcaccttgct ccac 54
<210> 383
   <211> 54
   <212> DNA
   <213> Artificial Sequence
<220>
   <223> Synthetic Polynucleotide
<400> 383
   tctctatggg cagtcggtga ttcaccggac tcctgtcatc tcaccttgct ccac 54
<210> 384
   <211> 54
   <212> DNA
   <213> Artificial Sequence
<220>
   <223> Synthetic Polynucleotide
<400> 384
   tctctatggg cagtcggtga tcgcgcaagc tcctgtcatc tcaccttgct ccac 54
<210> 385
   <211> 65
   <212> DNA
   <213> Artificial Sequence
<220>
   <223> Synthetic Polynucleotide
<400> 385
   tcaccgactg cccatagaga ggactccagt cactagatcg catctcgtat gccgtcttct 60
   gcttg 65
<210> 386
   <211> 65
   <212> DNA
   <213> Artificial Sequence
<220>
   <223> Synthetic Polynucleotide
<400> 386
   tcaccgactg cccatagaga ggactccagt cacctctcta tatctcgtat gccgtcttct 60
   gcttg 65
<210> 387
   <211> 65
   <212> DNA
   <213> Artificial Sequence
<220>
   <223> Synthetic Polynucleotide
<400> 387
   tcaccgactg cccatagaga ggactccagt cactatcctc tatctcgtat gccgtcttct 60
   gcttg 65
<210> 388
   <211> 65
   <212> DNA
   <213> Artificial Sequence
<220>
   <223> Synthetic Polynucleotide
<400> 388
   tcaccgactg cccatagaga ggactccagt cacagagtag aatctcgtat gccgtcttct 60
   gcttg 65
<210> 389
   <211> 65
   <212> DNA
   <213> Artificial Sequence
<220>
   <223> Synthetic Polynucleotide
<400> 389
   tcaccgactg cccatagaga ggactccagt cacgtaagga gatctcgtat gccgtcttct 60
   gcttg 65
<210> 390
   <211> 65
   <212> DNA
   <213> Artificial Sequence
<220>
   <223> Synthetic Polynucleotide
<400> 390
   tcaccgactg cccatagaga ggactccagt cacactgcat aatctcgtat gccgtcttct 60
   gcttg 65
<210> 391
   <211> 65
   <212> DNA
   <213> Artificial Sequence
<220>
   <223> Synthetic Polynucleotide
<400> 391
   tcaccgactg cccatagaga ggactccagt cacaaggagt aatctcgtat gccgtcttct 60
   gcttg 65
<210> 392
   <211> 65
   <212> DNA
   <213> Artificial Sequence
<220>
   <223> Synthetic Polynucleotide
<400> 392
   tcaccgactg cccatagaga ggactccagt cacctaagcc tatctcgtat gccgtcttct 60
   gcttg 65
<210> 393
   <211> 65
   <212> DNA
   <213> Artificial Sequence
<220>
   <223> Synthetic Polynucleotide
<400> 393
   tcaccgactg cccatagaga ggactccagt cacgacattg tatctcgtat gccgtcttct 60
   gcttg 65
<210> 394
   <211> 65
   <212> DNA
   <213> Artificial Sequence
<220>
   <223> Synthetic Polynucleotide
<400> 394
   tcaccgactg cccatagaga ggactccagt cacactgatg gatctcgtat gccgtcttct 60
   gcttg 65
<210> 395
   <211> 65
   <212> DNA
   <213> Artificial Sequence
<220>
   <223> Synthetic Polynucleotide
<400> 395
   tcaccgactg cccatagaga ggactccagt cacgtaccta gatctcgtat gccgtcttct 60
   gcttg 65
<210> 396
   <211> 65
   <212> DNA
   <213> Artificial Sequence
<220>
   <223> Synthetic Polynucleotide
<400> 396
   tcaccgactg cccatagaga ggactccagt caccagagct aatctcgtat gccgtcttct 60
   gcttg 65
<210> 397
   <211> 65
   <212> DNA
   <213> Artificial Sequence
<220>
   <223> Synthetic Polynucleotide
<400> 397
   tcaccgactg cccatagaga ggactccagt caccatagtg aatctcgtat gccgtcttct 60
   gcttg 65
<210> 398
   <211> 65
   <212> DNA
   <213> Artificial Sequence
<220>
   <223> Synthetic Polynucleotide
<400> 398
   tcaccgactg cccatagaga ggactccagt cactacctag tatctcgtat gccgtcttct 60
   gcttg 65
<210> 399
   <211> 65
   <212> DNA
   <213> Artificial Sequence
<220>
   <223> Synthetic Polynucleotide
<400> 399
   tcaccgactg cccatagaga ggactccagt caccgcgata tatctcgtat gccgtcttct 60
   gcttg 65
<210> 400
   <211> 65
   <212> DNA
   <213> Artificial Sequence
<220>
   <223> Synthetic Polynucleotide
<400> 400
   tcaccgactg cccatagaga ggactccagt cactggattg tatctcgtat gccgtcttct 60
   gcttg 65
<210> 401
   <211> 65
   <212> DNA
   <213> Artificial Sequence
<220>
   <223> Synthetic Polynucleotide
<400> 401
   tcaccgactg cccatagaga ggactccagt cacggacttc catctcgtat gccgtcttct 60
   gcttg 65
<210> 402
   <211> 65
   <212> DNA
   <213> Artificial Sequence
<220>
   <223> Synthetic Polynucleotide
<400> 402
   tcaccgactg cccatagaga ggactccagt cacggtatgg catctcgtat gccgtcttct 60
   gcttg 65
<210> 403
   <211> 65
   <212> DNA
   <213> Artificial Sequence
<220>
   <223> Synthetic Polynucleotide
<400> 403
   tcaccgactg cccatagaga ggactccagt caccaatacg aatctcgtat gccgtcttct 60
   gcttg 65
<210> 404
   <211> 65
   <212> DNA
   <213> Artificial Sequence
<220>
   <223> Synthetic Polynucleotide
<400> 404
   tcaccgactg cccatagaga ggactccagt cacacaggta gatctcgtat gccgtcttct 60
   gcttg 65
<210> 405
   <211> 65
   <212> DNA
   <213> Artificial Sequence
<220>
   <223> Synthetic Polynucleotide
<400> 405
   tcaccgactg cccatagaga ggactccagt cactggagag gatctcgtat gccgtcttct 60
   gcttg 65
<210> 406
   <211> 65
   <212> DNA
   <213> Artificial Sequence
<220>
   <223> Synthetic Polynucleotide
<400> 406
   tcaccgactg cccatagaga ggactccagt cacttacgtg gatctcgtat gccgtcttct 60
   gcttg 65
<210> 407
   <211> 65
   <212> DNA
   <213> Artificial Sequence
<220>
   <223> Synthetic Polynucleotide
<400> 407
   tcaccgactg cccatagaga ggactccagt cacatagccg aatctcgtat gccgtcttct 60
   gcttg 65
<210> 408
   <211> 65
   <212> DNA
   <213> Artificial Sequence
<220>
   <223> Synthetic Polynucleotide
<400> 408
   tcaccgactg cccatagaga ggactccagt cacgagtatc tatctcgtat gccgtcttct 60
   gcttg 65
<210> 409
   <211> 65
   <212> DNA
   <213> Artificial Sequence
<220>
   <223> Synthetic Polynucleotide
<400> 409
   tcaccgactg cccatagaga ggactccagt cacgtcgtgt aatctcgtat gccgtcttct 60
   gcttg 65
<210> 410
   <211> 65
   <212> DNA
   <213> Artificial Sequence
<220>
   <223> Synthetic Polynucleotide
<400> 410
   tcaccgactg cccatagaga ggactccagt cactatccaa gatctcgtat gccgtcttct 60
   gcttg 65
<210> 411
   <211> 65
   <212> DNA
   <213> Artificial Sequence
<220>
   <223> Synthetic Polynucleotide
<400> 411
   tcaccgactg cccatagaga ggactccagt cacagtcgct aatctcgtat gccgtcttct      60
   gcttg                                                                  65
<210> 412
   <211> 65
   <212> DNA
   <213> Artificial Sequence
<220>
   <223> Synthetic Polynucleotide
<400> 412
   tcaccgactg cccatagaga ggactccagt cacgacaggt tatctcgtat gccgtcttct      60
   gcttg                                                                  65
<210> 413
   <211> 65
   <212> DNA
   <213> Artificial Sequence
<220>
   <223> Synthetic Polynucleotide
<400> 413
   tcaccgactg cccatagaga ggactccagt cacagattga catctcgtat gccgtcttct      60
   gcttg                                                                  65
<210> 414
   <211> 65
   <212> DNA
   <213> Artificial Sequence
<220>
   <223> Synthetic Polynucleotide
<400> 414
   tcaccgactg cccatagaga ggactccagt cactatcacc gatctcgtat gccgtcttct      60
   gcttg                                                                  65
<210> 415
   <211> 65
   <212> DNA
   <213> Artificial Sequence
<220>
   <223> Synthetic Polynucleotide
<400> 415
   tcaccgactg cccatagaga ggactccagt cacataggct gatctcgtat gccgtcttct 60
   gcttg 65
<210> 416
   <211> 65
   <212> DNA
   <213> Artificial Sequence
<220>
   <223> Synthetic Polynucleotide
<400> 416
   tcaccgactg cccatagaga ggactccagt cacagtggca catctcgtat gccgtcttct 60
   gcttg 65
<210> 417
   <211> 65
   <212> DNA
   <213> Artificial Sequence
<220>
   <223> Synthetic Polynucleotide
<400> 417
   tcaccgactg cccatagaga ggactccagt cacactcatc tatctcgtat gccgtcttct 60
   gcttg 65
<210> 418
   <211> 65
   <212> DNA
   <213> Artificial Sequence
<220>
   <223> Synthetic Polynucleotide
<400> 418
   tcaccgactg cccatagaga ggactccagt cacgcagcca tatctcgtat gccgtcttct 60
   gcttg 65
<210> 419
   <211> 65
   <212> DNA
   <213> Artificial Sequence
<220>
   <223> Synthetic Polynucleotide
<400> 419
   tcaccgactg cccatagaga ggactccagt cacttgcagt gatctcgtat gccgtcttct 60
   gcttg 65
<210> 420
   <211> 65
   <212> DNA
   <213> Artificial Sequence
<220>
   <223> Synthetic Polynucleotide
<400> 420
   tcaccgactg cccatagaga ggactccagt caccgactgc aatctcgtat gccgtcttct 60
   gcttg 65
<210> 421
   <211> 65
   <212> DNA
   <213> Artificial Sequence
<220>
   <223> Synthetic Polynucleotide
<400> 421
   tcaccgactg cccatagaga ggactccagt caccggtcaa tatctcgtat gccgtcttct 60
gcttg 65
<210> 422
   <211> 65
   <212> DNA
   <213> Artificial Sequence
<220>
   <223> Synthetic Polynucleotide
<400> 422
   tcaccgactg cccatagaga ggactccagt cacgctgcta catctcgtat gccgtcttct 60
   gcttg 65
<210> 423
   <211> 65
   <212> DNA
   <213> Artificial Sequence
<220>
   <223> Synthetic Polynucleotide
<400> 423
   tcaccgactg cccatagaga ggactccagt cacgcagtct aatctcgtat gccgtcttct 60
   gcttg 65
<210> 424
   <211> 65
   <212> DNA
   <213> Artificial Sequence
<220>
   <223> Synthetic Polynucleotide
<400> 424
   tcaccgactg cccatagaga ggactccagt cactggacca catctcgtat gccgtcttct 60
   gcttg 65
<210> 425
   <211> 65
   <212> DNA
   <213> Artificial Sequence
<220>
   <223> Synthetic Polynucleotide
<400> 425
   tcaccgactg cccatagaga ggactccagt cacgtcacat catctcgtat gccgtcttct 60
   gcttg 65
<210> 426
   <211> 65
   <212> DNA
   <213> Artificial Sequence
<220>
   <223> Synthetic Polynucleotide
<400> 426
   tcaccgactg cccatagaga ggactccagt cacgttgctg aatctcgtat gccgtcttct 60
   gcttg 65
<210> 427
   <211> 65
   <212> DNA
   <213> Artificial Sequence
<220>
   <223> Synthetic Polynucleotide
<400> 427
   tcaccgactg cccatagaga ggactccagt cacgagttag catctcgtat gccgtcttct 60
   gcttg 65
<210> 428
   <211> 65
   <212> DNA
   <213> Artificial Sequence
<220>
   <223> Synthetic Polynucleotide
<400> 428
   tcaccgactg cccatagaga ggactccagt cacacgatca tatctcgtat gccgtcttct 60
   gcttg 65
<210> 429
   <211> 65
   <212> DNA
   <213> Artificial Sequence
<220>
   <223> Synthetic Polynucleotide
<400> 429
   tcaccgactg cccatagaga ggactccagt caccgtcgtc tatctcgtat gccgtcttct 60
   gcttg 65
<210> 430
   <211> 65
   <212> DNA
   <213> Artificial Sequence
<220>
   <223> Synthetic Polynucleotide
<400> 430
   tcaccgactg cccatagaga ggactccagt cacgacatgc gatctcgtat gccgtcttct 60
   gcttg 65
<210> 431
   <211> 65
   <212> DNA
   <213> Artificial Sequence
<220>
   <223> Synthetic Polynucleotide
<400> 431
   tcaccgactg cccatagaga ggactccagt cacgtgccat aatctcgtat gccgtcttct 60
   gcttg 65
<210> 432
   <211> 65
   <212> DNA
   <213> Artificial Sequence
<220>
   <223> Synthetic Polynucleotide
<400> 432
   tcaccgactg cccatagaga ggactccagt caccgctagg aatctcgtat gccgtcttct 60
   gcttg 65
<210> 433
   <211> 65
   <212> DNA
   <213> Artificial Sequence
<220>
   <223> Synthetic Polynucleotide
<400> 433
   tcaccgactg cccatagaga ggactccagt caccgtaggt aatctcgtat gccgtcttct 60
   gcttg 65
<210> 434
   <211> 65
   <212> DNA
   <213> Artificial Sequence
<220>
   <223> Synthetic Polynucleotide
<400> 434
   tcaccgactg cccatagaga ggactccagt cacagctagc gatctcgtat gccgtcttct 60
   gcttg 65
<210> 435
   <211> 65
   <212> DNA
   <213> Artificial Sequence
<220>
   <223> Synthetic Polynucleotide
<400> 435
   tcaccgactg cccatagaga ggactccagt cactcctgtg catctcgtat gccgtcttct 60
   gcttg 65
<210> 436
   <211> 65
   <212> DNA
   <213> Artificial Sequence
<220>
   <223> Synthetic Polynucleotide
<400> 436
   tcaccgactg cccatagaga ggactccagt cacgtaatct gatctcgtat gccgtcttct 60
   gcttg 65
<210> 437
   <211> 65
   <212> DNA
   <213> Artificial Sequence
<220>
   <223> Synthetic Polynucleotide
<400> 437
   tcaccgactg cccatagaga ggactccagt cacaacgtag gatctcgtat gccgtcttct 60
   gcttg 65
<210> 438
   <211> 65
   <212> DNA
   <213> Artificial Sequence
<220>
   <223> Synthetic Polynucleotide
<400> 438
   tcaccgactg cccatagaga ggactccagt cacttcctgt tatctcgtat gccgtcttct 60
   gcttg 65
<210> 439
   <211> 65
   <212> DNA
   <213> Artificial Sequence
<220>
   <223> Synthetic Polynucleotide
<400> 439
   tcaccgactg cccatagaga ggactccagt cactgtccag tatctcgtat gccgtcttct 60
   gcttg 65
<210> 440
   <211> 65
   <212> DNA
   <213> Artificial Sequence
<220>
   <223> Synthetic Polynucleotide
<400> 440
   tcaccgactg cccatagaga ggactccagt cacacaaggc aatctcgtat gccgtcttct 60
   gcttg 65
<210> 441
   <211> 65
   <212> DNA
   <213> Artificial Sequence
<220>
   <223> Synthetic Polynucleotide
<400> 441
   tcaccgactg cccatagaga ggactccagt cacccttgac catctcgtat gccgtcttct 60
   gcttg 65
<210> 442
   <211> 65
   <212> DNA
   <213> Artificial Sequence
<220>
   <223> Synthetic Polynucleotide
<400> 442
   tcaccgactg cccatagaga ggactccagt caccgcttgt gatctcgtat gccgtcttct 60
   gcttg 65
<210> 443
   <211> 65
   <212> DNA
   <213> Artificial Sequence
<220>
   <223> Synthetic Polynucleotide
<400> 443
   tcaccgactg cccatagaga ggactccagt cactccaagc gatctcgtat gccgtcttct 60
   gcttg 65
<210> 444
   <211> 65
   <212> DNA
   <213> Artificial Sequence
<220>
   <223> Synthetic Polynucleotide
<400> 444
   tcaccgactg cccatagaga ggactccagt cacctagtga catctcgtat gccgtcttct 60
   gcttg 65
<210> 445
   <211> 65
   <212> DNA
   <213> Artificial Sequence
<220>
   <223> Synthetic Polynucleotide
<400> 445
   tcaccgactg cccatagaga ggactccagt cacagaaccg tatctcgtat gccgtcttct 60
   gcttg 65
<210> 446
   <211> 65
   <212> DNA
   <213> Artificial Sequence
<220>
   <223> Synthetic Polynucleotide
<400> 446
   tcaccgactg cccatagaga ggactccagt cactaattgc aatctcgtat gccgtcttct        60
   gcttg                                                                     65
<210> 447
   <211> 65
   <212> DNA
   <213> Artificial Sequence
<220>
   <223> Synthetic Polynucleotide
<400> 447
   tcaccgactg cccatagaga ggactccagt cacctagtac aatctcgtat gccgtcttct        60
   gcttg                                                                     65
<210> 448
   <211> 65
   <212> DNA
   <213> Artificial Sequence
<220>
   <223> Synthetic Polynucleotide
<400> 448
   tcaccgactg cccatagaga ggactccagt cacgctatat catctcgtat gccgtcttct        60
   gcttg                                                                     65
<210> 449
   <211> 65
   <212> DNA
   <213> Artificial Sequence
<220>
   <223> Synthetic Polynucleotide
<400> 449
   tcaccgactg cccatagaga ggactccagt caccaatcgg catctcgtat gccgtcttct        60
   gcttg                                                                     65
<210> 450
   <211> 65
   <212> DNA
   <213> Artificial Sequence
<220>
   <223> Synthetic Polynucleotide
<400> 450
   tcaccgactg cccatagaga ggactccagt caccgatatc aatctcgtat gccgtcttct        60
   gcttg                                                                    65
<210> 451
   <211> 65
   <212> DNA
   <213> Artificial Sequence
<220>
   <223> Synthetic Polynucleotide
<400> 451
   tcaccgactg cccatagaga ggactccagt caccagtcag gatctcgtat gccgtcttct        60
   gcttg                                                                    65
<210> 452
   <211> 65
   <212> DNA
   <213> Artificial Sequence
<220>
   <223> Synthetic Polynucleotide
<400> 452
   tcaccgactg cccatagaga ggactccagt cacgtaataa tatctcgtat gccgtcttct        60
   gcttg                                                                    65
<210> 453
   <211> 65
   <212> DNA
   <213> Artificial Sequence
<220>
   <223> Synthetic Polynucleotide
<400> 453
   tcaccgactg cccatagaga ggactccagt cacggagaga tatctcgtat gccgtcttct        60
   gcttg                                                                    65
<210> 454
   <211> 65
   <212> DNA
   <213> Artificial Sequence
<220>
   <223> Synthetic Polynucleotide
<400> 454
   tcaccgactg cccatagaga ggactccagt cacctctcat aatctcgtat gccgtcttct 60
   gcttg 65
<210> 455
   <211> 65
   <212> DNA
   <213> Artificial Sequence
<220>
   <223> Synthetic Polynucleotide
<400> 455
   tcaccgactg cccatagaga ggactccagt caccagcgac tatctcgtat gccgtcttct 60
   gcttg 65
<210> 456
   <211> 65
   <212> DNA
   <213> Artificial Sequence
<220>
   <223> Synthetic Polynucleotide
<400> 456
   tcaccgactg cccatagaga ggactccagt cacggccaag gatctcgtat gccgtcttct 60
   gcttg 65
<210> 457
   <211> 65
   <212> DNA
   <213> Artificial Sequence
<220>
   <223> Synthetic Polynucleotide
<400> 457
   tcaccgactg cccatagaga ggactccagt cacgcatatg catctcgtat gccgtcttct 60
   gcttg 65
<210> 458
   <211> 65
   <212> DNA
   <213> Artificial Sequence
<220>
   <223> Synthetic Polynucleotide
<400> 458
   tcaccgactg cccatagaga ggactccagt cacactagga tatctcgtat gccgtcttct 60
   gcttg 65
<210> 459
   <211> 65
   <212> DNA
   <213> Artificial Sequence
<220>
   <223> Synthetic Polynucleotide
<400> 459
   tcaccgactg cccatagaga ggactccagt cacccttacc tatctcgtat gccgtcttct        60
   gcttg 65
<210> 460
   <211> 65
   <212> DNA
   <213> Artificial Sequence
<220>
   <223> Synthetic Polynucleotide
<400> 460
   tcaccgactg cccatagaga ggactccagt cactgttgac gatctcgtat gccgtcttct 60
   gcttg 65
<210> 461
   <211> 65
   <212> DNA
   <213> Artificial Sequence
<220>
   <223> Synthetic Polynucleotide
<400> 461
   tcaccgactg cccatagaga ggactccagt cactacagtt aatctcgtat gccgtcttct 60
   gcttg 65
<210> 462
   <211> 65
   <212> DNA
   <213> Artificial Sequence
<220>
   <223> Synthetic Polynucleotide
<400> 462
   tcaccgactg cccatagaga ggactccagt cacttgttac gatctcgtat gccgtcttct 60
   gcttg 65
<210> 463
   <211> 65
   <212> DNA
   <213> Artificial Sequence
<220>
   <223> Synthetic Polynucleotide
<400> 463
   tcaccgactg cccatagaga ggactccagt cactcgtgtt gatctcgtat gccgtcttct 60
   gcttg 65
<210> 464
   <211> 65
   <212> DNA
   <213> Artificial Sequence
<220>
   <223> Synthetic Polynucleotide
<400> 464
   tcaccgactg cccatagaga ggactccagt cacagtcaat gatctcgtat gccgtcttct 60
   gcttg 65
<210> 465
   <211> 65
   <212> DNA
   <213> Artificial Sequence
<220>
   <223> Synthetic Polynucleotide
<400> 465
   tcaccgactg cccatagaga ggactccagt cactctgtag aatctcgtat gccgtcttct 60
   gcttg 65
<210> 466
   <211> 65
   <212> DNA
   <213> Artificial Sequence
<220>
   <223> Synthetic Polynucleotide
<400> 466
   tcaccgactg cccatagaga ggactccagt cacgacaacg aatctcgtat gccgtcttct 60
   gcttg 65
<210> 467
   <211> 65
   <212> DNA
   <213> Artificial Sequence
<220>
   <223> Synthetic Polynucleotide
<400> 467
   tcaccgactg cccatagaga ggactccagt cacccatggc tatctcgtat gccgtcttct 60
   gcttg 65
<210> 468
   <211> 65
   <212> DNA
   <213> Artificial Sequence
<220>
   <223> Synthetic Polynucleotide
<400> 468
   tcaccgactg cccatagaga ggactccagt cactgactct gatctcgtat gccgtcttct 60
   gcttg 65
<210> 469
   <211> 65
   <212> DNA
   <213> Artificial Sequence
<220>
   <223> Synthetic Polynucleotide
<400> 469
   tcaccgactg cccatagaga ggactccagt cacaacgagg catctcgtat gccgtcttct 60
   gcttg 65
<210> 470
   <211> 65
   <212> DNA
   <213> Artificial Sequence
<220>
   <223> Synthetic Polynucleotide
<400> 470
   tcaccgactg cccatagaga ggactccagt caccagaagg tatctcgtat gccgtcttct 60
   gcttg 65
<210> 471
   <211> 65
   <212> DNA
   <213> Artificial Sequence
<220>
   <223> Synthetic Polynucleotide
<400> 471
   tcaccgactg cccatagaga ggactccagt cactgaagtc aatctcgtat gccgtcttct 60
   gcttg 65
<210> 472
   <211> 65
   <212> DNA
   <213> Artificial Sequence
<220>
   <223> Synthetic Polynucleotide
<400> 472
   tcaccgactg cccatagaga ggactccagt cacatgttcc tatctcgtat gccgtcttct 60
   gcttg 65
<210> 473
   <211> 65
   <212> DNA
   <213> Artificial Sequence
<220>
   <223> Synthetic Polynucleotide
<400> 473
   tcaccgactg cccatagaga ggactccagt cacaagtggc tatctcgtat gccgtcttct 60
   gcttg 65
<210> 474
   <211> 65
   <212> DNA
   <213> Artificial Sequence
<220>
   <223> Synthetic Polynucleotide
<400> 474
   tcaccgactg cccatagaga ggactccagt cacggtacaa tatctcgtat gccgtcttct 60
   gcttg 65
<210> 475
   <211> 65
   <212> DNA
   <213> Artificial Sequence
<220>
   <223> Synthetic Polynucleotide
<400> 475
   tcaccgactg cccatagaga ggactccagt cacacaagtg catctcgtat gccgtcttct 60
   gcttg 65
<210> 476
   <211> 65
   <212> DNA
   <213> Artificial Sequence
<220>
   <223> Synthetic Polynucleotide
<400> 476
   tcaccgactg cccatagaga ggactccagt cactcacggt gatctcgtat gccgtcttct 60
   gcttg 65
<210> 477
   <211> 65
   <212> DNA
   <213> Artificial Sequence
<220>
   <223> Synthetic Polynucleotide
<400> 477
   tcaccgactg cccatagaga ggactccagt cacttgcgtt aatctcgtat gccgtcttct 60
   gcttg 65
<210> 478
   <211> 65
   <212> DNA
   <213> Artificial Sequence
<220>
   <223> Synthetic Polynucleotide
<400> 478
   tcaccgactg cccatagaga ggactccagt cacttgtagc catctcgtat gccgtcttct 60
   gcttg 65
<210> 479
   <211> 65
   <212> **DNA**
   <213> Artificial Sequence
<220>
   <223> Synthetic Polynucleotide
<400> 479
   tcaccgactg cccatagaga ggactccagt cactcaccgg aatctcgtat gccgtcttct 60
   gcttg 65
<210> 480
   <211> 65
   <212> DNA
   <213> Artificial Sequence
<220>
   <223> Synthetic Polynucleotide
<400> 480
   tcaccgactg cccatagaga ggactccagt caccgcgcaa gatctcgtat gccgtcttct 60
   gcttg 65

## Claims

1. A method for determining the history of a droplet, comprising:
exposing a microfluidic droplet to a first condition;
adding a first nucleic acid to the droplet, wherein the first nucleic acid encodes the step of exposing a microfluidic droplet to the first condition;
exposing the microfluidic droplet to a second condition;
adding a second nucleic acid to the droplet, wherein the second nucleic acid encodes the step of exposing the microfluidic droplet to the second condition; and
amplifying the first nucleic acid and the second nucleic acid within the microfluidic droplet to produce an amplified nucleic acid; and
sequencing the amplified nucleic acid to determine the history of the droplet.

2. The method of claim 1, wherein exposing the microfluidic droplet to the first condition and adding the first nucleic acid to the droplet comprises:
(a) fusing the microfluidic droplet to a second droplet containing the first nucleic acid; and/or
(b) exposing the droplet to a molecular species, preferably wherein exposing the droplet to the molecular species comprises fusing the droplet with a second droplet containing the molecular species, preferably wherein the second droplet:
(i) further contains the first nucleic acid, whereby when the second droplet is fused to the droplet, the first nucleic acid is added to the droplet; and/or
(ii) is fused to the droplet via dipoles induced in the droplets; and/or
(iii) is fused to the droplet via opposite electric charges placed on the droplets.

3. The method of claim 2, wherein exposing the microfluidic droplet to the molecular species comprises injecting the microfluidic droplet with a fluid containing the molecular species, preferably wherein the fluid containing the molecular species further contains the first nucleic acid.

4. The method of any one of claims 2 or 3, wherein:
(a) the molecular species and the first nucleic acid are added to the microfluidic droplet simultaneously; and/or
(b) the molecular species is added to the droplet before the first nucleic acid is added to the droplet; and/or
(c) the molecular species is added to the droplet after the first nucleic acid is added to the droplet.

5. The method of any one of claims 1-4, wherein the first nucleic acid and/or the second nucleic acid comprises a primer sequence; and/or wherein the method further comprises immobilizing the first nucleic acid and the second nucleic acid with respect to each other, preferably comprising ligating the first nucleic acid to the second nucleic acid.

6. The method of any one of claims 1-5, wherein amplifying the first nucleic acid and the second nucleic acid within the microfluidic droplet comprises:
(a) using PCR to amplify the first nucleic acid and the second nucleic acid; and/or
(b) adding a polymerase to the microfluidic droplet; and/or
(c) adding Taq polymerase to the microfluidic droplet; and/or
(d) exposing the microfluidic droplet to a temperature of at least about 50°C, preferably at least about 90°C; and/or
(e) adding deoxyribonucleotides to the microfluidic droplet.

7. The method of any one of claims 1-6, wherein exposing the microfluidic droplet to the first condition comprises:
(a) exposing the droplet to an external physical condition, preferably wherein exposing the droplet to an external physical condition comprises exposing the droplet to a predetermined temperature and/or a predetermined pressure; and/or
(b) fusing the droplet to a second droplet having a pH of less than 7 and/or a pH of greater than 7.

8. The method of any one of claims 1-7, wherein the microfluidic droplet:
(a) has an average cross-sectional diameter of less than about 1 mm; and/or
(b) is one of a plurality of microfluidic droplets having a distribution of diameters such that no more than about 5% of the microfluidic droplets have a diameter less than about 90% or greater than about 110% of the overall average diameter of the plurality of microfluidic droplets; and/or
(c) is contained within a microfluidic channel.

9. The method of any one of claims 1-8, further comprising exposing the microfluidic droplet to a third condition and adding a third nucleic acid to the microfluidic droplet, wherein the third nucleic acid encodes the third condition, preferably further comprising attaching the third nucleic acid to one or both of the first nucleic acid and the second nucleic acid.

10. The method of any one of claims 1-9, further comprising determining a property of the microfluidic droplet, preferably comprising sorting the droplet based on the property, and/or wherein the property is:
(a) fluorescence; and/or
(b) the average cross-sectional diameter of the droplet; and/or
(c) light absorption; and/or
(d) the concentration of an agent contained within the droplet; and/or
(e) the condition of a cell contained within the droplet, preferably wherein the property is whether the cell is alive or dead and/or the property is a concentration of an agent produced and/or consumed by the cell.

11. The method of claim 10, wherein the cell is contained within a gel, preferably wherein the gel is agarose gel and/or wherein the gel has a solidification temperature of less than about 25 °C.

12. The method of any one of claims 1-11, further comprising separating the first nucleic acid and the second nucleic acid from the microfluidic droplet, and/or bursting the microfluidic droplet, preferably wherein the droplet is burst by exposing the droplet to ultrasound.

13. The method of any one of claims 1-12, wherein the droplet contains a cell,
(a) wherein the cell is a human cell; and/or
(b) wherein the cell is a cancer cell; and/or
(c) wherein the cell is an immune cell; and/or
(d) wherein the cell is a bacterial cell; and/or
(e) wherein the cell is a naturally-occurring cell; and
wherein the first condition is:
(a) the cell's type; and/or
(b) exposure to a drug suspected of being capable of interacting with the cell; and/or
(c) exposure to a suspected drug, preferably wherein the drug is cytotoxic.

14. Use of an article, comprising:
a plurality of microfluidic droplets, at least some of the droplets containing one or more primers and one or more nucleic acids encoding a plurality of conditions that the at least some droplets were exposed to, preferably wherein the nucleic acids encode at least three conditions, in a method for determining the history of a droplet of claim 1.

15. The use according to claim 14, wherein at least some of the nucleic acids encode molecular species contained within at least some of the microfluidic droplets, preferably wherein at least some of the microfluidic droplets further comprise the respective molecular species encoded by the nucleic acids, preferably wherein at least some of the microfluidic droplets contain at least two molecular species and nucleic acids encoding the at least two molecular species; and/or
wherein at least some of the microfluidic droplets:
(a) contain at least three molecular species and nucleic acids encoding the at least three molecular species; and/or
(b) contain a polymerase; and/or
(c) contain Taq polymerase; and/or
(d) are at a temperature of at least about 50°C; and/or
(e) are at a temperature of at least about 90°C; and/or
(f) contain deoxyribonucleotides.

## Patentansprüche

1. Verfahren zum Bestimmen der Geschichte eines Tröpfchens, umfassend:
Exposition eines mikrofluidischen Tröpfchens an einen ersten Zustand;
Zugeben einer ersten Nukleinsäure zu dem Tröpfchen, wobei die erste Nukleinsäure den Schritt der Exposition des mikrofluidischen Tröpfchens an den ersten Zustand codiert;
Exposition des mikrofluidischen Tröpfchens an einen zweiten Zustand;
Zugeben einer zweiten Nukleinsäure zu dem Tröpfchen, wobei die zweite Nukleinsäure den Schritt der Exposition des mikrofluidischen Tröpfchens an den zweiten Zustand codiert; und
Amplifizieren der ersten Nukleinsäure und der zweiten Nukleinsäure innerhalb des mikrofluidischen Tröpfchens, um eine amplifizierte Nukleinsäure zu erzeugen; und
Sequenzieren der amplifizierten Nukleinsäure, um die Geschichte des Tröpfchens zu bestimmen.

2. Verfahren nach Anspruch 1, wobei die Exposition des mikrofluidischen Tröpfchens an den ersten Zustand und das Zugeben der ersten Nukleinsäure zu dem Tröpfchen umfasst:
(a) Fusionieren des mikrofluidischen Tröpfchens mit einem zweiten Tröpfchen, welches die erste Nukleinsäure enthält; und/oder
(b) Exposition des Tröpfchens an eine molekulare Spezies, wobei bevorzugt die Exposition des Tröpfchens an die molekulare Spezies das Fusionieren des Tröpfchens mit einem zweiten Tröpfchen umfasst, welches die molekulare Spezies enthält, wobei das zweite Tröpfchen bevorzugt:
(i) weiterhin die erste Nukleinsäure enthält, wodurch, wenn das zweite Tröpfchen mit dem Tröpfchen fusioniert ist, die erste Nukleinsäure zu dem Tröpfchen zugegeben wird; und/oder
(ii) über in den Tröpfchen induzierte Dipole mit dem Tröpfchen fusioniert wird; und/oder
(iii) über entgegengesetzte elektrische Ladungen, die auf die Tröpfchen aufgebracht werden, mit dem Tröpfchen fusioniert wird.

3. Verfahren nach Anspruch 2, wobei die Exposition des mikrofluidischen Tröpfchens an die molekulare Spezies das Injizieren des mikrofluidischen Tröpfchens mit einem die molekulare Spezies enthaltenden Fluid umfasst, wobei bevorzugt das die molekulare Spezies enthaltende Fluid weiterhin die erste Nukleinsäure enthält.

4. Verfahren nach irgendeinem der Ansprüche 2 oder 3, wobei:
(a) die molekulare Spezies und die erste Nukleinsäure gleichzeitig zu dem mikrofluidischen Tröpfchen zugegeben werden; und/oder
(b) die molekulare Spezies zu dem Tröpfchen zugegeben wird, bevor die erste Nukleinsäure zu dem Tröpfchen zugegeben wird; und/oder
(c) die molekulare Spezies zu dem Tröpfchen zugegeben wird, nachdem die erste Nukleinsäure zu dem Tröpfchen zugegeben wird.

5. Verfahren nach irgendeinem der Ansprüche 1-4, wobei die erste Nukleinsäure und/oder die zweite Nukleinsäure eine Primersequenz umfasst; und/oder wobei das Verfahren weiterhin das Immobilisieren der ersten Nukleinsäure und der zweiten Nukleinsäure in Bezug aufeinander umfasst, bevorzugt umfassend das Ligieren der ersten Nukleinsäure mit der zweiten Nukleinsäure.

6. Verfahren nach irgendeinem der Ansprüche 1-5, wobei das Amplifizieren der ersten Nukleinsäure und der zweiten Nukleinsäure innerhalb des mikrofluidischen Tröpfchens umfasst:
(a) die Verwendung von PCR, um die erste Nukleinsäure und die zweite Nukleinsäure zu amplifizieren; und/oder
(b) das Zugeben einer Polymerase zu dem mikrofluidischen Tröpfchen; und/oder
(c) das Zugeben von Taq-Polymerase zu dem mikrofluidischen Tröpfchen; und/oder
(d) die Exposition des mikrofluidischen Tröpfchens an eine Temperatur von wenigstens etwa 50°C, bevorzugt wenigstens 90°C; und/oder
(e) das Zugeben von Desoxyribonukleotiden zu dem mikrofluidischen Tröpfchen.

7. Verfahren nach irgendeinem der Ansprüche 1-6, wobei die Exposition des mikrofluidischen Tröpfchens an den ersten Zustand umfasst:
(a) Exposition des Tröpfchens an einen äußeren physikalischen Zustand, wobei bevorzugt die Exposition des Tröpfchens an einen äußeren physikalischen Zustand die Exposition des Tröpfchens an eine vorbestimmte Temperatur und/oder einen vorbestimmten Druck umfasst; und/oder
(b) Fusionieren des Tröpfchens mit einem zweiten Tröpfchen mit einem pH-Wert von kleiner als 7 und/oder einem pH-Wert von größer als 7.

8. Verfahren nach irgendeinem der Ansprüche 1-7, wobei das mikrofluidische Tröpfchen:
(a) einen durchschnittlichen Querschnittsdurchmesser von weniger als etwa 1 mm hat; und/oder
(b) eines aus einer Mehrzahl mikrofluidischer Tröpfchen mit einer derartigen Verteilung von Durchmessern ist, dass nicht mehr als etwa 5% der mikrofluidischen Tröpfchen einen Durchmesser von weniger als etwa 90% oder mehr als etwa 110% des gesamten durchschnittlichen Durchmessers der Mehrzahl mikrofluidischer Tröpfchen aufweisen; und/oder
(c) innerhalb eines mikrofluidischen Kanals enthalten ist.

9. Verfahren nach irgendeinem der Ansprüche 1-8, welches weiterhin die Exposition des mikrofluidischen Tröpfchens an einen dritten Zustand und das Zugeben einer dritten Nukleinsäure zu dem mikrofluidischen Tröpfchen umfasst, wobei die dritte Nukleinsäure den dritten Zustand codiert, bevorzugt weiterhin umfassend das Anheften der dritten Nukleinsäure an eine oder beide der ersten Nukleinsäure und der zweiten Nukleinsäure.

10. Verfahren nach irgendeinem der Ansprüche 1-9, weiterhin umfassend das Bestimmen einer Eigenschaft des mikrofluidischen Tröpfchens, bevorzugt umfassend das Sortieren des Tröpfchens basierend auf der Eigenschaft, und/oder wobei die Eigenschaft ist:
(a) Fluoreszenz; und/oder
(b) der durchschnittliche Querschnittsdurchmesser des Tröpfchens; und/oder
(c) Lichtabsorption; und/oder
(d) die Konzentration eines innerhalb des Tröpfchens enthaltenen Agens; und/oder
(e) der Zustand einer innerhalb des Tröpfchens enthaltenen Zelle, wobei bevorzugt die Eigenschaft darin besteht, ob die Zelle lebendig oder tot ist, und/oder die Eigenschaft eine Konzentration eines durch die Zelle produzierten und/oder konsumierten Agens ist.

11. Verfahren nach Anspruch 10, wobei die Zelle innerhalb eines Gels enthalten ist, wobei bevorzugt das Gel Agarosegel ist, und/oder wobei das Gel eine Verfestigungstemperatur von weniger als etwa 25°C hat.

12. Verfahren nach irgendeinem der Ansprüche 1-11, weiterhin umfassend das Abtrennen der ersten Nukleinsäure und der zweiten Nukleinsäure von dem mikrofluidischen Tröpfchen und/oder das Platzen des mikrofluidischen Tröpfchens, wobei bevorzugt das Tröpfchen durch Exposition des Tröpfchens an Ultraschall zum Platzen gebracht wird.

13. Verfahren nach irgendeinem der Ansprüche 1-12, wobei das Tröpfchen eine Zelle enthält,
(a) wobei die Zelle eine menschliche Zelle ist; und/oder
(b) wobei die Zelle eine Krebszelle ist; und/oder
(c) wobei die Zelle eine Immunzelle ist; und/oder
(d) wobei die Zelle eine Bakterienzelle ist; und/oder
(e) wobei die Zelle eine natürlich vorkommende Zelle ist; und
wobei der erste Zustand ist:
(a) der Typ der Zelle; und/oder
(b) die Exposition an einen Arzneistoff, bei dem vermutet wird, dass er zur Interaktion mit der Zelle in der Lage ist; und/oder
(c) die Exposition an einen mutmaßlichen Arzneistoff, wobei bevorzugt der Arzneistoff zytotoxisch ist.

14. Verwendung eines Gegenstands, umfassend:
eine Mehrzahl mikrofluidischer Tröpfchen, wobei wenigstens einige der Tröpfchen einen oder mehrere Primer und eine oder mehrere Nukleinsäuren, die eine Mehrzahl von Zuständen codieren, denen die wenigstens einigen Tröpfchen ausgesetzt waren, umfassen, wobei bevorzugt die Nukleinsäuren wenigstens drei Zustände codieren, in einem Verfahren zum Bestimmen der Geschichte eines Tröpfchens nach Anspruch 1.

15. Verwendung nach Anspruch 14, wobei wenigstens einige der Nukleinsäuren molekulare Spezies codieren, die innerhalb wenigstens einiger der mikrofluidischen Tröpfchen enthalten sind, wobei bevorzugt wenigstens einige der mikrofluidischen Tröpfchen weiterhin die jeweiligen molekularen Spezies umfassen, die durch die Nukleinsäuren codiert werden, wobei bevorzugt wenigstens einige der mikrofluidischen Tröpfchen wenigstens zwei molekulare Spezies und Nukleinsäuren, die die wenigstens zwei molekularen Spezies codieren, enthalten; und/oder
wobei wenigstens einige der mikrofluidischen Tröpfchen:
(a) wenigstens drei molekulare Spezies und Nukleinsäuren, die die wenigstens drei molekularen Spezies codieren, enthalten; und/oder
(b) eine Polymerase enthalten; und/oder
(c) Taq-Polymerase enthalten; und/oder
(d) bei einer Temperatur von wenigstens etwa 50°C vorliegen; und/oder
(e) bei einer Temperatur von wenigstens etwa 90°C vorliegen; und/oder
(f) Desoxyribonukleotide enthalten.

## Revendications

1. Procédé pour déterminer l'historique d'une gouttelette, comprenant :
l'exposition d'une gouttelette microfluidique à une première condition ;
l'ajout d'un premier acide nucléique à la gouttelette, dans lequel le premier acide nucléique code pour l'étape d'exposition d'une gouttelette microfluidique à la première condition ;
l'exposition de la gouttelette microfluidique à une deuxième condition ;
l'ajout d'un deuxième acide nucléique à la gouttelette, dans lequel le deuxième acide nucléique code pour l'étape d'exposition de la gouttelette microfluidique à la deuxième condition ; et
l'amplification du premier acide nucléique et du deuxième acide nucléique dans la gouttelette microfluidique pour produire un acide nucléique amplifié ; et
le séquençage de l'acide nucléique amplifié pour déterminer l'historique de la gouttelette.

2. Procédé selon la revendication 1, dans lequel l'exposition de la gouttelette microfluidique à la première condition et l'ajout du premier acide nucléique à la gouttelette comprennent :
(a) la fusion de la gouttelette microfluidique à une seconde gouttelette contenant le premier acide nucléique ; et/ou
(b) l'exposition de la gouttelette à une espèce moléculaire, de préférence dans lequel l'exposition de la gouttelette à l'espèce moléculaire comprend la fusion de la gouttelette à une seconde gouttelette contenant l'espèce moléculaire, de préférence dans lequel la seconde gouttelette :
(i) contient en outre le premier acide nucléique, de sorte que, lorsque la seconde gouttelette est fusionnée à la gouttelette, le premier acide nucléique est ajouté à la gouttelette ; et/ou
(ii) est fusionnée à la gouttelette via des dipôles induits dans les gouttelettes ; et/ou
(iii) est fusionnée à la gouttelette via des charges électriques opposées placées sur les gouttelettes.

3. Procédé selon la revendication 2, dans lequel l'exposition de la gouttelette microfluidique à l'espèce moléculaire comprend l'injection de la gouttelette microfluidique avec un fluide contenant l'espèce moléculaire, de préférence dans lequel le fluide contenant l'espèce moléculaire contient en outre le premier acide nucléique.

4. Procédé selon l'une quelconque des revendications 2 ou 3, dans lequel :
(a) l'espèce moléculaire et le premier acide nucléique sont ajoutés simultanément à la gouttelette microfluidique ; et/ou
(b) l'espèce moléculaire est ajoutée à la gouttelette avant que le premier acide nucléique ait été ajouté à la gouttelette ; et/ou
(c) l'espèce moléculaire est ajoutée à la gouttelette après que le premier acide nucléique a été ajouté à la gouttelette.

5. Procédé selon l'une quelconque des revendications 1 à 4, dans lequel le premier acide nucléique et/ou le deuxième acide nucléique comprennent une séquence d'amorce ; et/ou dans lequel le procédé comprend en outre l'immobilisation du premier acide nucléique et du deuxième acide nucléique l'un par rapport à l'autre, comprenant de préférence la ligature du premier acide nucléique au deuxième acide nucléique.

6. Procédé selon l'une quelconque des revendications 1 à 5, dans lequel l'amplification du premier acide nucléique et du deuxième acide nucléique dans la gouttelette microfluidique comprend :
(a) l'utilisation d'une PCR pour amplifier le premier acide nucléique et le deuxième acide nucléique ; et/ou
(b) l'ajout d'une polymérase à la gouttelette microfluidique ; et/ou
(c) l'ajout d'une Taq polymérase à la gouttelette microfluidique ; et/ou
(d) l'exposition de la gouttelette microfluidique à une température d'au moins environ 50 °C, de préférence d'au moins environ 90 °C ; et/ou
(e) l'ajout de désoxyribonucléotides à la gouttelette microfluidique.

7. Procédé selon l'une quelconque des revendications 1 à 6, dans lequel l'exposition de la gouttelette microfluidique à la première condition comprend :
(a) l'exposition de la gouttelette à une condition physique externe, de préférence dans lequel l'exposition de la gouttelette à une condition physique externe comprend l'exposition de la gouttelette à une température prédéterminée et/ou une pression prédéterminée ; et/ou
(b) la fusion de la gouttelette à une seconde gouttelette présentant un pH inférieur à 7 et/ou un pH supérieur à 7.

8. Procédé selon l'une quelconque des revendications 1 à 7, dans lequel la gouttelette microfluidique :
(a) présente un diamètre moyen en coupe transversale inférieur à environ 1 mm ; et/ou
(b) est l'une d'une pluralité de gouttelettes microfluidiques présentant une distribution de diamètres telle que pas plus d'environ 5 % des gouttelettes microfluidiques présentent un diamètre inférieur à environ 90 % ou supérieur à environ 110 % du diamètre moyen global de la pluralité de gouttelettes microfluidiques ; et/ou
(c) est contenue dans un canal microfluidique.

9. Procédé selon l'une quelconque des revendications 1 à 8, comprenant en outre l'exposition de la gouttelette microfluidique à une troisième condition et l'ajout d'un troisième acide nucléique à la gouttelette microfluidique, dans lequel le troisième acide nucléique code pour la troisième condition, comprenant en outre de préférence la fixation du troisième acide nucléique à l'un ou aux deux des premier acide nucléique et deuxième acide nucléique.

10. Procédé selon l'une quelconque des revendications 1 à 9, comprenant en outre la détermination d'une propriété de la gouttelette microfluidique, comprenant de préférence le tri de la gouttelette sur la base de la propriété, et/ou dans lequel la propriété est :
(a) une fluorescence ; et/ou
(b) le diamètre moyen en coupe transversale de la gouttelette ; et/ou
(c) une absorption de lumière ; et/ou
(d) la concentration d'un agent contenu dans la gouttelette ; et/ou
(e) l'état d'une cellule contenue dans la gouttelette, de préférence dans lequel la propriété est le fait que la cellule est vivante ou morte et/ou la propriété est une concentration d'un agent produit et/ou consommé par la cellule.

11. Procédé selon la revendication 10, dans lequel la cellule est contenue dans un gel, de préférence dans lequel le gel est un gel d'agarose et/ou dans lequel le gel présente une température de solidification inférieure à environ 25 °C.

12. Procédé selon l'une quelconque des revendications 1 à 11, comprenant en outre la séparation du premier acide nucléique et du deuxième acide nucléique à partir de la gouttelette microfluidique, et/ou l'éclatement de la gouttelette microfluidique, de préférence dans lequel la gouttelette est éclatée en exposant la gouttelette à un ultrason.

13. Procédé selon l'une quelconque des revendications 1 à 12, dans lequel la gouttelette contient une cellule,
(a) dans lequel la cellule est une cellule humaine ; et/ou
(b) dans lequel la cellule est une cellule cancéreuse ; et/ou
(c) dans lequel la cellule est une cellule immunitaire ; et/ou
(d) dans lequel la cellule est une cellule bactérienne ; et/ou
(e) dans lequel la cellule est une cellule d'origine naturelle ; et
dans lequel la première condition est :
(a) le type de cellule ; et/ou
(b) l'exposition à un médicament suspecté d'être capable d'interagir avec la cellule ; et/ou
(c) l'exposition à un médicament suspecté, de préférence dans lequel le médicament est cytotoxique.

14. Utilisation d'un article, comprenant :
une pluralité de gouttelettes microfluidiques, au moins certaines des gouttelettes contenant une ou plusieurs amorces et un ou plusieurs acides nucléiques codant pour une pluralité de conditions auxquelles les au moins certaines gouttelettes ont été exposées, de préférence dans laquelle les acides nucléiques codent pour au moins trois conditions, dans un procédé pour déterminer l'historique d'une gouttelette selon la revendication 1.

15. Utilisation selon la revendication 14, dans laquelle au moins certains des acides nucléiques codent pour des espèces moléculaires contenues dans au moins certaines des gouttelettes microfluidiques, de préférence dans laquelle au moins certaines des gouttelettes microfluidiques comprennent en outre les espèces moléculaires respectives codées par les acides nucléiques, de préférence dans laquelle au moins certaines des gouttelettes microfluidiques contiennent au moins deux espèces moléculaires et des acides nucléiques codant pour les au moins deux espèces moléculaires ; et/ou
dans laquelle au moins certaines des gouttelettes microfluidiques :
(a) contiennent au moins trois espèces moléculaires et des acides nucléiques codant pour les au moins trois espèces moléculaires ; et/ou
(b) contiennent une polymérase ; et/ou
(c) contiennent une Taq polymérase ; et/ou
(d) sont à une température d'au moins environ 50 °C ; et/ou
(e) sont à une température d'au moins environ 90 °C ; et/ou
(f) contiennent des désoxyribonucléotides.
